# EUROPEAN PATENT APPLICATION

(11) **EP 2 837 620 A1**
(43) Date of publication of application: **18.02.2015**
(21) Application number: 13775655.7
(22) Date of filing: 08.04.2013
(51) Int. Cl.: C07C 317/32, A01N 47/28, A01N 47/34, A01P 7/02, A61K 31/423, A61K 31/428, A61K 31/437, A61K 31/44, A61K 31/4439, A61P 33/14, C07D 213/70, C07D 277/62, C07D 413/12, C07D 417/12, C07D 417/14, C07D 513/04

(54) **ARYLOXYUREA COMPOUND AND PEST CONTROL AGENT**

(30) Priority: 10.04.2012 JP 2012089459; 01.10.2012 JP 2012219095
(71) Applicant: Nippon Soda Co., Ltd., Tokyo 100-8165 (JP)
(72) Inventor: FURUKAWA, Hironori, Odawara-shi Kanagawa 250-0280 (JP); HANAI, Daisuke, Odawara-shi Kanagawa 250-0280 (JP); TAMAI, Tetsuo, Odawara-shi Kanagawa 250-0280 (JP); KANAZAWA, Jun, Odawara-shi Kanagawa 250-0280 (JP); TANAKA, Katsunori, Odawara-shi Kanagawa 250-0280 (JP); NAGAGATA, Asaho, Odawara-shi Kanagawa 250-0280 (JP)
(74) Representative: Wills, Andrew Jonathan
(86) International application number: PCT/JP2013/060658
(87) International publication number: WO 2013/154080

(57) **Abstract**

The present invention provides an aryloxyurea compound or salt thereof, and a pest control agent including the aryloxyurea compound or salt thereof as an active ingredient. The aryloxyurea compound has a superior acaricidal and/or insecticidal activity, superior safety, and can be synthesized advantageously and industrially. The compound of the present invention includes aryloxyurea compounds represented by the following formula or salts thereof: 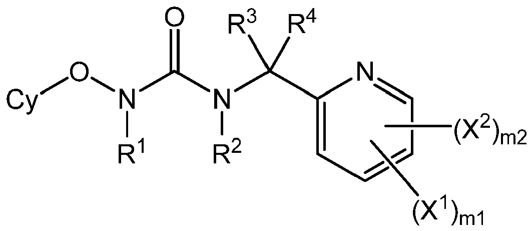 in the formula, Cy represents an unsubstituted or X-substituted pheyl group or the like, X represents a halogen atom or the like, R¹ represents an ethyl group or the like, R² represents a hydrogen atom or the like, R³ and R⁴ represents a methyl group or the like, X¹ represents a methylthio group ro the like, m1 represents an integer of 1 to 4, X² represents a methyl group or the like, m2 is an integer of 0 to 3.

## Description

### TECHNICAL FIELD

The present invention relates to an aryloxyurea compound and a pest control agent. More specifically, the present invention relates to an aryloxyurea compound and a pest control agent containing the aryloxyurea compound as an active ingredient. The aryloxyurea compound has a superior acaricidal and/or insecticidal activity, superior safety, and can be synthesized advantageously and industrially.

Priority is claimed on Japanese Patent Application No. 2012-089459, filed April 10, 2012, and Japanese Patent Application No. 2012-219095, filed October 1, 2012, the content of which is incorporated herein by reference.

### BACKGROUND ART

Compounds represented by formulas (A) to (C), which are structurally relevant to the compound of the present invention, are disclosed in Patent documents 1 to 3.

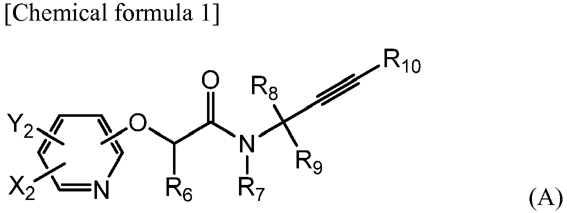

In formula (A), X₂ represents a hydrogen atom, halogen atom, C1-8 alkyl group or the like.
Y₂ represents a hydrogen atom, halogen atom, C1-8 alkyl group or the like.
R₆ represents a phenyl group, cyano group, C1-4 alkyl group or the like.
R₇ represents a hydrogen atom, C1-4 alkyl group or the like.
R₈ and R₉ independently represents a hydrogen atom, C1-3 alkyl group or the like.
R₁₀ represents a halogen atom or C1-4 alkyl group.

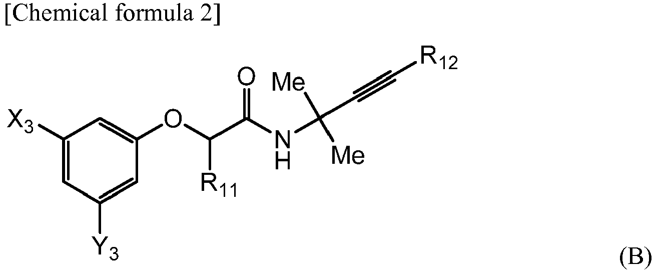

In formula (B), X₃ represents a chlorine atom, bromine atom, or methyl group.
Y₃ represents a chlorine atom, bromine atom, or methyl group.
R₁₁ represents an ethyl group or n-propyl group.
R₁₂ represents an ethyl group.

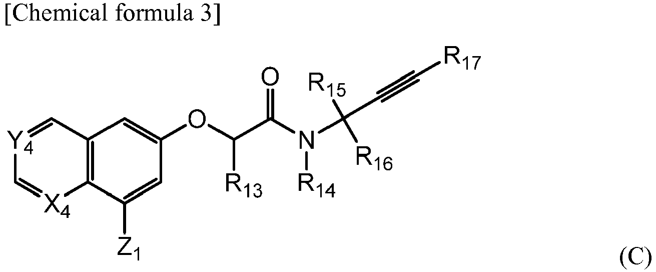

In formula (C), one of X₄ and Y₄ represents a nitrogen atom or nitrogen oxide, and the other one of X₄ and Y₄ represents CR (wherein R represents a hydrogen atom, halogen atom or the like), or both of X₄ and Y₄ represent a nitrogen atom.
Z₁ represents a hydrogen atom, halogen atom or the like.
R₁₃ represents an alkyl group, alkenyl group or the like.
R₁₄ represents a benzyloxymethyl group or the like, wherein the phenyl ring of the benzyl moiety is optionally substituted with a C1-4 alkoxy group.
R₁₅ and R₁₆ do not simultaneously represent a hydrogen atom, and when both of them are not a hydrogen atom, they independently represents a hydrogen atom, C1-3 alkyl group or the like, provided that the number of carbon atoms of the combination of them does not exceed 4.
R₁₇ represents a C1-4 alkyl group, C3-6 cycloalkyl group or the like.

### PRIOR ART LITERATURE

### Patent Documents

Patent document 1: Japanese Unexamined Patent Application Publication No. 2005-517642 (WO 2003/048128)
Patent document 2: Japanese Unexamined Patent Application Publication No. 2006-507338 (WO 2004/047537)
Patent document 3: Japanese Unexamined Patent Application Publication No. 2006-507339 (WO 2004/047538)

### DISCLOSURE OF INVENTION

### Problems to be Solved by the Invention

The objective of the present invention is to provide an aryloxyurea compound or salt thereof, and a pest control agent containing the aryloxyurea compound or salt thereof as an active ingredient. The aryloxyurea compound has a pesticidal activity, particularly, a superior acaricidal and/or insecticidal activity, superior safety, and can be synthesized advantageously and industrially.

### Means for Solving the Problems

In order to achieve the above objective, the present inventors conducted extensive studies. As a result, the present inventors discovered that an aryloxyurea compound or salt thereof having a specific structure demonstrates a superior acaricidal and/or insecticidal activity, excellent properties and high safety as an active ingredient of a pest control agent.

The present invention was achieved on the basis of this perception.

Namely, the present invention is as follows:
[1] An aryloxyurea compound represented by formula (I) or salt thereof: 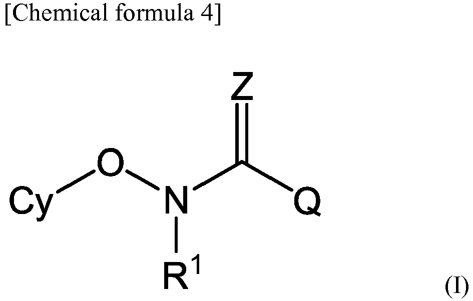
in formula (I),
Cy represents an unsubstituted or X-substituted C6-10 aryl group, or unsubstituted or X-substituted heteroaryl group;
X represents an unsubstituted or substituted C1-6 alkyl group, unsubstituted or substituted C3-8 cycloalkyl group, unsubstituted or substituted C2-6 alkenyl group, unsubstituted or substituted C2-6 alkynyl group, hydroxy group, unsubstituted or substituted C1-6 alkoxy group, unsubstituted or substituted amino group, unsubstituted or substituted C1-7 acyl group, unsubstituted or substituted C1-6 alkoxycarbonyl group, unsubstituted or substituted C1-6 alkylthio group, unsubstituted or substituted C1-6 alkyl sulfonyl group, unsubstituted or substituted C1-6 alkoxysulfonyl group, unsubstituted or substituted C6-10 aryl group, unsubstituted or substituted heteroaryl group, unsubstituted or substituted hydroxyimino C1-6 alkyl group, nitro group, cyano group, or halogen atom; X may be 2 or more, when X is 2 or more, Xs may be the same as or different from each other;
R¹ represents an unsubstituted or substituted C1-6 alkyl group, unsubstituted or substituted C2-6 alkenyl group, unsubstituted or substituted C2-6 alkynyl group, unsubstituted or substituted C1-7 acyl group, or unsubstituted or substituted C1-6 alkoxycarbonyl group;
Z represents an oxygen atom or sulfur atom;
Q represents a group represented by formula (II) or (III): 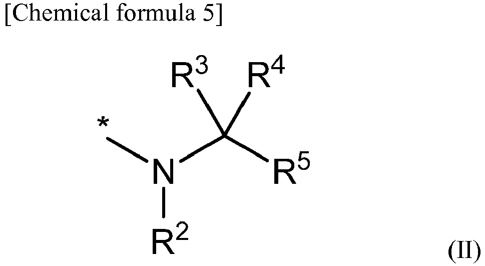 in formula (II),
* represents bonding position;
R² represents a hydrogen atom, unsubstituted or substituted C1-6 alkyl group, unsubstituted or substituted C2-6 alkenyl group, unsubstituted or substituted C2-6 alkynyl group, unsubstituted or substituted C1-7 acyl group, or unsubstituted or substituted C1-6 alkoxycarbonyl group;
R³ and R⁴ each independently represents a hydrogen atom, unsubstituted or substituted C1-6 alkyl group, unsubstituted or substituted C2-6 alkenyl group, unsubstituted or substituted C2-6 alkynyl group, unsubstituted or substituted C6-10 aryl group, unsubstituted or substituted heteroaryl group, or cyano group; R³ and R⁴ may bond to form a ring together with the carbon atom which bonds with R³ and R⁴;
R⁵ represents an unsubstituted or substituted C1-6 alkyl group, unsubstituted or substituted C3-8 cycloalkyl group, unsubstituted or substituted C2-6 alkenyl group, unsubstituted or substituted C2-6 alkynyl group, unsubstituted or substituted C1-7 acyl group, carboxyl group, unsubstituted or substituted C1-6 alkoxycarbonyl group, unsubstituted or substituted C2-6 alkenyloxycarbonyl group, unsubstituted or substituted C2-6 alkynyloxycarbonyl group, unsubstituted or substituted aminocarbonyl group, unsubstituted or substituted C6-10 aryl group, unsubstituted or substituted heteroaryl group, unsubstituted or substituted hydroxyimino C1-6 alkyl group, or cyano group; 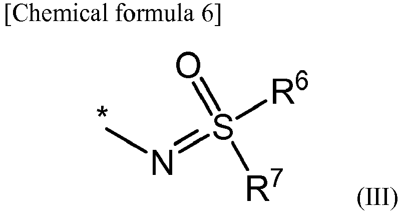 in formula (III),
* represents bonding position;
R⁶ and R⁷ each independently represents an unsubstituted or substituted C1-6 alkyl group, unsubstituted or substituted C3-8 cycloalkyl group, unsubstituted or substituted C2-6 alkenyl group, unsubstituted or substituted C2-6 alkynyl group, unsubstituted or substituted C6-10 aryl group, or unsubstituted or substituted heteroaryl group; R⁶ and R⁷ may bond to form a ring together with the sulfur atom which bonds with R⁶ and R⁷.

[2] The aryloxyurea compound or salt thereof according to [1], wherein
Cy of formula (I) is a group represented by formula (IV): 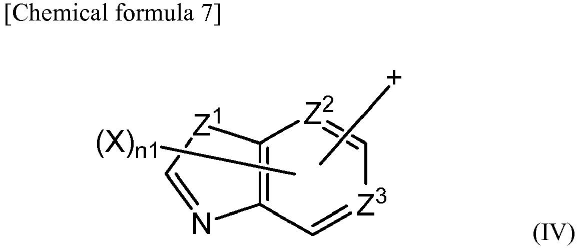 in formula (IV),
+ represents bonding position;
X is as defiend above;
n1 represents the number of X and represents an integer of 0 to 4;
Z¹ represents an oxygen atom or sulfur atom;
Z² and Z³ each independently represents a carbon atom or nitrogen atom.

[3] The aryloxyurea compound or salt thereof according to [1] or [2], wherein
Q of formula (I) is a group represented by formula (V): 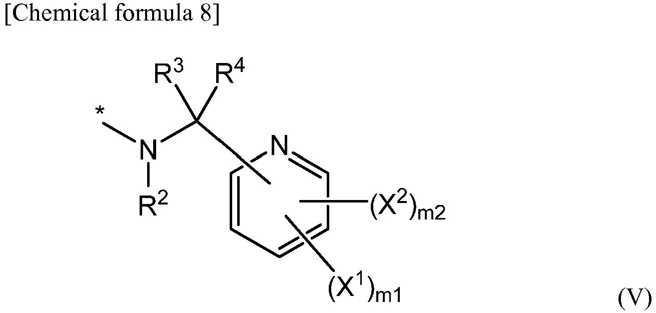 in formula (V),
* represents bonding position;
R², R³ and R⁴ are as defiend above;
X¹ represents an unsubstituted or substituted amino group, unsubstituted or substituted C1-6 alkylthio group, unsubstituted or substituted C1-6 alkyl sulfinyl group, or unsubstituted or substituted C1-6 alkyl sulfonyl group;
m1 represents the number of X¹ and represents an integer of 1 to 4, when m1 is 2 or more, X¹s may be the same as or different from each other;
X² represents an unsubstituted or substituted C1-6 alkyl group, unsubstituted or substituted C3-8 cycloalkyl group, unsubstituted or substituted C2-6 alkenyl group, unsubstituted or substituted C2-6 alkynyl group, hydroxy group, unsubstituted or substituted C1-6 alkoxy group, unsubstituted or substituted C1-7 acyl group, unsubstituted or substituted C1-6 alkoxycarbonyl group, unsubstituted or substituted C6-10 aryl group, unsubstituted or substituted heteroaryl group, oxo group, nitro group, cyano group, or halogen atom;
m2 represents the number of X² and represents an integer of 0 to 3, when m2 is 2 or more, X²s may be the same as or different from each other. The sum of m1 and m2 is 4 or less.

[4] The aryloxyurea compound or salt thereof according to [1] or [2], wherein
Q of formula (I) is a group represented by formula (VI): 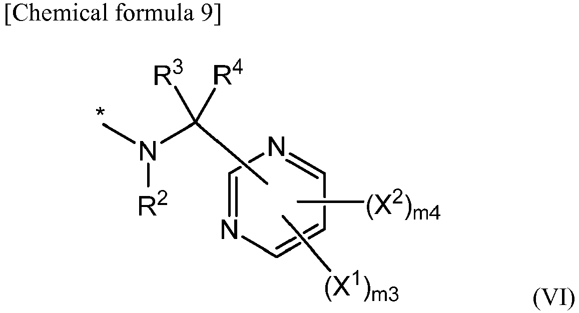 in formula (VI),
* represents bonding position;
R², R³, and R⁴ are as difined above;
X¹ represents an unsubstituted or substituted amino group, unsubstituted or substituted C1-6 alkylthio group, unsubstituted or substituted C1-6 alkyl sulfinyl group, or unsubstituted or substituted C1-6 alkyl sulfonyl group;
m3 represents the number of X¹ and represents an integer of 1 to 3, when m3 is 2 or more, X¹s may be the same as or different from each other;
X² represents an unsubstituted or substituted C1-6 alkyl group, unsubstituted or substituted C3-8 cycloalkyl group, unsubstituted or substituted C2-6 alkenyl group, unsubstituted or substituted C2-6 alkynyl group, hydroxy group, unsubstituted or substituted C1-6 alkoxy group, unsubstituted or substituted C1-7 acyl group, unsubstituted or substituted C1-6 alkoxycarbonyl group, unsubstituted or substituted C6-10 aryl group, unsubstituted or substituted heteroaryl group, oxo group, nitro group, cyano group, or halogen atom;
m4 represents the number of X² and represents an integer of 0 to 2, when m4 is 2 or more, X²s may be the same as or different from each other; the sum of m3 and m4 is 3 or less.

[5] The aryloxyurea compound or salt thereof according to [1] or [2], wherein
Q of formula (I) is a group represented by formula (VII): 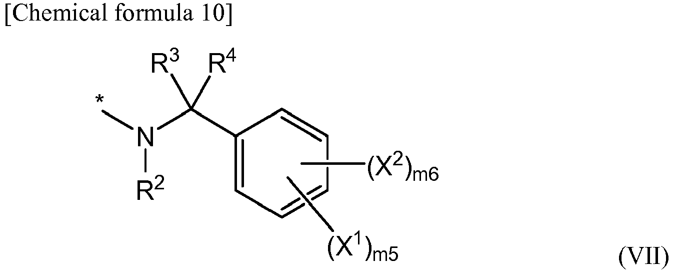 in formula (VII),
* represents bonding position;
R², R³, and R⁴ are as difined above;
X¹ represents an unsubstituted or substituted amino group, unsubstituted or substituted C1-6 alkylthio group, unsubstituted or substituted C1-6 alkyl sulfinyl group, or unsubstituted or substituted C1-6 alkyl sulfonyl group;
m5 represents the number of X¹ and represents an integer of 1 to 5, when m5 is 2 or more, X¹s may be the same as or different from each other;
X² represents an unsubstituted or substituted C1-6 alkyl group, unsubstituted or substituted C3-8 cycloalkyl group, unsubstituted or substituted C2-6 alkenyl group, unsubstituted or substituted C2-6 alkynyl group, hydroxy group, unsubstituted or substituted C1-6 alkoxy group, unsubstituted or substituted C1-7 acyl group, unsubstituted or substituted C1-6 alkoxycarbonyl group, unsubstituted or substituted C6-10 aryl group, unsubstituted or substituted heteroaryl group, oxo group, nitro group, cyano group, or halogen atom;
m6 represents the number X² and represents an integer of 0 to 4, when m6 is 2 or more, X²s may be the same as or different from each other, the sum of m5 and m6 is 5 or less.

[6] A pest control agent comprising as an active ingredient at least one selected from the aryloxyurea compounds and salts thereof defined in any one of [1] to [5].
[7] An acaricide or insenticide comprising as an active ingredient at least one selected from the aryloxyurea compounds and salts thereof defined in any one of [1] to [5].
[8] An ectoparasite control agent comprising as an active ingredient at least one selected from the aryloxyurea compounds and salts thereof defined in any one of [1] to [5].
[9] A benzyloxyurea compound represented by formula (VIII):

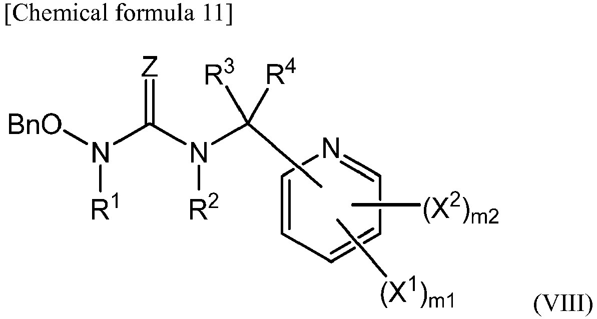

in formula (VIII),
R¹ represents an unsubstituted or substituted C1-6 alkyl group, unsubstituted or substituted C2-6 alkenyl group, unsubstituted or substituted C2-6 alkynyl group, unsubstituted or substituted C1-7 acyl group, or unsubstituted or substituted C1-6 alkoxycarbonyl group;
R² represents a hydrogen atom, unsubstituted or substituted C1-6 alkyl group, unsubstituted or substituted C2-6 alkenyl group, unsubstituted or substituted C2-6 alkynyl group, unsubstituted or substituted C1-7 acyl group, or unsubstituted or substituted C1-6 alkoxycarbonyl group;
R³ and R⁴ each independently represents a hydrogen atom, unsubstituted or substituted C1-6 alkyl group, unsubstituted or substituted C2-6 alkenyl group, unsubstituted or substituted C2-6 alkynyl group, unsubstituted or substituted C6-10 aryl group, unsubstituted or substituted heteroaryl group, or cyano group; R³ and R⁴ may bond to form a ring together with the carbon atom which bonds with R³ and R⁴.
Z represents an oxygen atom or sulfur atom;
X¹ represents an unsubstituted or substituted amino group, unsubstituted or substituted C1-6 alkylthio group, unsubstituted or substituted C1-6 alkyl sulfinyl group, or unsubstituted or substituted C1-6 alkyl sulfonyl group;
m1 represents the number of X¹ and represents an integer of 1 to 4, when m1 is 2 or more, X¹s may be the same as or different from each other;
X² represents an unsubstituted or substituted C1-6 alkyl group, unsubstituted or substituted C3-8 cycloalkyl group, unsubstituted or substituted C2-6 alkenyl group, unsubstituted or substituted C2-6 alkynyl group, hydroxy group, unsubstituted or substituted C1-6 alkoxy group, unsubstituted or substituted C1-7 acyl group, unsubstituted or substituted C1-6 alkoxycarbonyl group, unsubstituted or substituted C6-10 aryl group, unsubstituted or substituted heteroaryl group, oxo group, nitro group, cyano group, or halogen atom;
m2 represents the number of X² and represents an integer of 0 to 3, when m2 is 2 or more, X²s may be the same as or different from each other, the sum of m1 and m2 is 4 or less.

### Effects of the Invention

The aryloxyurea compound or salt thereof according to the present invention can prevent pests which are harmful for agricultural crops and cause the problem of hygiene. Particularly, the compound or salt thereof can effectively prevent acarus and insecticides.

### BEST MODE FOR CARRYING OUT THE INVENTION

### [Aryloxyurea Compound]

The aryloxyurea compound of the present invention is a compound represented by formula (I) (hereinafter, may be referred to as "Compound (I)").

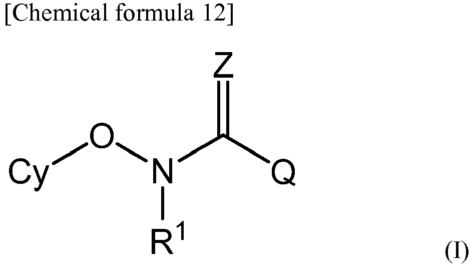

Firstly, in the present invention, the term "unsubstituted" indicates a group including only a mother nucleus. When a group is referred to as a name of a mother nucleus without "substituted", this refers to "unsubstituted" unless specifically indicated otherwise.

On the other hand, the term "substituted" indicates that at least one of the hydrogen atoms of the mother nucleus is substituted with a substituent having a structure that is the same as the structure of the mother nucleus or different from the structure of the mother nucleus. Thus, a "substituent" is another group bonded with the mother nucleus. There may be one substituent or two or more substituents. Two or more substituents may be the same as or different from each other.

For example, the term "C1-6" or the like indicates that the number of carbon atoms of the mother nucleus is 1 to 6. The number of carbon atoms present in substituents is not included in this number of carbon atoms. For example, a butyl group having an ethoxy group as a substituent thereof is classified as a C2 alkoxy C4 alkyl group.

The "substituent" is not particularly limited as long as it is chemically permissible and achieves the effects of the present invention.

Specific examples of the "substituent" include the following groups:
a halogen atom such as a fluorine atom, chlorine atom, bromine atom, iodine atom or the like;
a C1-6 alkyl group such as a methyl group, ethyl group, n-propyl group, i-propyl group, n-butyl group, s-butyl group, i-butyl group, t-butyl group, n-pentyl group, n-hexyl group or the like;
a C3-8 cycloalkyl group such as a cyclopropyl group, cyclobutyl group, cyclopentyl group, cyclohexyl group, cycloheptyl group or the like;
a C2-6 alkenyl group such as a vinyl group, 1-propenyl group, 2-propenyl group, 1-butenyl group, 2-butenyl group, 3-butenyl group, 1-methyl-2-propenyl group, 2-methyl-2-propenyl group, 1-pentenyl group, 2-pentenyl group, 3-pentenyl group, 4-pentenyl group, 1-methyl-2-butenyl group, 2-methyl-2-butenyl group, 1-hexenyl group, 2-hexenyl group, 3-hexenyl group, 4-hexenyl group, 5-hexenyl group or the like;
a C3-8 cycloalkenyl group such as a 2-cyclopropenyl group, 2-cyclopentenyl group, 3-cyclohexenyl group, 4-cyclooctenyl group or the like;
a C2-6 alkynyl group such as an ethynyl group, 1-propynyl group, 2-propynyl group, 1-butynyl group, 2-butynyl group, 3-butynyl group, 1-methyl-2-propynyl group, 2-methyl-3-butynyl group, 1-pentynyl group, 2-pentynyl group, 3-pentynyl group, 4-pentynyl group, 1-methyl-2-butynyl group, 2-methyl-3-pentynyl group, 1-hexynyl group, 1, 1-dimethyl-2-butynyl group or the like;

a C1-6 alkoxy group such as a methoxy group, ethoxy group, n-propoxy group, i-propoxy group, n-butoxy group, s-butoxy group, i-butoxy group, t-butoxy group or the like;
a C2-6 alkenyloxy group such as a vinyloxy group, allyloxy group, propenyloxy group, butenyloxy group or the like;
a C2-6 alkynyloxy group such as an ethynyloxy group, propargyloxy group or the like;
a C6-10 aryl group such as a phenyl group, naphthyl group or the like;
a C6-10 aryloxy group such as a phenoxy group, 1-naphthoxy group or the like;
a C7-11 aralkyl group such as a benzyl group, phenethyl group or the like;
a C7-11 aralkyloxy group such as a benzyloxy group, phenethyloxy group or the like;
a C1-7 acyl group such as a formyl group, acetyl group, propionyl group, benzoyl group, cyclohexyl carbonyl group or the like;
a C1-7 acyloxy group such as a formyloxy group, acetyloxy group, propionyloxy group, benzoyloxy group, cyclohexyl carbonyloxy group or the like;
a C1-6 alkoxycarbonyl group such as a methoxycarbonyl group, ethoxycarbonyl group, n-propoxycarbonyl group, i-propoxycarbonyl group, n-butoxycarbonyl group, t-butoxycarbonyl group or the like;
a carboxyl group;
a hydroxy group; an oxo group;
a cyclic ether group such as an oxiranyl group, tetrahydrofuryl group, dioxolanyl group, dioxanyl or the like;

a C1-6 haloalkyl group such as a chloromethyl group, chloroethyl group, trifluoromethyl group, 1, 2-dichloro-n-propyl group, 1-fluoro-n-butyl group, perfluoro-n-pentyl group or the like;
a C2-6 haloalkenyl group such as a 2-chloro-1-propenyl group, 2-fluoro-1-butenyl group or the like;
a C2-6 haloalkynyl group such as a 4, 4-dichloro-1-butynyl group, 4-fluoro-1-pentynyl group, 5-bromo-2-pentynyl group or the like;
a C1-6 haloalkoxy group such as a 2-chloro-n-propoxy group, 2, 3-dichlorobutoxy group or the like;
a C2-6 haloalkenyloxy group such as a 2-chloropropenyloxy group, 3-bromobutenyloxy group or the like;
a C6-10 haloaryl group such as a 4-chlorophenyl group, 4-fluorophenyl group, 2, 4-dichlorophenyl group or the like;
a C6-10 haloaryloxy group such as a 4-fluorophenyloxy group, 4-chloro-1-naphthoxy group or the like;
a C1-7 haloacyl group such as a chloroacetyl group, trifluoroacetyl group, trichloroacetyl group, 4-chlorobenzoyl group or the like;

a cyano group; a nitro group; an amino group;
a C1-6 alkyl amino group such as a methyl amino group, dimethyl amino group, diethyl amino group or the like;
a C6-10 aryl amino group such as an anilino group, naphthyl amino group or the like;
a C7-11 aralkyl amino group such as a benzyl amino group, phenyl ethyl amino group or the like;
a C1-7 acyl amino group such as a formyl amino group, acetyl amino group, propanoyl amino group, butyryl amino group, i-propyl carbonyl amino group, benzoyl amino group or the like;
a C1-6 alkoxycarbonyl amino group such as a methoxycarbonyl amino group, ethoxycarbonyl amino group, n-propoxycarbonyl amino group, i-propoxycarbonyl amino group or the like;
a cyclic amino group such as an aziridinyl group, pyrrolidinyl group, piperidyl group, piperazinyl group, morpholinyl group or the like;

an unsubstituted or substituted aminocarbonyl group such as an aminocarbonyl group, dimethyl aminocarbonyl group, phenyl aminocarbonyl group, N-phenyl-N-methyl aminocarbonyl group or the like;
an imino C1-6 alkyl group such as an iminomethyl group, (1-imino)ethyl group, (1-imino)-n-propyl group or the like;
a hydroxyimino C1-6 alkyl group such as a hydroxyiminomethyl group, (1-hydroxyimino)ethyl group, (1-hydroxyimino)propyl group or the like;
a methoxyiminomethyl group, (1-methoxyimino)ethyl group;

a mercapto group;
a C1-6 alkylthio group such as a methylthio group, ethylthio group, n-propylthio group, i-propylthio group, n-butylthio group, i-butylthio group, s-butylthio group, t-butylthio group or the like;
a C2-6 alkenylthio group such as a vinylthio group, allylthio group or the like;
a C2-6 alkynylthio group such as an ethynylthio group, propargylthio group or the like;
a C6-10 arylthio group such as a phenylthio group, naphthylthio group or the like;
a heteroarylthio group such as a thiazolylthio group, pyridylthio group or the like;
a C7-11 aralkylthio group such as a benzylthio group, phenethylthio group or the like;
a (C1-6 alkylthio)carbonyl group such as a (methylthio)carbonyl group, (ethylthio)carbonyl group, (n-propylthio)carbonyl group, (i-propylthio)carbonyl group, (n-butylthio)carbonyl group, (i-butylthio)carbonyl group, (s-butylthio)carbonyl group, (t-butylthio)carbonyl group or the like;

a C1-6 alkyl sulfinyl group such as a methyl sulfinyl group, ethyl sulfinyl group, t-butyl sulfinyl group or the like;
a C2-6 alkenyl sulfinyl group such as an allyl sulfinyl group or the like;
a C2-6 alkynyl sulfinyl group such as a propargyl sulfinyl group or the like;
a C6-10 aryl sulfinyl group such as a phenyl sulfinyl group or the like;
a heteroaryl sulfinyl group such as a thiazolyl sulfinyl group, pyridyl sulfinyl group or the like;
a C7-11 aralkyl sulfinyl group such as a benzyl sulfinyl group, phenethyl sulfinyl group or the like;
a C1-6 alkyl sulfonyl group such as a methyl sulfonyl group, ethyl sulfonyl group, t-butyl sulfonyl group or the like;
a C2-6 alkenyl sulfonyl group such as an allyl sulfonyl group or the like;
a C2-6 alkynyl sulfonyl group such as a propargyl sulfonyl group or the like;
a C6-10 aryl sulfonyl group such as a phenyl sulfonyl group or the like;
a heteroaryl sulfonyl group such as a thiazolyl sulfonyl group, pyridyl sulfonyl group or the like;
a C7-11 aralkyl sulfonyl group such as a benzyl sulfonyl group, phenethyl sulfonyl group or the like;

a 5-membered heteroaryl group such as a pyrrolyl group, furyl group, thienyl group, imidazolyl group, pyrazolyl group, oxazolyl group, isoxazolyl group, thiazolyl group, isothiazolyl group, triazolyl group, oxadiazolyl group, thiadiazolyl group, tetrazolyl group or the like;
a 6-membered heteroaryl group such as a pyridyl group, pyrazinyl group, pyrimidinyl group, pyridazinyl group, triazinyl group or the like;
a condensed heteroaryl group such as an indolyl group, benzofuryl group, benzothienyl group, benzimidazolyl group, benzoxazolyl group, benzothiazolyl group, quinolyl group, isoquinolyl group, quinoxalinyl group or the like;
a triC 1-6 alkyl-substituted silyl group such as a trimethyl silyl group, triethyl silyl group, t-butyl dimethyl silyl group or the like;
a triphenyl silyl group; and the like.

In addition, any of the hydrogen atoms in these "substituents" may be substituted with other groups having a different structure.

### [Cy]

In formula (I), Cy represents an unsubstituted or X-substituted C6-10 aryl group or an unsubstituted or X-substituted heteroaryl group.

The "C6-10 aryl group" may be a monocyclic ring or polycyclic ring. If the polycyclic aryl group has at least one aromatic ring, other rings may be a saturated alicyclic ring, an unsaturated alicyclic ring or an aromatic ring.

Examples of the C6-10 aryl group include a phenyl group, naphthyl group, azulenyl group, indenyl group, indanyl group, tetralinyl group and the like.

The "heteroaryl group" is a 5- to 10- membered aryl group which has, other than carbon atoms, 1 to 4 hetetroatoms selected from the group consisting of a nitrogen atom, oxygen atom and sulfur atom as the atoms constituting the ring. The heteroaryl group may be a monocyclic ring or polycyclic ring. If the polycyclic heteroaryl group has at least one heteroaryl gourp, other rings may be a saturated alicyclic ring, unsaturated alicyclic ring or aromatic ring.

Examples of the heteroaryl group include a 5-membered heteroaryl group, 6-membered heteroaryl group, condensed heteroaryl group and the like which are listed as the examples of the "substituent". Among these groups, a phenyl group, naphthyl group, pyridyl group, pyrimidinyl group, pyridazinyl group, indolyl group, benzofuryl group, benzothienyl group, benzimidazolyl group, benzoxazolyl group, benzothiazolyl group, quinolyl group, isoquinolyl group and quinoxalinyl group are preferable, and a phenyl group is more preferable.

The number of X of the "C6-10 aryl group" or "heteroaryl group", which is represented by "n" is an integer of 1 to 4. In addition, when the "C6-10 aryl group" or "heteroaryl group" is unsubstituted, n is 0.

### [X]

X represents an unsubstituted or substituted C1-6 alkyl group, unsubstituted or substituted C3-8 cycloalkyl group, unsubstituted or substituted C2-6 alkenyl group, unsubstituted or substituted C2-6 alkynyl group,hydroxy group, unsubstituted or substituted C1-6 alkoxy group, unsubstituted or substituted amino group, unsubstituted or substituted C1-7 acyl group, unsubstituted or substituted C1-6 alkoxycarbonyl group, unsubstituted or substituted C1-6 alkyl thio group, unsubstituted or substituted C1-6 alkyl sulfonyl group, unsubstituted or substituted C1-6 alkoxysulfonyl group, unsubstituted or substituted C6-10 aryl group, unsubstituted or substituted heteroaryl group, unsubstituted or substituted hydroxyimino C1-6 alkyl group,nitro group,cyano group, or halogen atom. The number of X may be 2 or more. The the upper limit of the number of X may be a chemically acceptable number, but the upper limit is preferably 4. When the number of X is more than 2, Xs may be the same as or different from each other.

The "C1-6 alkyl group" of X may be a linear alkyl group or a branched alkyl group. Examples of the "C1-6 alkyl group" include a methyl group, ethyl group, n-propyl group, n-butyl group, n-pentyl group, n-hexyl group, i-propyl group, i-butyl group, s-butyl group, t-butyl group, i-pentyl group, neopentyl group, 2-methyl butyl group, 2, 2-dimethyl propyl group, i-hexyl group or the like.

Examples of the "substituted C1-6 alkyl group" include
a C3-8 cycloalkyl C1-6 alkyl group such as a cyclopropyl methyl group, 2-cyclopropyl ethyl group, cyclopentyl methyl group, 2-cyclohexyl ethyl group, 2-cyclooctyl ethyl group or the like;
a C1-6 haloalkyl group such as a fluoromethyl group, chloromethyl group, bromomethyl group, difluoromethyl group, dichloromethyl group, dibromomethyl group, trifluoromethyl group, trichloromethyl group, tribromomethyl group, 2, 2, 2-tolufluoroethyl group, 2, 2, 2-trichloroethyl group, pentafluoroethyl group, 4-fluorobutyl group, 4-chlorobutyl group, 3, 3, 3-trifluoropropyl group, 2, 2, 2-trifluoro-1-trifluoromethyl ethyl group, perfluorohexyl group, perchlorohexyl group, 2, 4, 6-trichlorohexyl group or the like;

a hydroxy C1-6 alkyl group such as a hydroxymethyl group, 2-hydroxyethyl group or the like;
a C1-6 alkoxy C1-6 alkyl group such as a methoxymethyl group, ethoxymethyl group, methoxyethyl group, ethoxyethyl group, methoxy-n-propyl group, ethoxymethyl group, ethoxyethyl group, n-propoxymethyl group, i-propoxyethyl group, s-butoxymethyl group, t-butoxyethyl group or the like;
a C2-6 alkenyloxy C1-6 alkyl group such as a vinyloxymethyl group, allyloxymethyl group, propenyloxymethyl group, butenyloxymethyl group or the like;
a heteroaryloxy C1-6 alkyl group such as a pyridine-2-yloxymethyl group or the like;
a C1-7 acyl C1-6 methyl group such as a formyl methyl group, acetyl methyl group, propionyl methyl group or the like;
a C1-7 acyloxy C1-6 alkyl group such as a formyloxymethyl group, acetoxymethyl group, 2-acetoxyethyl group, propionyloxymethyl group, propionyloxyethyl group or the like;
a carboxyl group C1-6 alkyl group such as a carboxyl methyl group, carboxyl ethyl group or the like;
a C1-6 alkoxycarbonyl C1-6 alkyl group such as a methoxycarbonyl methyl group, ethoxycarbonyl methyl group, n-propoxycarbonyl methyl group, i-propoxycarbonyl methyl group or the like;
a C1-7 acyl amino C1-6 alkyl group such as a formamide methyl group, acetamide methyl group, 2-acetamide ethyl group, propionyl aminomethyl group, propionyl aminoethyl group or the like;
a C1-6 alkyl aminocarbonyl C1-6 alkyl group such as a methyl aminocarbonyl methyl group, ethyl aminocarbonyl methyl group, i-propyl aminocarbonyl methyl group, t-butyl aminocarbonyl methyl group, s-butyl aminocarbonyl methyl group, n-pentyl aminocarbonyl methyl group or the like;
a C1-6 alkoxycarbonyl amino C1-6 alkyl group such as a methoxycarbonyl aminomethyl group, ethoxycarbonyl aminomethyl group, i-propoxycarbonyl aminomethyl group, t-butoxycarbonyl aminomethyl group, s-butyloxycarbonyl aminomethyl group, n-pentyloxycarbonyl aminomethyl group or the like;
a C7-11 aralkyl group such as a benzyl group, phenethyl group or the like;
a C6-10 aryl carbonyl amino C1-6 alkyl group such as a benzoyl aminomethyl group or the like; and the like.

Examples of the "C3-8 cycloalkyl group" of X include a cyclopropyl group, cyclobutyl group, cyclopentyl group, cyclohexyl group, cycloheptyl group and the like.

Examples of the "C2-6 alkenyl group" of X include a vinyl group, 1-propenyl group, 2-propenyl group, 1-butenyl group, 2-butenyl group, 3-butenyl group, 1-methyl-2-propenyl group, 2-methyl-2-propenyl group, 1-pentenyl group, 2-pentenyl group, 3-pentenyl group, 4-pentenyl group, 1-methyl-2-butenyl group, 2-methyl-2-butenyl group, 1-hexenyl group, 2-hexenyl group, 3-hexenyl group, 4-hexenyl group, 5-hexenyl group and the like.

Examples of the "substituted C2-6 alkenyl group" include a C2-6 haloalkenyl group and the like, such as a 2-chloro-1-propenyl group, 2-fluoro-1-butenyl group or the like.

Examples of "C2-6 alkynyl group" of X include an ethynyl group, 1-propynyl group, 2-propynyl group, 1-butynyl group, 2-butynyl group, 3-butynyl group, 1-methyl-2-propynyl group, 2-methyl-3-butynyl group, 1-pentynyl group, 2-pentynyl group, 3-pentynyl group, 4-pentynyl group, 1-methyl-2-butynyl group, 2-methyl-3-pentynyl group, 1-hexynyl group, 1, 1-dimethyl-2-butynyl group and the like.

Examples of the "substituted C2-6 alkynyl group" include a C2-6 haloalkynyl group and the like, such as a 4, 4-dichloro-1-butynyl group, 4-fluoro-1-pentynyl group, 5-bromo-2-pentynyl group or the like.

Examples of the "C1-6 alkoxy group" of X include a methoxy group, ethoxy group, n-propoxy group, n-butoxy group, n-pentyloxy group, n-hexyloxy group, i-propoxy group, i-butoxy group, s-butoxy group, t-butoxy group, i-hexyloxy group and the like.

Examples of the "substituted C1-6 alkoxy group" include a C1-6 haloalkoxy group and the like, such as a chloromethoxy group, dichloromethoxy group, difluoromethoxy group, trichloromethoxy group, trifluoromethoxy group, 1-fluoroethoxy group, 1, 1-difluoroethoxy group, 2, 2, 2-trifluoroethoxy group, pentafluoroethoxy group or the like.

Examples of the "substituted amino group" of X include a C1-6 alkyl-substituted amino group such as a methyl amino group, dimethyl amino group, diethyl amino group and the like.

Examples of the "C1-7 acyl group" of X include a formyl group, acetyl group, propionyl group, benzoyl group and the like.

Examples of the "substituted C1-7 acyl group" include a C1-7 haloacyl group and the like, such as a chloroacetyl group, trifluoroacetyl group, trichloroacetyl group, 4-chlorobenzoyl group or the like.

Examples of the "C1-6 alkoxycarbonyl group" of X include a methoxycarbonyl group, ethoxycarbonyl group, n-propoxycarbonyl group, i-propoxycarbonyl group and the like.

Examples of the "substituted C1-6 alkoxycarbonyl group" include
a C3-8 cycloalkyl C1-6 alkoxycarbonyl group such as a cyclopropyl methoxycarbonyl group, cyclobutyl methoxycarbonyl group, cyclopentyl methoxycarbonyl group, cyclohexyl methoxycarbonyl group, 2-methyl cyclopropyl methoxycarbonyl group, 2, 3-dimethyl cyclopropyl methoxycarbonyl group, 2-chlorocyclopropyl methoxycarbonyl group, 2-cyclopropyl ethoxycarbonyl group or the like;

a C1-6 haloalkoxycarbonyl group such as a fluoromethoxycarbonyl group, chloromethoxycarbonyl group, bromomethoxycarbonyl group, difluoromethoxycarbonyl group, dichloromethoxycarbonyl group, dibromomethoxycarbonyl group, trifluoromethoxycarbonyl group, trichloromethoxycarbonyl group, tribromomethoxycarbonyl group, 2, 2, 2-trifluoroethoxycarbonyl group, 2, 2, 2-trichloroethoxycarbonyl group, pentafluoroethoxycarbonyl group, 4-fluorobutoxycarbonyl group, 3, 3, 3-trifluoropropoxycarbonyl group, 2, 2, 2-trifluoro-1-trifluoromethyl ethoxycarbonyl group, perfluorohexyloxycarbonyl group or the like; and the like.

Examples of the "C1-6 alkylthio group" of X include a methylthio group, ethylthio group, n-propylthio group, n-butylthio group, n-pentylthio group, n-hexylthio group, i-propylthio group or the like.

Examples of the "C1-6 alkyl sulfonyl group" of X include a methyl sulfonyl group, ethyl sulfonyl group, t-butyl sulfonyl group and the like.

Examples of the "C1-6 alkoxysulfonyl group" of X include a methoxysulfonyl group, ethoxysulfonyl group, t-butoxysulfonyl group and the like.

Examples of the "C6-10 aryl group" and "heteroaryl group" of X are the same as the examples of Cy.

Examples of the substituent of the "C6-10 aryl group" and "heteroaryl group" of X include
a halogen atom such as a fluorine atom, chlorine atom, bromine atom, iodine atom or the like;
a C1-6 alkyl group such as a methyl group, ethyl group, n-propyl group, i-propyl group, n-butyl group, s-butyl group, i-butyl group, t-butyl group, n-pentyl group, n-hexyl group or the like;
a C1-6 haloalkyl group such as a chloromethyl group, chloroethyl group, trifluoromethyl group, 1, 2-dichloro-n-propyl group, 1-fluoro-n-butyl group, perfluoro-n-pentyl group or the like;
a cyano group; and the like.

Examples of the "substituted heteroaryl group" include a 2, 5-dimethyl-pyrrole-1-yl group and the like.

Examples of the "hydroxyimino C1-6 alkyl group" of X include a hydroxyiminomethyl group, (1-hydroxyimino)ethyl group, (1-hydroxyimino)propyl group and the like.

Examples of the "substituted hydroxyimino C1-6 alkyl group" include a C1-6 alkoxyimino C1-6 alkyl group such as a methoxyiminomethyl group, (1-methoxyimino)ethyl group, (1-methoxyimino)propyl group, ethoxyiminomethyl group, (1-ethoxyimino)ethyl group, (1-ethoxyimino)propyl group or the like; a C3-8 cycloalkyl C1-6 alkoxyimino C1-6 alkyl group such as a (1-cyclopropyl methoxyimino)ethyl group or the like; a C7-11 aralkyloxyimino C1-6 alkyl group such as a benzyloxyiminomethyl group, (1-benzyloxyimino)ethyl group or the like; and the like.

Examples of the "halogen atom" of X include a fluorine atom, chlorine atom, bromine atom, iodine atom and the like.

### [Formula (IV)]

Cy is preferably represented by formula (IV).

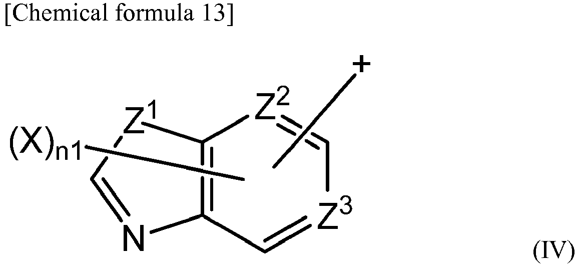

In formula (IV), + represents bonding position.
X is as defined above. n1 rerpesents the number of X and represents an integer of 0 to 4.
Z¹ reprsents an oxygen atom or sulfur atom.
Z² and Z³ each independently represents a carbon atom or nitrogen atom.

### [R¹]

In formula (I), R¹ represents an unsubstituted or substituted C1-6 alkyl group, unsubstituted or substituted C2-6 alkenyl group, unsubstituted or substituted C2-6 alkynyl group, unsubstituted or substituted C1-7 acyl group, or unsubstituted or substituted C1-6 alkoxycarbonyl group.

Examples of the "C1-6 alkyl group", "C2-6 alkenyl group", "C2-6 alkynyl group", "C1-7 acyl group" and "C1-6 alkoxycarbonyl group" of R¹ are the same as the examples of X described above.

R¹ is preferably a C1-6 alkyl group, C1-6 haloalkyl group or C2-6 alkynyl group.

### [Z]

In formula (I), Z represents an oxygen atom or sulfur atom, and preferably presents an oxygen atom.

### [Q]

In formula (I), Q represents a group represented by formula (II) or formula (III).
[Formula (II)]

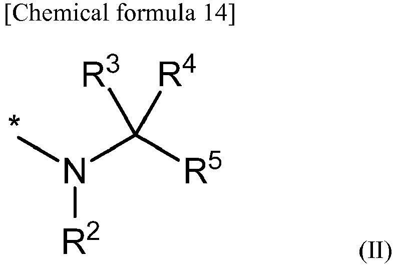

In formula (II), * represents bonding position.

### [R²]

In formula (II), R² represents a hydrogen atom, unsubstituted or substituted C1-6 alkyl group, unsubstituted or substituted C2-6 alkenyl group, unsubstituted or substituted C2-6 alkynyl group, unsubstituted or substituted C1-7 acyl group, or unsubstituted or substituted C1-6 alkoxycarbonyl group.

Examples of the "C1-6 alkyl group", "C2-6 alkenyl group", "C2-6 alkynyl group", "C1-7 acyl group", and "C1-6 alkoxycarbonyl group" of X² are the same as the examples of X described above.

### [R³, R⁴]

In formula (II), R³ and R⁴ each independently represents a hydrogen atom, unsubstituted or substituted C1-6 alkyl group, unsubstituted or substituted C2-6 alkenyl group, unsubstituted or substituted C2-6 alkynyl group, unsubstituted or substituted C6-10 aryl group, unsubstituted or substituted heteroaryl group, or cyano group. R³ and R⁴ may bond to form a ring together with the carbon atom which is bonded thereto.

Examples of the "C1-6 alkyl group", "C2-6 alkenyl group", and "C2-6 alkynyl group" of R³ and R⁴ are the same as the examples of X described above.

Examples of the "C6-10 aryl group" and "heteroaryl group" of R³ and R⁴ are the same as the examples of Cy described above.

R³ and R⁴ are preferably a C1-6 alkyl group.

The ring formed by bonding R³ and R⁴ together with the atom bonded thereto includes a cyclopropane ring, cyclobutane ring, cyclopentane ring, cyclohexane ring, oxirane ring and the like.

### [R⁵]

In formula (II), R⁵ represents an unsubstituted or substituted C1-6 alkyl group, unsubstituted or substituted C3-8 cycloalkyl group, unsubstituted or substituted C2-6 alkenyl group, unsubstituted or substituted C2-6 alkynyl group, unsubstituted or substituted C1-7 acyl group, carboxyl group, unsubstituted or substituted C1-6 alkoxycarbonyl group, unsubstituted or substituted C2-6 alkenyloxycarbonyl group, unsubstituted or substituted C2-6 alkynyloxycarbonyl group, unsubstituted or substituted aminocarbonyl group, unsubstituted or substituted C6-10 aryl group, unsubstituted or substituted heteroaryl group, unsubstituted or substituted hydroxyimino C1-6 alkyl group, or cyano group.

Examples of the "C1-6 alkyl group", "C3-8 cycloalkyl group", "C2-6 alkenyl group", "C2-6 alkynyl group", "C1-7 acyl group", and "C1-6 alkoxycarbonyl group"of R⁵ are the same as the examples of X described above.

The "C6-10 aryl group" of R⁵ may be a monocyclic ring or polycyclic ring. If the polycyclic aryl group has at least one aromatic ring, other rings may be a saturated alicyclic ring, an unsaturated alicyclic ring or an aromatic ring.

Examples of the "C6-10 aryl group" of R⁵ include a phenyl group, naphthyl group, azulenyl group, indenyl group, indanyl group, tetralinyl group or the like.

The "heteroaryl group" of R⁵ is a 5- to 10- membered aryl group which has, other than carbon atoms, 1 to 4 hetetroatoms selected from the group consisting of a nitrogen atom, oxygen atom and sulfur atom as the atoms constituting the ring. The heteroaryl group may be a monocyclic ring or polycyclic ring. If the polycyclic heteroaryl group has at least one heteroaryl gourp, other rings may be saturated alicyclic rings, unsaturated alicyclic rings or aromatic rings.

Examples of the "heteroaryl group" of R⁵ include a 5-membered heteroaryl group such as a pyrrolyl group, furyl group, thienyl group, imidazolyl group, pyrazolyl group, oxazolyl group, isoxazolyl group, thiazolyl group, isothiazolyl group, triazolyl group, oxadiazolyl group, thiadiazolyl group, tetrazolyl group or the like;
a 6-membered heteroaryl group such as a pyridyl group, pyrazinyl group, pyrimidinyl group, pyridazinyl group, triazinyl group or the like;
a condensed heteroaryl group such as an indolyl group, benzofuryl group, benzothienyl group, benzimidazolyl group, benzoxazolyl group, benzothiazolyl group, quinolyl group, isoquinolyl group, quinoxalinyl group or the like; and the like.

Examples of the substituent of the "aryl group" and "heteroaryl group" of R⁵ include the following substituents:
an amino group; a C1-6 alkyl-substituted amino group such as a methyl amino group, ethyl amino group, dimethyl amino group, diethyl amino group or the like;
a cyclic amino group such as an aziridinyl group, pyrrolidinyl group, piperidyl group, piperazinyl group, morpholinyl group or the like;
a hydrazino group; a C1-6 alkyl-substituted hydrazino group such as an N-methyl hydrazino group, N, N'-dimethyl hydrazino group or the like;
a C1-6 alkyl-substituted sulfoxiimino group such as an S, S-dimethyl sulfoxiimino group or the like;
a C1-6 alkylthio group such as a methylthio group, ethylthio group, n-propylthio group, i-propylthio group, n-butylthio group, i-butylthio group, s-butylthio group, t-butylthio group or the like;
a C1-6 haloalkylthio group such as a trifluoromethylthio group, 2, 2, 2-trifluoroethylthio group or the like;
a C1-6 alkyl sulfinyl group such as a methyl sulfinyl group, ethyl sulfinyl group, n-propyl sulfinyl group, n-butyl sulfinyl group, t-butyl sulfinyl group or the like;
a C1-6 alkyl sulfonyl group such as a methyl sulfonyl group, ethyl sulfonyl group, n-propyl sulfonyl group, i-propyl sulfonyl group, n-butyl sulfonyl group, t-butyl sulfonyl group or the like;

a C1-6 alkyl group such as a methyl group, ethyl group, n-propyl group, i-propyl group, n-butyl group, s-butyl group, i-butyl group, t-butyl group, n-pentyl group, n-hexyl group or the like;
a C1-6 haloalkyl group such as a chloromethyl group, chloroethyl group, trifluoromethyl group, 1, 2-dichloro-n-propyl group, 1-fluoro-n-butyl group, perfluoro-n-pentyl group or the like;
a C3-8 cycloalkyl group such as a cyclopropyl group, cyclobutyl group, cyclopentyl group, cyclohexyl group, cycloheptyl group or the like;
a C2-6 alkenyl group such as a vinyl group, 1-propenyl group, 2-propenyl group, 1-butenyl group, 2-butenyl group, 3-butenyl group, 1-methyl-2-propenyl group, 2-methyl-2-propenyl group, 1-pentenyl group, 2-pentenyl group, 3-pentenyl group, 4-pentenyl group, 1-methyl-2-butenyl group, 2-methyl-2-butenyl group, 1-hexenyl group, 2-hexenyl group, 3-hexenyl group, 4-hexenyl group, 5-hexenyl group or the like;
a C2-6 alkynyl group such as a ethynyl group, 1-propynyl group, 2-propynyl group, 1-butynyl group, 2-butynyl group, 3-butynyl group, 1-methyl-2-propynyl group, 2-methyl-3-butynyl group, 1-pentynyl group, 2-pentynyl group, 3-pentynyl group, 4-pentynyl group, 1-methyl-2-butynyl group, 2-methyl-3-pentynyl group, 1-hexynyl group, 1, 1-dimethyl-2-butynyl group or the like;

a hydroxyl group;
a C1-6 alkoxy group such as a methoxy group, ethoxy group, n-propoxy group, i-propoxy group, n-butoxy group, s-butoxy group, i-butoxy group, t-butoxy group or the like;
a C1-7 acyl group such as a formyl group, acetyl group, propionyl group, benzoyl group or the like;
a C1-6 alkoxycarbonyl group such as a methoxycarbonyl group, ethoxycarbonyl group, n-propoxycarbonyl group, i-propoxycarbonyl group or the like;
a C6-10 aryl group such as a phenyl group, naphthyl group or the like;
a 5-membered heteroaryl group such as a pyrrolyl group, furyl group, thienyl group, imidazolyl group, pyrazolyl group, oxazolyl group, isoxazolyl group, thiazolyl group, isothiazolyl group, triazolyl group, oxadiazolyl group, thiadiazolyl group, tetrazolyl group or the like;
a 6-membered heteroaryl group such as a pyridyl group, pyrazinyl group, pyrimidinyl group, pyridazinyl group, triazinyl group or the like;
an oxo group, a nitro group; a cyano group;
a halogen atom such as fluorine atom, chlorine atom, bromine atom, iodine atom or the like.

Examples of the "C2-6 alkenyloxycarbonyl group" of R⁵ include an ethenyloxycarbonyl group, 1-methyl-2-propenyloxycarbonyl group, 2-methyl-1-propenyloxycarbonyl group and the like.

Examples of the "C2-6 alkynyloxycarbonyl group"of R⁵ include an ethynyloxycarbonyl group, propargyloxycarbonyl group, 1-methyl propargyloxycarbonyl group, 2-butynyloxycarbonyl group and the like.

Examples of the "substituted aminocarbonyl group" of R⁵ include a C1-6 alkyl aminocarbonyl group such as a methyl aminocarbonyl group, ethyl aminocarbonyl group, i-propyl aminocarbonyl group, t-butyl aminocarbonyl group, s-butyl aminocarbonyl group, n-pentyl aminocarbonyl group or the like; a di C1-6 alkyl aminocarbonyl group such as a dimethyl aminocarbonyl group, diethyl aminocarbonyl group or the like; a C3-8 cycloalkyl aminocarbonyl group such as a cyclopropyl aminocarbonyl group, cyclopentyl aminocarbonyl group, cyclohexyl aminocarbonyl group or the like; a C2-6 alkynyl aminocarbonyl group such as a 2-propynyl aminocarbonyl group or the like; a phenyl aminocarbonyl group, N-phenyl-N-methyl aminocarbonyl group; a C1-6 alkoxy C1-6 alkyl aminocarbonyl group such as a methoxyethyl aminocarbonyl group or the like; a C1-6 haloalkyl aminocarbonyl group such as a 2, 2, 2-trifluoroethyl aminocarbonyl group or the like; a C3-8 cycloalkyl C1-6 alkyl aminocarbonyl group such as a cyclopropyl methyl aminocarbonyl group or the like; a C7-11 aralkyl aminocarbonyl group such as a benzyl aminocarbonyl group or the like; a 1-substituted cyclic amine carbonyl group such as a piperidine-1-yl carbonyl group or the like; and the like.

Examples of the "hydroxyimino C1-6 alkyl group" of R⁵ include a hydroxyiminomethyl group, (1-hydroxyimino)ethyl group, (1-hydroxyimino)propyl group and the like.

Examples of the "substituted hydroxyimino C1-6 alkyl group" include a C1-6 alkoxyimino C1-6 alkyl group such as a methoxyiminomethyl group, (1-methoxyimino)ethyl group, (1-methoxyimino)propyl group, ethoxyiminomethyl group, (1-ethoxyimino)ethyl group, (1-ethoxyimino)propyl group or the like; a C3-8 cycloalkyl C1-6 alkoxyimino C1-6 alkyl group such as a (1-cyclopropyl methoxyimino)ethyl group or the like; a C7-11 aralkyloxyimino C1-6 alkyl group such as a benzyloxyiminomethyl group, (1-benzyloxyimino)ethyl group or the like; and the like.

### [Formula (V)]

R⁵ of formula (II) is preferably a pyridyl group substituted with X¹ and X². Namely, Q is preferably a group represented by formula (V).

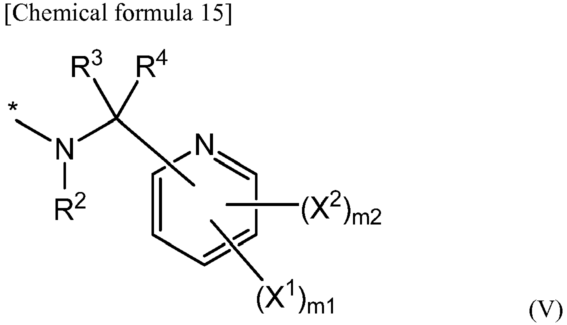

In formula (V), * represents bonding position.
R², R³ and R⁴ are as defined above.

### [X¹]

In formula (V), X¹ represents an unsubstituted or substituted amino group, an unsubstituted or substituted C1-6 alkylthio group, an unsubstituted or substituted C1-6 alkyl sulfinyl group, or an unsubstituted or substituted C1-6 alkyl sulfonyl group. m1 represents the number of X¹ and represents an integer of 1-4. When m1 is 2 or more, X¹s may be the same as or different from each other.

Examples of the "substituted amino group" of X¹ include a C1-6 alkyl-substituted amino group such as a methyl amino group, ethyl amino group, dimethyl amino group, diethyl amino group or the like; a cyclic amino group such as an aziridinyl group, pyrrolidinyl group, piperidyl group, piperazinyl group, morpholinyl group or the like; a hydrazino group; a C1-6 alkyl-substituted hydrazino group such as a N-methyl hydrazino group, N, N'-dimethyl hydrazino group or the like; a C1-6 alkyl-substituted sulfoxi imino group such as an S, S-dimethyl sulfoxi imino group or the like; and the like.

Examples of the "C1-6 alkylthio group" of X¹ include a methylthio group, ethylthio group, n-propylthio group, i-propylthio group, n-butylthio group, i-butylthio group, s-butylthio group, t-butylthio group and the like.

Examples of the "sustituted C1-6 alkylthio group" include a C1-6 haloalkylthio group such as a trifluoromethylthio group, 2, 2, 2-trifluoroethylthio group or the like.

Examples of the "C1-6 alkyl sulfinyl group" of X¹ include a methyl sulfinyl group, ethyl sulfinyl group, n-propyl sulfinyl group, i-propyl sulfinyl group, n-butyl sulfinyl group, i-butyl sulfinyl group, s-butyl sulfinyl group, t-butyl sulfinyl group and the like.

Examples of the "C1-6 alkyl sulfonyl group" of X¹ include a methyl sulfonyl group, ethyl sulfonyl group, n-propyl sulfonyl group, i-propyl sulfonyl group, n-butyl sulfonyl group, t-butyl sulfonyl group and the like.

### [X²]

In formula (V), X² represents an unsubstituted or substituted C1-6 alkyl group, an unsubstituted or substituted C3-8 cycloalkyl group, an unsubstituted or substituted C2-6 alkenyl group, an unsubstituted or substituted C2-6 alkynyl group, hydroxy group, an unsubstituted or substituted C1-6 alkoxy group, an unsubstituted or substituted C1-7 acyl group, an unsubstituted or substituted C1-6 alkoxycarbonyl group, an unsubstituted or substituted C6-10 aryl group, an unsubstituted or substituted heteroaryl group, oxo group, nitro group, cyano group, or halogen atom. m2 represents the number of X² and represents an integer of 0 to 3. When m2 is 2 or more, X²s may be the same as or different from each other. The sum of m1 and m2 is 4 or less.

Examples of the "C1-6 alkyl group", "C3-8 cycloalkyl group", "C2-6 alkenyl group", "C2-6 alkynyl group", "C1-6 alkoxy group", "C1-7 acyl group", "C1-6 alkoxycarbonyl group", "C6-10 aryl group", "heteroaryl group", and "halogen atom" of X² are the same as the examples of X described above.

### [Formula (VI)]

R⁵ of formula (II) is preferably a pyridyl group substituted with X¹ and X². Namely, Q is preferably a group represented by formula (VI).

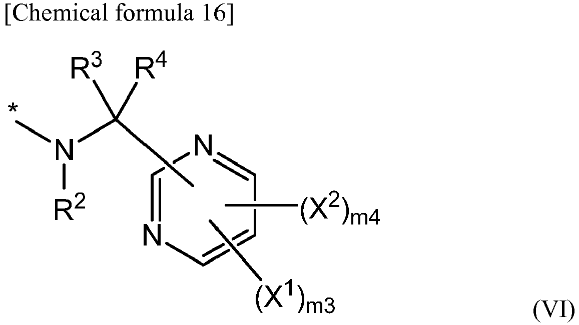

In formula (VI), * represents bonding position.
R², R³, R⁴, X¹ and X² are as defined above.
m3 represents the number of X¹ and represents an integer of 1 to 3. When m3 is 2 or more, X¹s may be the same as or different from each other.
m4 represents the number of X² and represents an integer of 0 to 2. When m4 is 2 or more, X²s may be the same as or different from each other. The sum of m3 and m4 is 3 or less.

### [Formula (VII)]

R⁵ of formula (II) is preferably a phenyl group substituted with X¹ and X². Namely, Q is preferably a group represented by formula (VII).

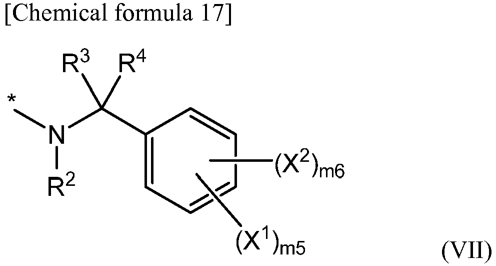

In formula (VII), * represent bonding position.
R², R³, R⁴, X¹, and X² are as defined above.
m5 represents the number of X¹ and represents an integer of 1 to 5. When m5 is 2 or more, X¹s may be the same as or different from each other.
m6 represents the number of X² and represents an integer of 0 to 4. When m6 is 2 or more, X²s may be the same as or different from each other. The sum of m5 and m6 is 5 or less.

### [Formula (III)]

Other than the groups represented by formulas (V), (VI), and (VII), Q may also be a group represented by formula (III).

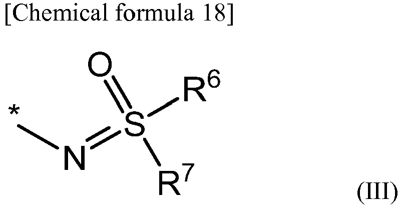

In formula (III), * represents bonding position.

### [R⁶, R⁷]

In formula (III), R⁶ and R⁷ each independently represents an unsubstituted or substituted C1-6 alkyl group, an unsubstituted or substituted C3-8 cycloalkyl group, an unsubstituted or substituted C2-6 alkenyl group, an unsubstituted or substituted C2-6 alkynyl group, an unsubstituted or substituted C6-10 aryl group, or an unsubstituted or substituted heteroaryl group. R⁶ and R⁷ may bond to form a ring together with the sulfur atom which is bonded thereto.

Examples of the "C1-6 alkyl group", "C3-8 cycloalkyl group", "C2-6 alkenyl group", and "C2-6 alkynyl group" of R⁶ and R⁷ are the same as the examples of X described above.

Examples of the "C6-10 aryl group" and "heteroaryl group" of R⁶ and R⁷ are the same as the examples of Cy described above.

Examples of the substituent of the "C6-10 aryl group" and "heteroaryl group" of R⁶ and R⁷ include
a halogen atom such as a fluorine atom, chlorine atom, bromine atom, iodine atom or the like;
a C1-6 alkyl group such as a methyl group, ethyl group, n-propyl group, i-propyl group, n-butyl group, s-butyl group, i-butyl group, t-butyl group, n-pentyl group, n-hexyl group or the like;
a C1-6 haloalkyl group such as a chloromethyl group, chloroethyl group, trifluoromethyl group, 1, 2-dichloro-n-propyl group, 1-fluoro-n-butyl group, perfluoro-n-pentyl group or the like;
a cyano group; and the like.

Examples of the "ring" formed by bonding R⁶ and R⁷ together with the sulfur atom which is bonded thereto include a tetrahydrothiophene ring, tetrahydrothiopyran ring, oxathiane ring and the like.

### [Salt of aryloxyurea compound]

The salt of the aryloxyurea comound of the present invention is a salt of compound (I). There are no particular limitations on the salt provided that it is an agriculturally and horticulturally allowable salt. Examples of the salt include salts of inorganic acids such as a hydrochloric acid, sulfuric acid or the like; salts of organic acids such as an acetic acid, lactic acid or the like; salts of alkaline metals such as a lithium, sodium, potassium or the like; salts of alkaline earth metals such as a calcium, magnesium or the like; salts of transition metals such as an iron, copper or the like; salts of organic bases such as an ammonia, triethylamine, tributylamine, pyridine, hydrazine or the like; and the like. The salts of aryloxyurea compound may be produced by well-known methods using compound (I).

### [Production method]

The following provides an explanation of a production method of the aryloxyurea compound of the present invention.
1) The production method shown in the following scheme can be an example of the first production method.

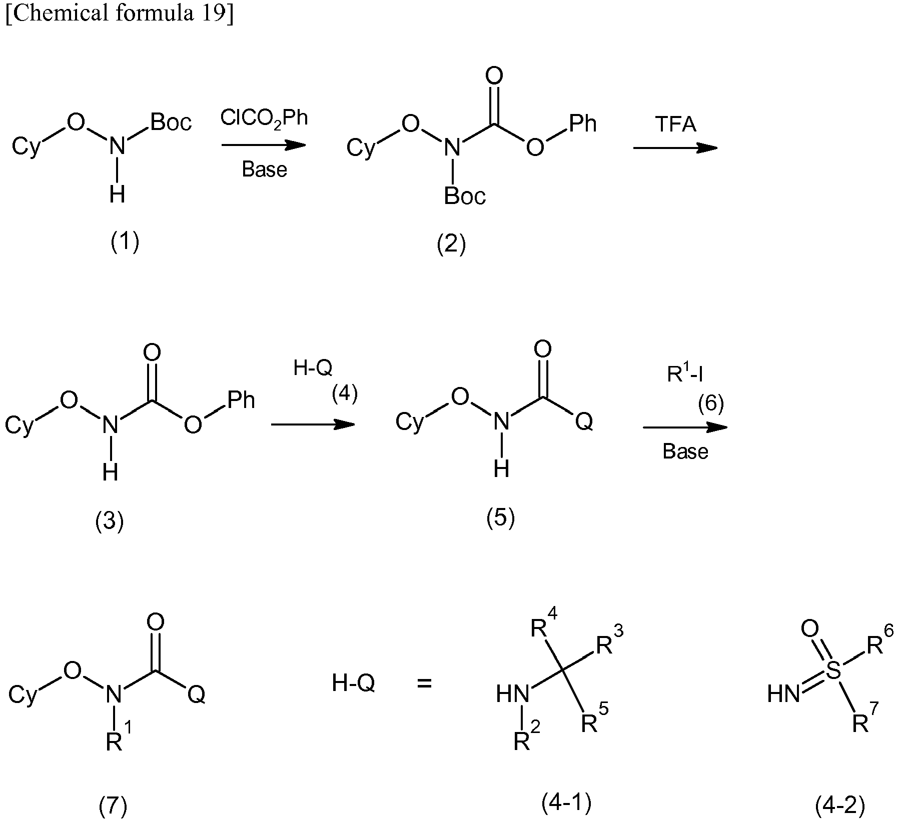

a) A diester compound represented by formula (2) (hereinafter, may be referred to as "compound (2)") is obtained by reacting an aryloxyamine compound represented by formula (1) (hereinafter, may be referred to as "compound (1)") with chloroformic acid phenyl ester ester in the presence of a base. Next, an N-aryloxycarbamic acid phenyl ester represented by formula (3) (hereinafter, may be referred to as "compound (3)") is produced by performing de-Boc reaction in the presence of an acid. In formulas (1) to (3), Cy is as defined as above.

The amount of chloroformic acid phenyl ester is generally 1 to 2 mol, and preferably 1.0 to 1.2 mol with respect to 1 mol of compound (1). Although the reaction between compound (1) and chloroformic acid phenyl ester may be performed in the absence of a base, it is preferable to perform the reaction in the presence of a base. Examples of the base include pyridine, triethylamine, potassium hydroxide and the like. The amount of the base is generally 1 to 2 mol with respect to 1 mol of compound (1). The reaction may be performed in a solvent. There are no particular limitations on the solvent provided that it is inactive against the reaction. Examples of the solvent include ether type solvents such as dioxane, 1, 2-dimethoxyethane, tetrahydrofuran; aromatic hydrocarbon type solvents such as toluene, benzene, xylene; aliphatic hydrocarbon type solvents such as n-pentane, n-hexane, n-heptane; halogenated hydrocarbon type solvents such as dichloromethane, chloroform, carbon tetrachloride, 1, 2-dichloroethane; amide type solvents such as N, N-dimethyl formamide, N, N-dimethyl acetamide, N-methyl pyrrolidone; nitrile type solvents such as acetonitrile, benzonitrile; and mixed solvents including two or more of these solvents; and the like. Although there are no particular limitations on the amount of the solvent, it is generally 1 to 100 ml with respect to 1 g of compound (1). The reaction temperature ranges from -20 °C to the boiling point of the solvent. Although the reaction time varies according to the reaction scale, it is generally from minutes to hours.

Next, the de-Boc reaction is performed in the presence of an acid. Examples of the acid include inorganic acids such as hydrochloric acid, sulfuric acid, nitric acid; acetic acids, trifluoroacetic acids, methane sulfonic acids, p-toluene sulfonic acids and the like. Among these examples, trifluoroacetic acid is preferable. The amount of the acid is generally 1 to 20 mol with respect to 1 mol of compound (2). The de-Boc reaction may be performed in a solvent. There are no particular limitations on the solvent provided that it is inactive against the reaction. Examples of the solvent are the same as the examples of the solvent used for producing compound (2). Although there are no particular limitations on the amount of the solvent, it is generally 1 to 100 ml with respect to 1 g of compound (2). The reaction temperature ranges from room temperature to the boiling point of the solvent. Although the reaction time varies according to the reaction scale, it is generally from minutes to hours.

b) an aryloxyurea compound represented by formula (5) (hereinafter, may be referred to as "compound (5)") is produced by reacting compound (3) with a compound represented by formula (4) (hereinafter, may be referred to as "compound (4)"). compound (4) may be an amine compound represented by formula (4-1) (hereinafter, may be referred to as "compound (4-1)"), or a sulfoximine compound represented by formula (4-2) (hereinafter, may be referred to as "compound (4-2)"). In formula (4) and formula (5), Q and Cy are as defined above. In formula (4-1) and formula (4-2), R², R³, R⁴, R⁵, R⁶ and R⁷ are as defined above. The amount of compound (4) is generally 1 to 2 mol, and preferably 1.0 to 1.2 mol with respect to 1 mol of compound (3). The reaction may be performed in a solvent. There are no particular limitations on the solvent provided that it is inactive against the reaction. Examples of the solvent are the same as the examples of the solvent used for producing compound (2). Although there are no particular limitations on the amount of the solvent, it is generally 1 to 100 ml with respect to 1 g of compound (3). In addition, when performing a reaction with compound (4-2), it is preferable to perform in the presence of a base. Examples of the base include pyridine, triethylamine, potassium hydroxide and the like. The amount of the base is generally 1 to 2 mol with respect to 1 mol of compound (3). The reaction temperature ranges from room temperature to the boiling point of the solvent. Although the reaction time varies according to the reaction scale, it is generally from minutes to hours.

b) an aryloxyurea compound represented by formula (7) (hereinafter, may be referred to as "compound (7)"), which is a target compound, is produced by reacting compound (5) with an iodinated compound represented by formula (6) (hereinafter, may be referred to as "compound (6)") in the presence of a base. In formula (6) and formula (7), Q, R¹ and Cy are as defined above. The amount of compound (6) is generally 1 to 2 mol, and preferably 1.0 to 1.2 mol with respect to 1 mol of compound (5). Examples of the base include pyridine, triethylamine, potassium hydroxide, calcium carbonate and the like. The amount of the base is generally 1 to 2 mol with respect to 1 mol of compound (5). The reaction may be performed in a solvent. There are no particular limitations on the solvent provided that it is inactive against the reaction. Examples of the solvent are the same as the examples of the solvent used for producing compound (2). Although there are no particular limitations on the amount of the solvent, it is generally 1 to 100 ml with respect to 1 g of compound (5). The reaction temperature ranges from -20 °C to the boiling point of the solvent. Although the reaction time varies according to the reaction scale, it is generally from minutes to hours.

2) The production method shown in the following scheme can be an example of the second production method.

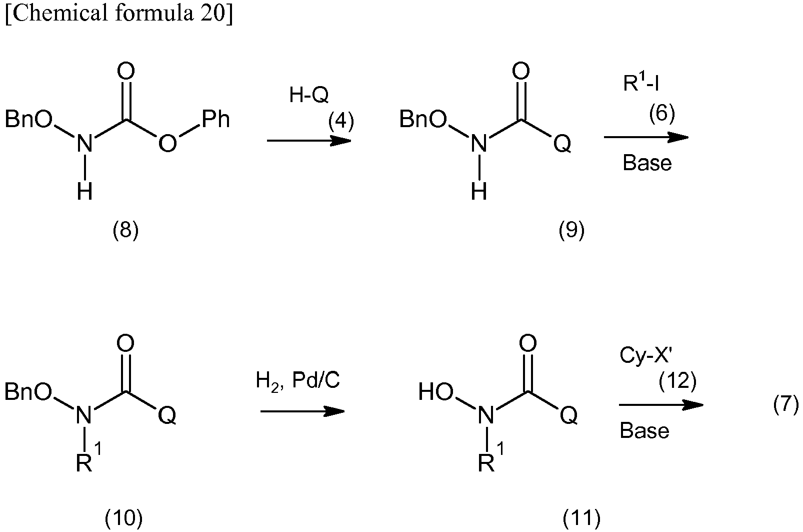

a) A benzyloxyurea compound represented by formula (9) (hereinafter, may be referred to as "compound (9)") is produced by reacting an N-benzyloxycarbamic acid phenyl ester (hereinafter, may be referred to as "compound (8)") able to be produced by well-known methods with compound (4). In formula (9), Q is as defined above. The amount of compound (4) is generally 1 to 2 mol, and preferably 1.0 to 1.2 mol with respect to 1 mol of compound (8). The reaction may be performed in a solvent. There are no particular limitations on the solvent provided that it is inactive against the reaction. Examples of the solvent are the same as the examples of the solvent used for producing compound (2). Although there are no particular limitations on the amount of the solvent, it is generally 1 to 100 ml with respect to 1 g of compound (8). In addition, when performing a reaction with compound (4-2), it is preferable to perform in the presence of a base. Examples of the base include pyridine, triethylamine, potassium hydroxide and the like. The amount of the base is generally 1 to 2 mol with respect to 1 mol of compound (8). The reaction temperature ranges from room temperature to the boiling point of the solvent. Although the reaction time varies according to the reaction scale, it is generally from minutes to hours.

b) A benzyloxyurea compound represented by formula (10) (hereinafter, may be referred to as "compound (10)") is produced by reacting compound (9) with compound (6), followed by debenzylating by catalytic reduction to produce an oxyurea compound represent by formula (11) (hereinafter, may be referred to as "compound (11)"). In formula (10) and (11), Q and R¹ are as defined above. The amount of compound (6) is generally 1 to 2 mol, and preferably 1.0 to 1.2 mol with respect to 1 mol of compound (9). The reaction may be performed in a solvent. There are no particular limitations on the solvent provided that it is inactive against the reaction. Examples of the solvent are the same as the examples of the solvent used for producing compound (2). Although there are no particular limitations on the amount of the solvent, it is generally 1 to 100 ml with respect to 1 g of compound (9). The reaction temperature ranges from -20 °C to the boiling point of the solvent. Although the reaction time varies according to the reaction scale, it is generally from minutes to hours.

Next, the debenzylating reaction is performed by catalytic reduction using a palladium catalyst or the like. Examples of the palladium catalyst include palladium black, palladium carbon and the like. The amount of palladium catalyst is generally 0.01 to 0.1 mol with respect to 1 mol of compound (10). The reaction is performed in a solvent. There are no particular limitations on the solvent. Examples of the solvent are the same as the examples of the solvent used for producing compound (2). The examples of the solvent also include alcohol type solvents such as methanol, ethanol, n-propanol, and the like. Although there are no particular limitations on the amount of the solvent, it is generally 1 to 100 ml with respect to 1 g of compound (10). The reaction temperature ranges from room temperature to the boiling point of the solvent. Although the reaction time varies according to the reaction scale, it is generally from minutes to hours.

c) A target compound (7) is produced by reacting compound (11) with an aryl compound represented by formula (12) (hereinafter, may be referred to as "compound (12)") in the presence of a base. In formula (12), Cy is as defined above, X' represents a fluorine atom or the like. The amount of compound (12) is generally 1 to 2 mol, and preferably 1.0 to 1.2 with respect to 1 mol of compound (11). Examples of the base include metal hydroxide such as lithium hydroxide, sodium hydroxide, potassium hydroxide, magnesium hydroxide, calcium hydroxide; metal alkoxide such as sodium methoxide, sodium ethoxide, potassium methoxide, potassium ethoxide, potassium t-butoxide; metal hydride such as sodium hydride, potassium hydride, calcium hydride; organic base such as triethylamine, diisopropyl ethylamine, pyridine, 1, 8-diazabicyclo[5.4.0]undecene-7-ene (DBU), 1, 4-diazabicyclo[2.2.2]octane. The amount of the base is generally 1 to 2 mol with respect to 1 mol of compound (11). The reaction is performed in a solvent. There are no particular limitations on the solvent provided that it is inactive against the reaction. Examples of the solvent are the same as the examples of the solvent used for producing compound (2). Although there are no particular limitations on the amount of the solvent, it is generally 1 to 100 ml with respect to 1 g of compound (11). The reaction temperature ranges from -20 °C to the boiling point of the solvent. Although the reaction time varies according to the reaction scale, it is generally from minutes to hours.

After the reactions are completed, an ordinary post-treatment procedure, and if needed, known methods such as distillation, recrystallization or column chromatography, can be carried out to purify and isolate the target compound.

The structure of the tarrget compound can be identified and confirmed by a known analysis such as IR spectroscopy, NMR spectroscopy, mass spectroscopy or elementary analysis.

### [Formula (VIII)]

Compound (10) of the second production method is preferably a benzyloxyurea compound represented by formula (VIII).

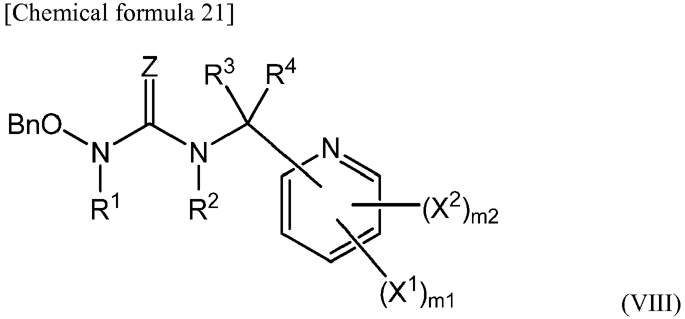

In formula (VIII), R¹, R², R³, R⁴, Z, X¹, m1, X², m2 are as defiend above.

The aryloxyurea compound or salt thereof according to the present invention (hereinafter, may be referred to as "the compound of the present invention") causes little chemical damage, demonstrates low levels of toxicity in fish and warm-blooded animals, and has a particularly high degree of safety. Since the compound of the present invention is effective for preventing various insents or acarus, it is useful as an active ingredient of the acaricides or insecticides.

### [Pest control agent]

The pest control agent of the present invention contains as an active ingredient at least one type of compound selected from the compounds of the present invention.

Although the pest control agent of the present invention may only contain the compound of the present invention, it may also contain carriers such as a solid carrier, liquid carrier or gaseous carrier. In addition, the pest control agent of the present invention may have the compound of the present invention impregnated in a base material such as a porous ceramic plate or non-woven fabric. Moreover, a surfactant or other adjunct may be added as necessary.

The pest control agent according to the present invention can be formulated into a form able to be typically adopted in agricultural chemicals, namely in the form of a water-dispersible powder, granules, powder, emulsion, water-soluble powder, suspension, granular water-dispersible powder, flowable preparation, microcapsules, aerosol, fog, heat transpiration agent, fumigant or poison bait.

Examples of the additives and carriers used when formulating a solid preparation include vegetable powders such as soybean powder or flour, mineral fine powders such as diatomaceous earth, apatite, plaster, talc, bentonite, pyrophyllite or clay; and organic and inorganic compounds such as sodium benzoate, urea or sodium sulfate.

Examples of the liquid carriers used when formulating liquid preparations include kerosene, xylene, and petroleum-based aromatic hydrocarbon, cyclohexane, cyclohexanone, dimethylformamide, dimethylsulfoxide, alcohols, acetone, trichloroethylene, methyl isobutyl ketone, mineral oils, vegetable oils, water and the like.

Examples of the gaseous carriers used when formulating propellants include butane gas, LPG, dimethyl ether and carbon dioxide gas.

Examples of the base materials of poison bait include bait components such as grain powder, vegetable oil, sugar or crystalline cellulose, antioxidants such as dimethylhydroxytoluene or nordihydroguaiaretic acid, preservatives such as dehydroacetic acid, accidental swallowing preventives for small children and pets such as cayenne pepper powder, insect-attracting fragrances such as cheese fragrance or onion fragrance.

A surfactant can be added according to need in order to obtain a uniform and stable form during formulation. There are no limitations on the surfactants to be added. Examples of surfactants include nonionic surfactants such as polyoxyethylene alkyl phenyl ethers, polyoxyethylene alkyl ethers, polyoxyethylene higher fatty acid esters, polyoxyethylene sorbitan higher fatty acid esters or polyoxyethylene tristyryl phenyl ethers; sulfate esters of polyoxyethylene alkyl phenyl ethers, alkyl benzene sulfonate, higher fatty alcohol sulfate, alkylnaphthalene sulfonates, polycarboxylates, lignin sulfonates, formaldehyde condensates of alkylnaphthalene sulfonates and isobutylene-maleic anhydride copolymers.

The amount of the compound of the present invention contained in the preparation is preferably 0.01 to 90% by weight, and more preferably 0.05 to 85% by weight.

The water-dispersible powder, emulsion, flowable preparation, water-soluble powder, granular water-dispersible powder which are obtained in this manner can be prepared in the form of a solution, suspension or emulsion and diluted with water to a prescribed concentration to spray onto plants or soil, or in the case of using them in the form of powder or granules, they can be sprayed directly onto plants or soil.

In addition, in the case of using the pest control agent of the present invention as an acaricide for epidemic prevention, a preparation that is supplied in the form of oil solution, aerosol, fog, poison bait or miticidal sheet can be directly used.

In addition, in the case of using the pest control agent of the present invention to prevent animal parasitic acari of livestock such as cows or pigs and pets such as dogs or cats, the compound of the present invention can be used at a ratio of 0.01 mg to 1000 mg per 1 kg of host animal. The pesiticide of the present invention can be applied on the animals using a known veterinary method. Examples of such methods include methods in which the acaricide is administered to an animal by a tablet, capsule, immersion liquid, food additive, suppository or injection (intramuscular, subcutaneous, intravenous or intraabdominal injection) when administered for the purpose of systemic control, methods in which an oily or aqueous liquid preparation is administered by spraying, pouring on or spotting on when administered for the purpose of non-systemic control, and methods in which the acaricide is mixed with a resin and the kneaded product is molded into a suitable shape such as that of a collar or ear tag which is then attached to the animal.

The pest control agent of the present invention can be mixed or combined with fungicides, other insecticides or acaricides, nematocides, soil pesticides, plant regulators, synergists, fertilizers, soil improvers or animal feeds and the like.

The following lists typical examples of fungicides, other insecticides or acaricides, nematocides, soil pesticides, anthelmintic agents, and plant regulators able to be used by mixing or combination with the pest control agent of the present invention.

Insecticides, acaricides, nematocides, soil pesticides and antiparasitic agent:
(1) organic (thio)phosphate-based: such as acephate, azamethiphos, azinphos-methyl, azinphos-ethyl, bromophos-ethyl, bromfenvinphos, BRP, chlorpyriphos, chlorpyriphos-methyl, chlorpyrifos-ethyl, chlorfenvinphos, cadusafos, carbophenothion, chlorethoxyfos, chlormephos, coumaphos, cyanofenphos, cyanophos, CYAP, diazinon, dichlorvos, dicrotophos, dimethoate, disulfoton, demeton-S-methyl, dimethylvinfos, demeton-S-methyl sulphone, dialifos, diazinon, dichlofenthion, dioxabenzofos, disulfoton, ethion, ethoprophos, etrimfos, EPN, fenamiphos, fenitrothion, fenthion, fensulfothion, flupyrazofos, fonofos, formothion, fosmethilan, heptenophos, isazophos, iodofenphos, isofenphos, isoxathion, iprobenfos, malathion, mevinphos, methamidophos, methidathion, monocrotophos, mecarbam, methacrifos, naled, omethoate, oxydemeton-metyl, paraoxon, parathion, parathion-methyl, phenthoate, phosalone, phosmet, phosphamidon, phorate, phoxim, pirimiphos-methyl, pirimiphos-ethyl, profenofos, prothiofos, fosthiazete, phosphocarb, propaphos, propetamphos, prothoate, pyridafenthion, pyraclofos, quinalphos, salithion, sulprofos, sulfotep, tetrachlorvinphos, terbufos, triazophos, trichlorfon, tebupirimfos, temephos, thiomethon, vamidothion, pyraclofos;

(2) carbamate-based: alanycarb, aldicarb, bendiocarb, benfuracarb, carbaryl, carbofuran, carbosulfan, fenoxycarb, fenothiocarb, methiocarb, methomyl, oxamyl, pirimicarb, propoxur, thiodicarb, triazamate, ethiofencarb, fenobucarb, MIPC, MPMC, MTMC, pyridafenthion, furathiocarb, XMC, aldoxycarb, allyxycarb, aminocarb, bendiocarb, bufencarb, butacarb, butocarboxim, butoxycarboxim, cloethocarb, dimetilan, formetanate, isoprocarb, metam-sodium, metolcarb, thiofanox, trimethacarb, xylycarb;
(3) pyrethroid-based: allethrin, bifenthrin, cyfluthrin, β-cyfluthrin, cyhalothrin, lambdacyhalothrin, cyphenothrin, cypermethrin, alphacypermethrin, betacypermethrin, zetacypermethrin, deltamethrin, esfenvalerate, etofenprox, fenpropathrin, fenvalerate, imiprothrin, permethrin, prallethrin, pyrethrin, pyrethrin I, pyrethrin II, resmethrin, silafluofen, fluvalinate, tefluthrin, tetramethrin, tralomethrin, transfluthrin, profluthrin, dimefluthrin, acrinathrin, cycloprothrin, halfenprox, flucythrinate, bioallethrin, bioethanomethrin, biopermethrin, bioresmethrin, transpermethirn, empenthrin, fenfluthrin, fenpirithrin, flubrocythrinate, lufenoprox, flumethrin, metofluthrin, phenothrin, protrifenbute, pyresmethrin, terallethrin;

(4) growth regulators:
   (a) chitin synthesis inhibitors: chlorfluazuron, diflubenzuron, flucycloxuron, flufenoxuron, hexaflumuron, lufenuron, novaluron, teflubenzuron, triflumuron, bistrifluron, nobifumuron, buprofezin, hexythiazox, etoxazole, clofentezine, fluazuron, penfluron;
   (b) ecdysone antagonists: halofenozide, methoxyfenozide, tebufenozide, chromafenozide, azadirachtin;
   (c) juvenile hormone-like substances: pyriproxyfen, methoprene, diofenolan, epofeneonane, hydroprene, kinoprene, triprene;
   (d) lipid biosynthesis inhibitors: spirodiclofen, spiromesifen, spirotetramat, flonicamid;

(5) nicotine receptor agonist/antagonist compounds: acetamiprid, clothianidine, dinotefuran, imidacloprid, nitenpyram, thiacloprid, thiamethoxam, nithiazine, nicotine, bensultap, cartap; flupyradifurone;
(6) GABA antagonist compounds:
   (a) acetochlor, ethiprole, fipronil, vaniliprole, pyrafluprole, pyriprole;
   (b) organochlorine compound: camphechlore, chlordane, endosulfan, HCH, γ-HCH, heptachlor, methoxychlor;
(7) macrocyclic lactone insecticides: abamectin, emamectin, milbemectin, lepimectin, spinosad, ivermectin, seramectin, doramectin, epinomectin, moxidectin; milbemycin; milbemycin oxime;
(8) METI I compounds: fenazaquin, pyridaben, tebufenpyrad, tolfenpyrad, flufenirim, hydramethylnon, fenpyroxymate, pyrimidifen, dicofol;
9) METI II and III compounds: acequinocyl, fluacrypyrim, rotenone;
(10) uncoupling agent compounds: chlorfenapyr, dinobuton, dinocap, DNOC;

11) oxidative phosphorylation inhibitor compounds: cyhexitin, diafenthiuron, fenbutatin oxide, propargite, azocyclotin;
(12) molting disruption compounds: cyromazine;
(13) mixed function oxidase inhibitor compounds: piperonyl butoxide;
(14) sodium channel blocker compounds: indoxacarb, metaflumizone;
(15) microbial pesticides: BT agents, insect pathogen viral agents, insect pathogen fungal agents, nematode pathogen fungal agents, bacillus, beauveria bassiana, metarhizium anisopliae, paecilomyces, thuringiensin, verticillium;
(16) latrophilin receptor agonist: depsipeptide, cyclodepsipeptide, 24-membered cyclodepsipeptide, emodepside;
(17) octopamine agonist: amitraz;
(18) ryanodine derivative agonist: flubendiamide, chlorantraniliprole, cyantraniliprole;
(19) magnesium-stimulated ATPase inhibitor: thiocyclarm, thiosultap, nereistoxin;
(20) antifeedant: pymetrozine;
(21) acari growth inhibitor: clofentezine, etoxazole;
(22) other compounds: benclothiaz, bifenazate, pyradalyl, sulfur, cyenopyrafen, cyflumetofen, amidoflumet, tetradifon, chlordimeform, 1, 3-dichloropropene, DCIP, phenisobromolate, benzomate, methaldehyde, spinetoram, pyrifluquinzaon, benzomate, bromopropylate, quinomethionate, chlorobenzilate, chloropicrin, chlothiazoben, dicyclanil, fenoxacrim, fentrifanil, flubenzimine, fluphenazine, gossyplure, japonilure, metoxadiazone, oil, potassium oleate, sulfluramid, tetrasul, triarathene; afidopyropen, pyflubumide, flometoquin, flufiprole, fluensulfone, meperfluthrin, tetramethylfluthrin, sulfoxaflor, imicyafos, tralopyril, diflovidazin, dimefluthrin, methylneodecanamide;
(23) antiparastic agent
   (a) benzimidazoles: fenbendazole, albendazole, triclabendazole, oxibendazole;
   (b) salicylanilides: closantel, oxyclozanide;
   (c) substituted phenols: nitroxinil;
   (d) pyridines: pyrantel;
   (e) imidazothiazoles: levamisole;
   (f) tetrahydropyrimidines: praziquantel;
   (g) other antiparastic agents: cyclodien, riania, clorsulon, metronidazole, demijitorazu;

Fungicides:
(1) benzimidazole-based: benomyl, carbendazim, fuberidazole, thiabendazole, methylthiophanate; chlorfenazole;
(2) dicarboxyimide-based: chlozolinate, iprodione, procymidone, vinclozolin;
(3) DMI fungicides: imazalil, oxpoconazole, pefurazoate, prochloraz, triflumizole, triforine, pyrifenox, fenarimol, nuarimol, azaconazole, bitertanol, bromconazole, cyproconazole, difenoconazole, diniconazole, epoxyconazole, fenbuconazole, fluquinconazole, flusilazole, flutriafol, hexaconazole, imibenconazole, ipuconazole, metconazole, myclobutanil, penconazole, propiconazole, prothioconazole, simeconazole, tebuconazole, tetraconazole, triadimefon, triadimenol, triticonazole, etaconazole, furconazole-cis; diclobutrazol, diniconazole-M, dodemorph-acetate, furconazole, imazalil-sulphate, nafuchifen, uniconazole P, viniconazole, voriconazole;

(4) phenylamide-based: benalaxyl, furalaxyl, metalaxyl, metalaxyl-M, oxadixyl, ofurace; benalaxyl-M, clozylacon;
(5) amine-based: aldimorph, dodemorph, fenpropimorph, tridemorph, fenpropidine, piperalin, spiroxamine;
(6) phosphothiolate-based: EDDP, iprobenfos, pyrazophos;
(7) dithiolane-based: isoprothiolane;
(8) carboxamide-based: benodanil, boscalid, carboxin, fenfuran, flutolanil, furametpyr, mepronil, oxycarboxin, penthiopyrad, thifluzamide; bixafen, isopyrazam, penflufen, fluxapyroxad, Sedaxan;
(9) hydroxy(2-amino)pyrimidine-based: bupirimate, dimethirimol, ethirimol;

(10) AP fungicides (anilinopyrimidines-based): cyprodinil, mepanipyrim, pyrimethanil or the like;
(11) N-phenylcarbamate-based: diethofencarb;
(12) QoI fungicides (Qo inhibitor-based): azoxystrobin, picoxystrobin, pyraclostrobin, kresoxim-methyl, trifloxystrobin, dimoxystrobin, metominostrobin, orysastrobin, famoxadone, fluoxastrobin, fenamidone, metominofen; ametoctradin, pyrametostrobin, pyraoxystrobin, pyribencarb; coumethoxystrobin, coumoxystrobin, enestroburin, fenoxystrobin, triclopyricarb;
(13) PP fungicides (phenylpyrrole-based): fenpiconil, fludioxonil;
(14) quinoline-based: quinoxyfen;

(15) AH fungicides (aromatic hydrocarbon-based): biphenyl, chloroneb, dichloran, quintozene, tecnazene;
(16) MBI-R-based: fthalide, pyroquilon, tricyclazole;
(17) MBI-D-based: carpropamid, diclocymet, fenoxanil;
(18) SBI agents: fenhexamid, pyributicarb, terbinafine;
(19) phenylureas: pencycuron;
(20) QiI fungicides (Qi inhibitors): cyazofamid; amisulbrom, furmecyclox;
(21) benzamide-based: zoxamide;
(22) enopyranurone-based: blasticidin;
(23) hexopyranosyl-based: kasugamycin; kasugamycin hydrochloride;
(24) glucopyranosyl-based: streptomycin, validamycin; validamycin A;
(25) cyanoacetoamide-based: cymoxanil;
(26) carbamate-based: iodocarb, propamocarb, prothiocarb, polycarbamate;
(27) uncoupling agents: binapacryl, dinocap, ferimzone, fluazinam; meptyldinocap;
(28) organic tin compounds: triphenyltin acetate, triphenyltin chloride, triphenyltin hydroxide;

(29) phosphate esters: phosphonic acid, tolclofos-methyl, fosetyl; tolctophos-methyl;
(30) phthalamide-based: tecloftalam;
(31) benzotriazine-based: triazoxide;
(32) benzene sulfonamide-based: flusulfamide;
(33) pyridazinones: diclomezine;
(34) CAA fungicide (carboxylic amide)-based: dimethomorph, flumorph, benthiavalicarb-isopropyl, iprovalicarb, mandipropamide; valifenalate;
(35) tetracyclines: oxytetracycline;
(36) thiocarbamate-based: methasulfocarb;
(37) other compounds: etridiazole, polyoxins, oxolinic acid, hydroxyisoxazole, octinoline, silthiofam, diflumetorim, acibenzolar-s-methyl, probenazole, tiadinil, ethaboxam, cyflufenamid, proquinazid, metrafenone, fluopicolide, cupric hydroxide, organic copper, sulfur, ferbam, manzeb, maneb, metiram, propineb, thiuram, zineb, ziram, captan, captafol, folpet, chlorothalonil, dichlofluanid, tolylfluanid, dodine, guazatine, iminoctadine acetate, iminoctadine dodecylbenzene sulfonate, anilazine, dithianon, chloropicrin, dazomet, chinomethionat, cyprofuram, silthiofam, agrobacterium, fluoroimide. isotianil, polyoxorim, bordeaux mixture, copper naphthalate, copper oxide, oxychloride copper, copper sulfate, copper man, bis (8-quinolinolato) copper (II), calcium polysulfide, iminoctadine; isofetamido, tolprocarb, fenpyrazamine, pyriofenone, tebufloquin, flupyram, zarilamide; fluor folpet, propamidine, edifenphos; benthiazole, bethoxazin, capsaicin, carvone, curfraneb, mancozeb, cyprosulfamide, debacarb, dichlorophen, difenzoquat, difenzoquat· methyl sulfonate, diphenylamine, flumetover, fluoroimide, flutianil, fosetyl·aluminum, fosetyl·calcium, fosetyl·sodium, irmamycin, methylisothiocyanate (MITC), mildew-mycin, natamycin, nitrothal isopropyl, oxamocarb, oxyphenthiin, propamocarb-fosetylate, puropamocin sodium, pyrimorph, pyrrolnitrin, tolnifanide, trichlaamide;

Examples of the plant growth regulators include:
abscisic acid, indole butyric acid, uniconazole, ethychlozate, ethephon, cloxyfonac, chlormequat, chlorella extract, calcium peroxide, cyanamide, dichlorprop, gibberellin, daminozide, decyl alcohol, trinexapac-ethyl, mepiquat-chloride, paclobutrazol, paraffin wax, piperonyl butoxide, pyraflufen ethyl, flurprimidol, prohydrojasmon, prohexadione-calcium, benzylaminopurine, pendimethalin, forchlorfenuron, potassium hydrazide maleate, 1-naphthylacetoamide, 4-CPA, MCPB, choline, oxyquinoline sulfate, ethychlozate, butralin, 1-methylcyclopropene, aviglycine hydrochloride.

### [Acaricide]

The pest control agent of the present invention is prererably used as an acaricide. Since the pest control agent of the present invention has insecticidal action on adult insects, immature insects, larvae, insect eggs and the like, it can be used to prevent acarus present on agricultural crops. Examples of applicable plants include crops, green stuff, edible roots, tuber crops, trees, grasses for pastures, grasses for lawns and the like. In this case, the acaricide can be applied on each part of the plants. Examples of the part of the plants include leaf, stem, stalk, flower, bud, fruit, seed, sprout, root, tuber, tuberous root, shoot, cutting and the like. In addition, the arcaricide can be applied on the improved variety / varietiy of plants, cultivated variety of plants, mutant plants, hydrid plants, genetically-modified plants (GMO) and the like.

Examples of the acari targeted to prevent are shown below.
(1) Acaridida of Astigmata order:
   (a) Acari belonging to the Acaridae family, for example, Rhizoglyphus echinopus and Rhizoglyphus robini of Rhizoglyphus spp.; Tyrophagus putrescentiae, Tyrophagus neiswanderi, Tyrophagus perniciosus and Tyrophagus similis of Tyrophagus spp.; and others such as Acarus siro, Aleuroglyphus ovatus, Mycetoglyphus fungivorus;

(2) Actinedida of Prostigmata order
(a) Acari belonging to the Tetranychidae family, for example, Bryobia praetiosa and Bryobia rubrioculus of Bryobia spp.; for example, Eotetranychus boreus, otetranychus geniculatus, Eotetranychus pruni, Eotetranychus uncatus, Eotetranychus shii, Eotetranychus suginamensis, Eotetranychus celtis, Eotetranychus smithi, Eotetranychus asiaticus and Eotetranychus kankitus of Eotetranychus spp.; for example, Oligonychus mangiferus, Oligonychus perseae, Oligonychus pustulosus, Oligonychus karamatus, Oligonychus hondoensis, Oligonychus ilicis, Oligonychus ununguis, Oligonychus shinkajii and Oligonychus orthius of Oligonychus spp.; for example, Panonychus citri, Panonychus mori and Panonychus ulmi of Panonychus spp.; for example, Tetranychus viennensis, Tetranychus quercivorus, Tetranychus ludeni, Tetranychus phaselus, Tetranychus cinnabarinus, Tetranychus kanzawai and Tetranychus urticae of Tetranychus spp.; Aponychus corpuzae and Aponychus firmianae of Aponychus spp.; Sasanychus akitanus and Sasanychus pusillus of Sasanychus spp.; Shizotetranychus celarius, Shizotetranychus miscanthi, Shizotetranychus longus, Shizotetranychus schizopus and Shizotetranychus recki of Shizotetranychus spp.; and others such as Tuckerella pavoniformis, Tetranychina harti, Yezonychus sapporensis;

(b) Acari belonging to the Tenuipalpidae family, for example, Brevipalpus lewisi, Brevipalpus russulus, Brevipalpus obovatus and Brevipalpus phoenicis of Brevipalpus spp.; for example, Tenuipalpus pacificus and Tenuipalpus zhizhilashviliae of Tenuipalpus spp.; and others such as Dolichotetranychus floridanus;
(c) Acari belonging to the Eriophyidae family, for example, Aceria diospyri, Aceria ficus, Aceria japonica, Aceria kuko, Aceria paradianthi, Aceria tiyingi, Aceria tulipae and Aceria zoysiea of Aceria spp.; for example, Eriophyes chibaensis and Eriophyes emarginatae of Eriophyes spp.; for example, Aculops lycopersici and Aculops pelekassi of Aculops spp.; for example, Aculus fockeui, Aculus schlechtendali, which belong Aculus spp.; and others such as Colomerus vitis, Calepitrimerus vitis, Phyllocotruta citri, Paracalacarus podocarpi, Calacarus carinatus, Acaphylla theavagrans, Paraphytoptus kikus, Epitrimerus pyri;
(d) Acari belonging to the Transonemidae family, for example, Tarsonemus bilobatus and Tarsonemus waitei of Tarsonemus spp.; others such as Phytonemus pallidus, Polyphagotarsonemus latus;
(e) Acari belonging to the Penthaleidae family, for example, Penthaleus erythrocephalus and Penthaleus major of Penthaleus spp.;

The acaricide of the present invention has a superior prevention effect against acarus parasitic on animals (prevention of ectoparasite). Examples of the acarus parasitic in animals include those acarus which are parasitic in the back, armpit, underbelly, and inner thigh of host animals to obtain nutritional sources such as blood, dandruff from the animals to live. Examples of the host animals include dogs, cats, mice, rats, hamsters, guinea pigs, squirrels, rabbits, ferrets; pet birds (for example, pigeon, parrot, magpie, java sparrow, parakeet, bengalee, canary); cows, horses, pigs, sheep, goats; poultry (for example, ducks, chickens, quails, geese); bees (for example, apis mellifera, Japanese honey bee); and the like.

Examples of the acari targeted to prevent are shown below.
(1) Mite of the Mesostigmata order
   (a) Acari belonging to the Dermanyssidae family, for example, Dermanyssus gallinae;
   (b) Acari belonging to the Macronyssidae family, for example, Ornithonyssus sylviarum, Ornithonyssus bursa and Ornithonyssus bacoti of Ornithonyssus spp.;
   (c) Acari belonging to the Laelapidae family, for example, Laelaps echidninus and Laelaps jettmari of Laelaps spp.; Tropilaelaps clarae;
   (d) Acari belonging to the Varroidae family, for example, Varroa destructor, Varroa jacobsoni and Varroa underwoodi of Varroa spp.;

(2) Tick of the Metastigmata order
   (a) Acari belonging to the Argasidae family, for example, Argas persicus and Argas reflexus of Argas spp.; for example, Ornithodoros moubata, which belongs to Ornithodoros spp.;
   (b) Acari belonging to the Ixodidae family, for example, Haemaphysalis concinna, Haemaphysalis punctata, Haemaphysalis cinnabarina, Haemaphysalis otophila, Haemaphysalis leachi, Haemaphysalis longicornis, Haemaphysalis mageshimaensis, Haemaphysalis yeni, Haemaphysalis campanulata, Haemaphysalis pentalagi, Haemaphysalis flava, Haemaphysalis megaspinosa, Haemaphysalis japonica and Haemaphysalis douglasi of Haemaphysalis spp.; for example, Amblyomma americanum, Amblyomma variegatum, Amblyomma maculatum, Amblyomma hebraeum, Amblyomma cajennense and Amblyomma testudinarium of Amblyomma spp.; for example, Ixodes ricinus, Ixodes hexagonus, Ixodes canisuga, Ixodes pilosus, Ixodes rubicundus, Ixodes scapularis, Ixodes holocyclus, Ixodes ovatus, Ixodes persulcatus and Ixodes nipponensis of Ixodes spp.; for example, Rhipicephalus (Boophilus) microplus), Rhipicephalus (Boophilus) decoloratus), Rhipicephalus (Boophilus) annulatus), Rhipicephalus (Boophilus) calceratus), which belong to Boophilus spp.; for example, Rhipicephalus evertsi, Rhipicephalus sanguineus, Rhipicephalus bursa, Rhipicephalus appendiculatus, Rhipicephalus capensis, Rhipicephalus turanicus and Rhipicephalus zambeziensis of Rhipicephalus spp.; for example, Dermacentor marginatus, Dermacentor reticulatus, Dermacentor pictus, Dermacentor albipictus, Dermacentor andersoni and Dermacentor variabilis of Dermacentor spp.;

(3) Acaridida of the Astigmata order
   (a) Acari belonging to the Psoroptidae family, for example, Psoroptes ovis, Psoroptes cuniculi, Psoroptes equi, which are Psoroptes spp.; for example, Chorioptes bovis, which belongs to Chorioptes spp.; Otodectes cynotis, which belongs to Otodectes spp.;
   (b) Acari belonging to the Sarcoptidae family, for example, Sarcoptes scabiei, Sarcoptes canis, Sarcoptes bovis, Sarcoptes ovis, Sarcoptes rupicaprae, Sarcoptes equi and Sarcoptes suis of Sarcoptes spp.; for example, Notoedres cati, which belongs to Notoedres spp.;
   (c) Acari belonging to the Knemidokoptidae family, for example,
Knemidokoptes mutans, which belongs to Knemidokoptes spp.;

(4) Actinedida of the Prostigmata order
   (a) Acari belonging to the Demodixidae family, for example, Demodex canis, Demodex bovis, Demodex ovis, Demodex caprae, Demodex equi, Demodex caballi, Demodex suis and Demodex cati of Demodex spp.;
   (b) Acari belonging to the Trombiculidae family, for example, Trombicula alfreddugesi and Trombicula akamushi of Trombicula spp.;
   (c) Acari belonging to the Tarsonemidae family, for example, Acarapis woodi, which belongs to Acarapis spp.;

### (Insecticide)

The pest control agent of the present invention is preferably used as an insecticide. The insecticide of the present invention can be used to prevent pests other than the acari present on agricultural crops, sanitary pests, stored grain pests, clothes pests, household pests or the like.

Examples of the pests targeted to prevent are shown below.
(1) Lepidopteran pests, for example, Spodoptera litura, Mamestra brassicae, Agrotis ypsilon, Autographa nigrisigna, Plutella xylostella, Adoxophyes honmai, Homona magnanima, Carposina sasakii, Grapholitha molesta, Phyllocnistis citrella, Caloptilia theivora, Phyllonorycter ringoniella, Lymantria dispar, Euproctis pseudoconspersa, Chilo suppressalis, Cnaphalocrocis medinalis, Ostrinia nubilalis, Hyphantria cunea, Cadra cautella, Heliothis spp., Helioverpa, Agrotis spp., Tinea translucens, Cydia pomonella, Pectinophora gossypiella, or the like;
(2) Hemipteran pests, for example, Myzus persicae, Aphis gossypii, Lipaphis erysimi, Rhopalosiphum padi, Riptortus clavatus, Acrosternum hilare, Unaspis yanonensis, Pseudococcus comstocki, Trialeurodes vaporariorum, Bemisia tabaci, Bemisia argentifolii, Psylla pyricola, Stephanitis nashi, Nilaparvata lugens, Laodelphax stratella, Sogatella furcifera, Nephotettix cincticeps, or the like;
(3) Coleopteran pests, for example, Phyllotreta striolata, Aulacophora indica, Leptinotarsa decemlineata, Lissorhoptrus oryzophilus, Sitophilus oryzae, Callosobruchus chinensis, Popillia japonica, Anomala rufocuprea, Diabrotica spp., Lasioderma serricorne, Lyctus brunneus, Monochamus alternatus, Anoplophora malasiaca, Agriotes spp., Epilachna vigintioctomaculata, Tenebroides mauritanicus, Anthonomus grandis, or the like;
(4) Dipteran pests, for example, Bactrocera cucurbitae, Bactrocera dorsalis, Delia platura, Hydrellia griseola, Drosophila melanogaster, or the like;
(5) Thysanopteran pests, for example, Thrips palmi, Scirtothrips dorsalis, or the like;
(6) Hymenopteran pests, for example, Monomorium pharaonis, Vespa simillima, Athalia rosae, or the like;
(7) Orthopteran pests, for example, Locusta migratoria, or the like;
(8) Blattodea pests, for example, Blattella germanica, Periplaneta fuligginosa, Periplaneta japonica, Periplaneta americana, Periplaneta australasiae, or the like;
(9) Isopteran pests, for example, Coptotermes formosanus, Reticulitermes speratus, or the like;
(10) Plant parasitic nematodes, for example, Meloidogyne incognita, Pratylenchus spp., Heterodera glycines, Aphelenchoides besseyi, Bursaphelenchus xylophilus, or the like.

The insecticide of the present invention has a superior prevention effect against pests parasitic on animals (prevention of ectoparasite).

Examples of the pests targeted to prevent are shown below.

### (1) Phthiraptera

Louse belonging to Haematopinidae family, Louse belonging to Linognathidae family, Biting louse belonging to Menoponidae family, Biting louse belonging to Philopteridae family, Biting louse of Trichodectidae family;

### (2) Siphonaptera

Flea belonging to Pulicidae family, for example, Ctenocephalides canis and Ctenocephalides felis of Ctenocephalides spp.;

Flea belonging to Tungidae family, Flea belonging to Ceratophyllidae family, Flea of Leptopsyllidae family;

### (3) Pest of Hemiptera;

### (4) Pest of Diptera

Mosquito belonging to Culicidae family, Black fly belonging to Simuliidae family, Punkie belonging to Ceratopogonidae family, Fly belonging to Tabanidae family, Fly belonging to Muscidae family, Glossina belonging to Glossinidae family; Fly belonging to Hippoboscidae family, Fly belonging to Calliphoridae family, Fly belonging to Oestridae family;

### Examples

The following provides Examples to explain the present invention more specifically. However, the present invention is not limited to the following examples.

### Example 1

Production of 1-ethyl-3-[2-(pyridine-2-yl)propan-2-yl]-1-(thiazolo[5, 4-b]pyridine-5-yloxy)urea (Compound 7) (the following Compound No. 1-2)

Production of phenyl benzyloxycarbamate (Compound 2)

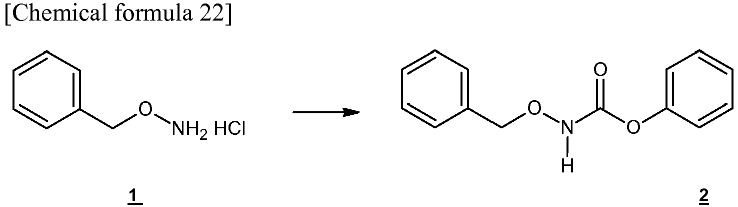

25.0 g of O-benzylhydroxylamine hydrochloride (Compound 1) was dissolved in 250 ml of acetonitrile. Then, 24.8g of pyridine and 24.5g of chloroformic acid phenyl ester were added to the resulting solution while cooling with ice, followed by stirring for 3 hours at room temperature. Then, brine was added to the resulting mixture, followed by extracting with ethyl acetate. The resulting organic layer was dried by adding magnesium sulfate and filtered, followed by distilling the solvent under reduced pressure. The resulting residue was purified by silica gel column chromatography to obtain 38.0 g of Compound 2 (yield: 99%). Compound 2 recorded the following ¹H-NMR spectra.
¹H-NMR (CDCl₃/TMS, δ(ppm)) 7.54(br, 1H), 7.45-7.34(m, 7H), 7.22(t, 1H), 7.13(d, 2H), 4.97(s, 2H)

Production of 1-benzyloxy-3-[2-(pyridin-2-yl)propan-2-yl]urea (Compound 3)

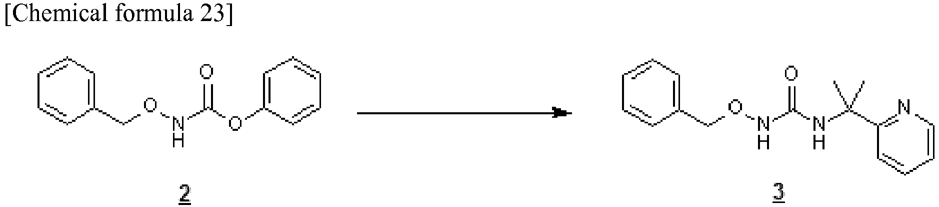

10.7 g of Compound 2 was dissolved in 200 ml of tetrahydrofuran. Then, 6.0 g of 2-(pyridin-2-yl)propan-2-amine and 4.90 g of triethylamine were added to the resulting solution, followed by stirring for 6 hours while being heated under reflux. The solvent was then distilled under reduced pressure. The resulting residue was purified by silica gel column chromatography to obtain 7.93 g of Compound 3 (yield: 63%, melting point: 104-106 °C).

Production of 1-(benzyloxy)-1-ethyl-3-[2-(pyridin-2-yl)propan-2-yl]urea (Compound 4)

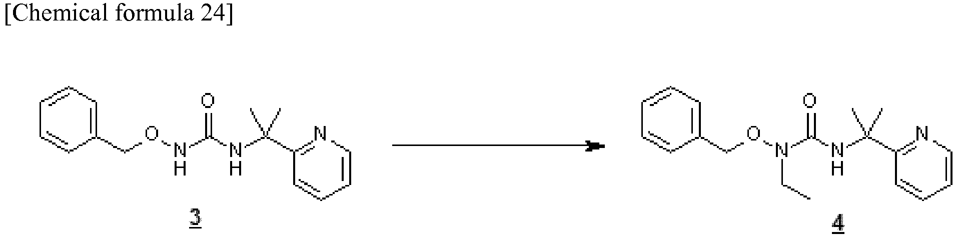

15.79 g of Compound 3 was dissolved in 300 ml of N, N-dimethyl formamide. Then, 2.43g of sodium hydride was added to the resulting solution, followed by stirring for 1 hour at room temperature while cooling with ice. Then, ethyl iodide (9.06 g) was added to the resulting mixture, followed by stirring for 3 hours at room temperature. Then, brine was added to the resulting mixture, followed by extracting with ethyl acetate. The resulting organic layer was dried by adding magnesium sulfate and filtered, followed by distilling the solvent under reduced pressure. The resulting residue was purified by silica gel column chromatography to obtain 17.12 g of Compound 4 (yield: 99%). Compound 4 recorded the following ¹H-NMR spectra.
¹H-NMR (CDCl₃/TMS, δ(ppm)) 8.48(dd, 1H), 7.88(br, 1H), 7.66(dt, 1H), 7.28-7.53(m, 5H), 7.16(dd, 1H), 6.94(br, 1H), 4.86(s, 2H), 3.58(q, 2H), 1.67(s, 6H), 1.19(t, 3H)

Production of 1-ethyl-1-hydroxy-3-[2-(pyridin-2-yl)propan-2-yl]urea (Compound 5)

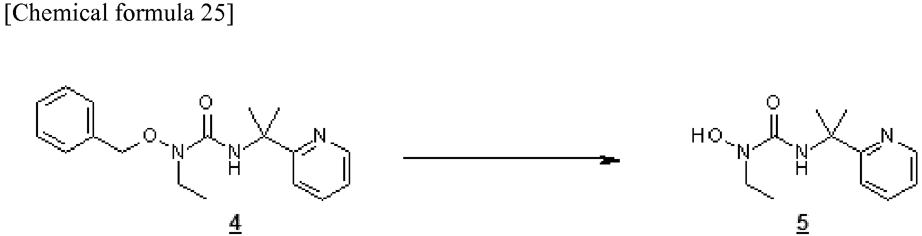

4.71 g of Compound 4 was dissolved in 40 ml of methanol. Then, 1 g of 5% palladium carbon was added to the resulting solution followed by performing hydrogen gas-replacement followed by stirring for 24 hours at room temperature. The resulting mixture was then filtered over celite. The solvent was distilled under reduced pressure to obtain 3.25 g of Compound 5 (yield: 97%, melting point: 73-75 °C).

Production of 1-ethyl-3-[2-(pyridine-2-yl)propan-2-yl]-1-(thiazolo[5, 4-b]pyridine-5-yloxy)urea (Compound 7)

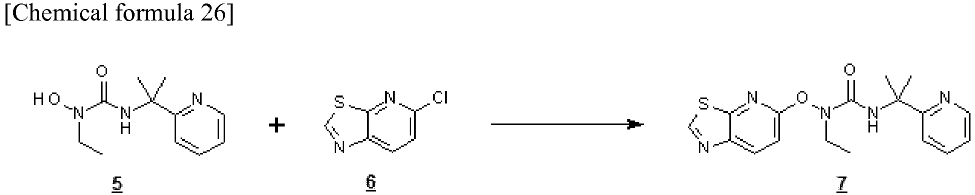

0.22 g of Compound 5 and 0.22 g of Compound 6 were dissolved in 5 ml of dimethyl sulfoxide. Then, 0.12 g of potassium t-butoxide was added to the resulting solution followed by stirring for 1 hour at room temperature. An aqueous solution of ammonium chloride was then added to the resulting mixture, followed by extracting with ethyl acetate. The resulting residue was purified by silica gel column chromatography to obtain 0.080 g of Compound 7 (yield: 23%, melting point: 116-118 °C).

### Example 2

Production of 1-[benzo(d)thiazol-6-yloxy]-1-ethyl-3-[2-(pyridin-2-yl)propan-2-yl]urea (Compound 11) (the following Compound No. 1-1)

Production of phenyl benzo(d)thiazol-6-yloxycarbamate (Compound 9)

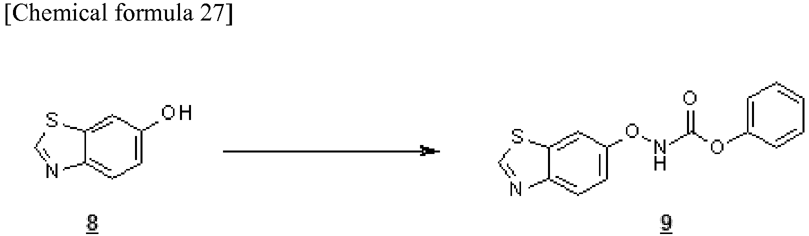

0.30 g of benzo(d)thiazol-6-ol was dissolved in 10 ml of tetrahydrofuran. Then, 0.25 g of potassium t-butoxide was added to the resulting solution, followed by stirring for 2 hours while being heated to reflux. The solvent was then distilled under reduced pressure. The resulting residue was dissolved in 10 ml ofN, N-dimethyl formamide. Then, 5 ml of methylene chloride solution of 1.06 g of O-mesityl sulfonyl hydroxylamine was added to the resulting solution while cooling with ice followed by stirring for 30 min. Then, 0.22 g of pyridine and 0.31 g of chloroformic acid phenyl ester were added to the resulting mixture while cooling with ice followed by stirring for 2 hours at room temperature. Then, brine was added to the resulting mixture, followed by extracting with ethyl acetate. The resulting organic layer was dried by adding magnesium sulfate and filtered, followed by distilling the solvent under reduced pressure. The resulting residue was purified by silica gel column chromatography to obtain 0.20 g of Compound 9 (yield: 35%, melting point: 152-154 °C).

Production of 1-[benzo(d)thiazol-6-yloxy]-3-[2-(pyridin-2-yl)propan-2-yl]urea (Compound 10)

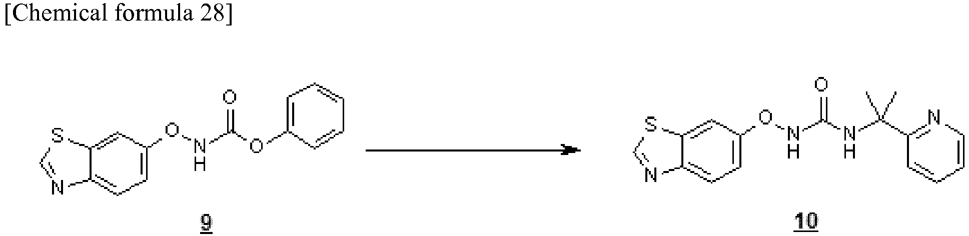

0.20 g of Compound 9 was dissolved in 10 ml of tetrahydrofuran. Then, 0.10 g of 2-(pyridin-2-yl)propan-2-amine and 0.08 g of triethylamine were added to the resulting solution, followed by stirring over nigh while being heated under reflux. The solvent was then distilled under reduced pressure. The resulting residue was purified by silica gel column chromatography to obtain 0.20 g of Compound 10 (yield: 87%, melting point: 164-166 °C).

Production of 1-[benzo(d)thiazol-6-yloxy]-1-ethyl-3-[2-(pyridin-2-yl)propan-2-yl]urea (Compound 11)

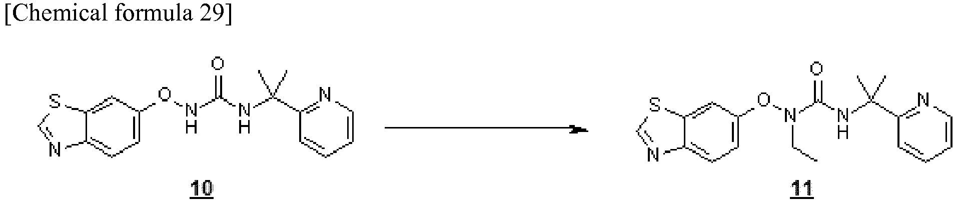

0.20 g of Compound 10 was dissolved in 4 ml of N, N-dimethyl formamide. Then, 0.09 g of potassium carbonate and 0.10 g of ethyl iodide were added to the resulting solution, followed by stirring for 3 hours at room temperature. Then, brine was added to the resulting mixture, followed by extracting with ethyl acetate. The resulting organic layer was dried by adding magnesium sulfate and filtered, followed by distilling the solvent under reduced pressure. The resulting residue was purified by silica gel column chromatography to obtain 0.16 g of Compound 11 (yield: 74%, melting point: 76-77 °C).

### Example 3

Production of 1-ethyl-3-[2-(pyridin-2-yl)propan-2-yl]-1-[2-(trifluoromethyl)thiazolo[4, 5-c]pyridin-6-yloxy]urea (Compound 18) (the following Compound No. 1-11)

Production of N-(4, 6-dichloropyridin-3-yl)-2, 2, 2-trifluoroacetamide (Compound 13)

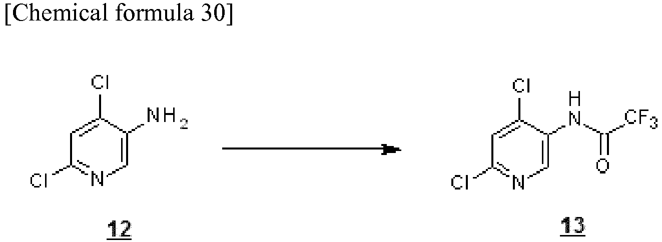

2.00 g of Compound 12 was dissolved in 20 ml of dichloromethane. Then, 2.71 g of trifluoroacetic acid anhydride was added to the resulting solution followed by stirring for 3 hours at room temperature. An aqueous solution of sodium carbonate was then added to the mixture to neutralize, followed by extracting with ethyl acetate. The resulting organic layer was dried by adding magnesium sulfate and filtered, followed by distilling the solvent under reduced pressure. The resulting residue was purified by silica gel column chromatography to obtain 3.00 g of Compound 13 (yield: 94%). Compound 13 recorded the following ¹H-NMR spectra.
¹H-NMR (CDCl₃/TMS, δ(ppm)) 9.28(s, 1H), 8.27(s, 1H), 7.50(s, 1H)

Production of 6-chloro-2-(trifluoromethyl)thiazolo[4, 5-c]pyridine (Compound 14)

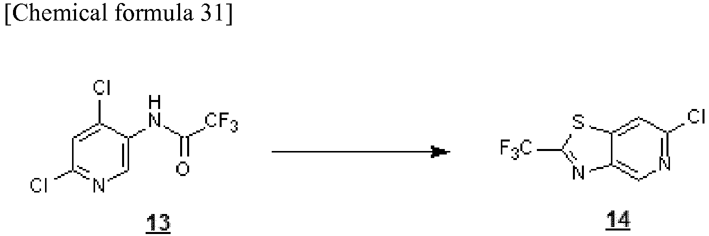

0.40 g of Compound 13 was dissolved in 30 ml of toluene. Then, 2.79 g of Davy-reagent p-tolyl was added to the resulting solution followed by stirring for 7 hours while being heated to reflux, followed by stirring overnight at room temperature. An aqueous solution of sodium carbonate was then added to the mixture, followed by extracting with ethyl acetate. The resulting organic layer was dried by adding magnesium sulfate and filtered, followed by distilling the solvent under reduced pressure. The resulting residue was purified by silica gel column chromatography to obtain 1.92 g of Compound 14 (yield: 70%). Compound 14 recorded the following ¹H-NMR spectra.
¹H-NMR (CDCl₃/TMS, δ(ppm)) 9.29(s, 1H), 8.00(s, 1H)

Production of tert-butyl 2-(trifluoromethyl)thiazolo[4, 5-c]pyridin-6-yloxycarbamate (Compound 15)

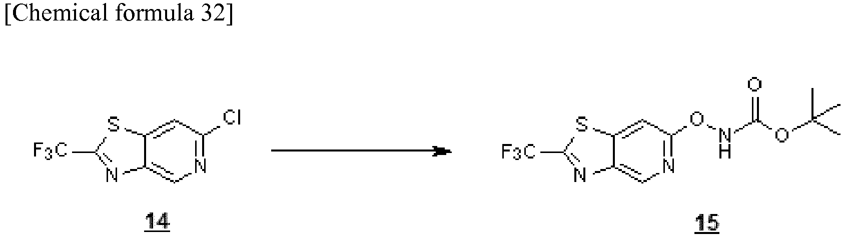

1.05 g of Compound 14 and 0.71 g of Boc-hydroxylamine were dissolved in 20 ml of dimethyl sulfoxide. Then, 0.75 g of potassium hydroxide was added to the resulting solution, followed by stirring for 5 hours at room temperature. An aqueous solution of ammonium chloride was then added to the resulting mixture, followed by extracting with ethyl acetate. The resulting organic layer was dried by adding magnesium sulfate and filtered, followed by distilling the solvent under reduced pressure. The resulting residue was purified by silica gel column chromatography to obtain 0.96 g of Compound 15 (yield: 65%m melting point: 149-150 °C).

Production of phenyl 2-(trifluoromethyl)thiazolo[4, 5-c]pyridin-6-yloxycarbamate (Compound 16)

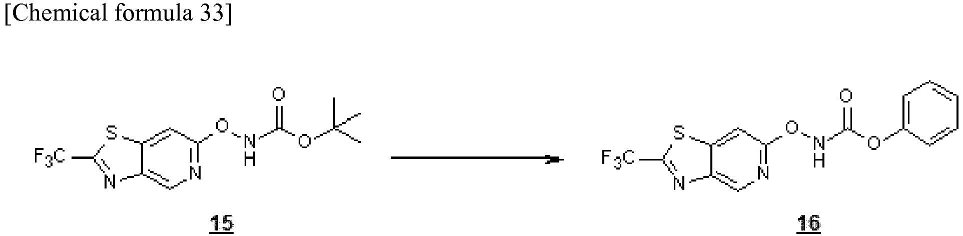

1.19 g of Compound 15 was dissolved in 30 ml of dichloromethane. Then, 0.54 g of triethylamine was added to the resulting solution while cooling with ice followed by dropping 0.61 g of chloroformic acid phenyl ester followed by stirring for 1 hour while cooling with ice. Then, an aqueous solution of ammonium chloride was added to the resulting mixture to extract the dichloromethane layer. The layer was then dried by adding magnesium sulfate and filtered, followed by distilling the solvent under reduced pressure. Then, 24 ml of dichloromethane was added to the resulting residue followed by dropping 8 ml of trifluoroacetic acid followed by stirring for 5 hours at room temperature. The reaction solution was diluted with ethyl acetate, neutralized by a saturated aqueous solution of sodium hydrogen carbonate, followed by extracting the organic layer. The resulting organic layer was dried by adding magnesium sulfate and filtered, followed by distilling the solvent under reduced pressure. The resulting residue was purified by silica gel column chromatography to obtain 1.14 g of Compound 16 (yield: 90%). Compound 16 recorded the following ¹H-NMR spectra.
¹H-NMR (CDCl3/TMS, δ(ppm)) 9.16(s, 1H), 8.71(s, 1H), 7.65(s, 1H), 7.40-7.36(m, 2H), 7.25-7.23(m, 1H), 7.19-7.17(m, 2H)

Production of 1-[2-(pyridin-2-yl)propan-2-yl]-3-[2-(trifluoromethyl)thiazolo[4, 5-c]pyridin-6-yloxy] urea (Compound 17)

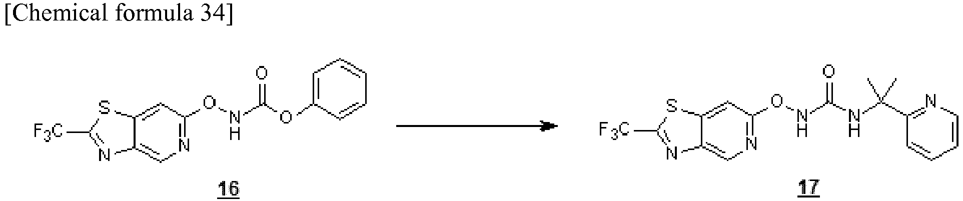

0.30 g of Compound 16 was dissolved in 20 ml of tetrahydrofuran. Then, 0.15 g of 2-(pyridin-2-yl)propan-2-amine and 0.5 ml of triethylamine were added to the resulting solution, followed by stirring for 5 hours while being heated under reflux. The solvent was then distilled under reduced pressure. The resulting residue was purified by silica gel column chromatography to obtain 0.23 g of Compound 17 (yield: 68%). Compound 17 recorded the following ¹H-NMR spectra.
¹H-NMR (CDCl₃/TMS, δ(ppm)) 9.17(s, 1H), 8.43-8.41(m, 1H), 8.22(s, 1H), 7.98(s, 1H), 7.84(d, 1H), 7.69(ddd, 1H), 7.39-7.37(m, 1H), 7.16(ddd, 1H), 1.76(s, 6H)

Production of 1-ethyl-3-[2-(pyridin-2-yl)propan-2-yl]-1-[2-(trifluoromethyl)thiazolo[4,5-c] pyridin-6-yloxy]urea (Compound 18)

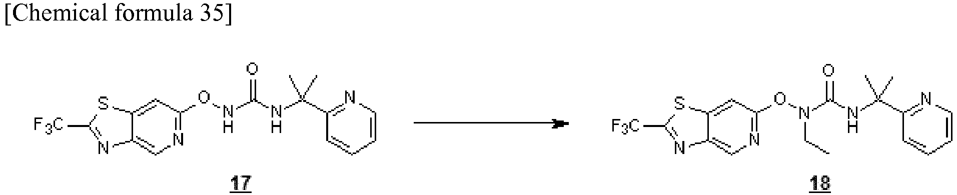

0.23 g of Compound 17 was dissolved in 7 ml of N, N-dimethyl formamide. Then, 0.23 g of potassium carbonate and 0.090 g of ethyl iodide were added to the resulting solution, followed by stirring for 5 hours at room temperature. Then, ethyl acetate was added to the resulting mixture, followed by washing with an aqueous solution of ammonium chloride. The organic layer was dried by adding magnesium sulfate and filtered, followed by distilling the solvent under reduced pressure. The resulting residue was purified by silica gel column chromatography to obtain 0.21 g of Compound 18 (yield: 84%, melting point: 120-122 °C).

### Example 4

Production of 1-[6-chloro-4-(trifluoromethyl)pyridine-2-yloxy]-1-ethyl-3-{2-[4-(methylthio)pyridin-2-yl]propan-2-yl}urea (Compound No. 3-9)

Production of 1-benzyloxy-3-{2-[4-(methylthio)pyridin-2-yl]propan-2-yl}urea (Compound 19)

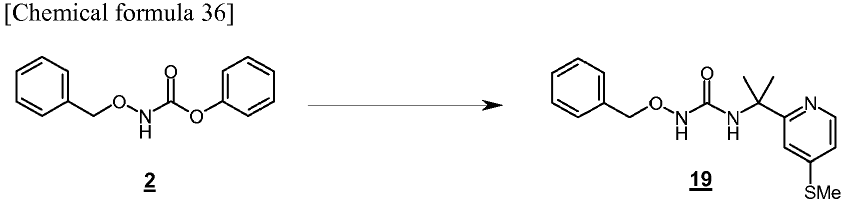

2.57 g of Compound 2 was dissolved in 32 ml of acetonitrile. Then, 6.0 g of 2-[4-(methylthio)pyridin-2-yl]propan-2-amine was added to the resulting solution, followed by stirring for 24 hours while being heated to reflux. The solvent was distilled under reduced pressure after the reaction. The resulting residue was purified by silica gel column chromatography to obtain 3.29 g of Compound 19 (yield: 94%). Compound 19 recorded the following ¹H-NMR spectra.
¹H-NMR (CDCl₃ / TMS, δ(ppm)) 8.32(d, 1H), 7.81(s, 1H), 7.50-7.47(m, 2H), 7.40-7.35 (m, 3H), 7.13(d, 1H), 6.99(dd, 1H), 6.88(s, 1H), 4.87(s, 2H), 2.50(s, 3H), 1.69(s, 6H)

Production of 1-Benzyloxy-1-ethyl-3-{2-[4-(methylthio)pyridin-2-yl]propan-2-yl}urea (Compound 20)

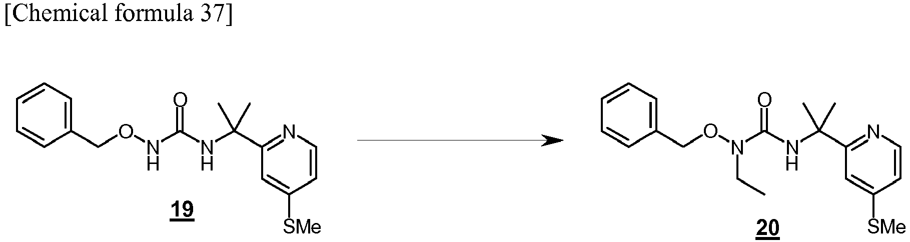

3.28 g of Compound 19 was dissolved in 40 ml of N, N-dimethyl formamide. Then, 0.45 g of 55% sodium hydride was added to the resulting solution while cooling with ice, followed by stirring for 15 min at room temperature. Then, 1.59 g of ethyl iodide was added to the resulting mixture followed by stirring for 3 hours at room temperature. After the reaction, a saturated aqueous solution of ammonium chloride was added followed by extracting with ethyl acetate. The resulting organic layer was dried by adding magnesium sulfate and filtered, followed by distilling the solvent under reduced pressure. The resulting residue was purified by silica gel column chromatography to obtain 3.41 g of Compound 20 (yield: 96%). Compound 20 recorded the following ¹H-NMR spectra.
¹H-NMR (CDCl₃ / TMS, δ(ppm)) 8.28(d, 1H), 7.77(s, 1H), 7.52-7.49(m, 2H), 7.40-7.35 (m, 3H), 7.12(d, 1H), 6.97(dd, 1H), 4.84(s, 2H), 3.57(q, 2H), 2.49(s, 3H), 1.65(s, 6H), 1.18(t, 3H)

Production of 1-Ethyl-1-hydroxy-3-{2-[4-(methylthio)pyridin-2-yl]propan-2-yl}urea (Compound 21)

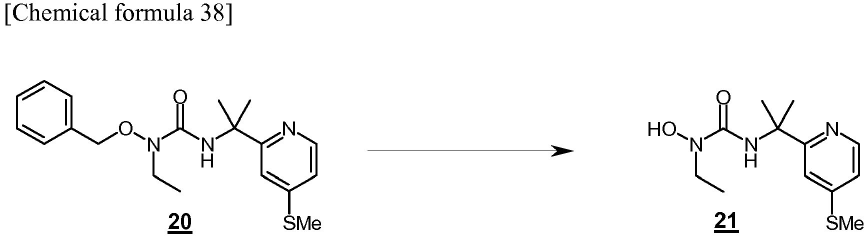

3.41 g of Compound 20 was dissolved in 27 ml of dichloromethane. Then, 8 ml of trifluoromethanesulfonic acid was dropped into the resulting solution while cooling with ice, followed by stirring for 2 hours at room temperature. After the reaction, a saturated aqueous solution of sodium hydrogen carbonate was added to the mixture to neutralize, followed by extracting the organic layer. The organic layer was dried by adding magnesium sulfate and filtered, followed by distilling the solvent under reduced pressure. The resulting residue was purified by silica gel column chromatography to obtain 2.70 g of Compound 21 (quant.). Compound 21 recorded the following ¹H-NMR spectra.
¹H-NMR (CDCl₃/TMS, δ(ppm)) 8.61(s, 1H), 8.31(d, 1H), 7.40(s, 1H), 7.23 (d, 1H), 6.97(dd, 1H), 3.49(q, 2H), 2.49(s, 3H), 1.72(s, 6H), 1.13(t, 3H)

Production of 1-[6-Chloro-4-(trifluoromethyl)pyridine-2-yloxy]-1-ethyl-3-{2-[4-(methylthio)pyridin-2-yl]propan-2-yl}urea (Compound 22)

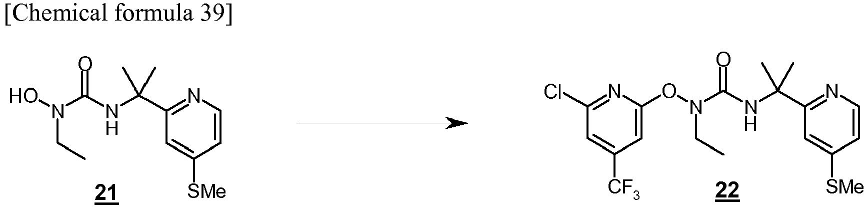

0.27 g of Compound 21 and 0.23 g of 2, 6-dichloro-4-(trifluoromethyl)pyridine were dissolved in 8 ml of tetrahydrofuran. Then, 0.12 g of potassium t-butoxide was added to the resulting solution at 5 °C, followed by stirring for 1 hour at 5 to 10 °C. After the reaction, the reaction solution was poured into a saturated aqueous solution of ammonium chloride, followed by extracting with ethyl acetate. The organic layer was dried by adding magnesium sulfate and filtered, followed by distilling the solvent under reduced pressure. The resulting residue was purified by silica gel column chromatography to obtain 0.29 g of Compound 22 (yield: 64%m melting point: 64-67 °C).

The aryloxyurea compounds shown in Tables 1 to 9 were produced by the methods described above or the like. Table 1 shows the substituents of the compound represented by formula (1). Table 2 shows the substituents of the compound represented by formula (2). Tables 3-1, 3-2 and 3-3 show the substituents of the compound represented by formula (3). Table 4 shows the substituents of the compound represented by formula (4). Table 5 shows the substituents of the compound represented by formula (5). Table 6 shows the substituents of the compound represented by formula (6). Table 7 shows the substituents of the compound represented by formula (7). Table 8 shows the substituents of the compound represented by formula (8). Table 9 shows the substituents of the compound represented by formula (9). In addition, Tables 1 to 9 show only a part of aryloxyurea compounds of the present invention. An ordinary skilled person can easily understand that other compounds which are not shown in this description, namely, the compounds which are substituted with various substituents complying with the purpose and scope of the present invention can also be obtained by the above-described method and can be used.

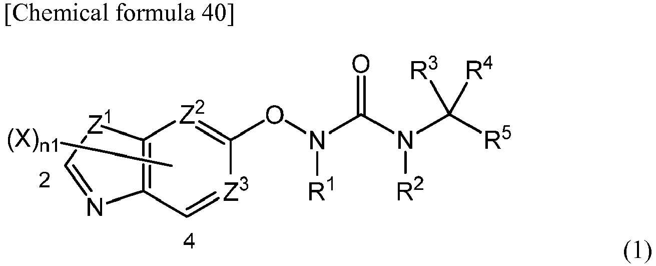

**[Table 1]**

| Compound No. | R¹ | R² | R³ | R⁴ | R⁵ | (X)ₙ₁ | Z¹ | Z² | Z³ |
|---|---|---|---|---|---|---|---|---|---|
| 1-1 | Et | H | Me | Me | Py-2-yl | - | S | C | C |
| 1-2 | Et | H | Me | Me | Py-2-yl | - | S | N | C |
| 1-3 | Et | H | Me | Me | Pyrimidin-2-yl | - | S | C | C |
| 1-4 | Et | H | Me | Me | Py-2-yl | 2-Me | S | C | C |
| 1-5 | Et | H | Me | Me | CONHCH₂CF₃ | - | S | C | C |
| 1-6 | Et | H | Me | Me | Py-2-yl | 2-(2,5-Me₂-pyrrol-1-yl) | S | C | C |
| 1-7 | Et | H | Me | Me | Py-2-yl | 2-CN | S | C | C |
| 1-8 | Et | H | Me | Me | Py-2-yl | 2-CF₃ | S | C | C |
| 1-9 | Et | H | Me | Me | Py-2-yl | 2-Me | S | C | C |
| 1-10 | Et | H | Me | Me | Py-2-yl | 2-Me | S | N | C |
| 1-11 | Et | H | Me | Me | Py-2-yl | 2-CF₃ | S | C | N |
| 1-12 | Et | H | Me | Me | Pyrimidin-2-yl | 2-CF₃ | S | C | N |
| 1-13 | Et | H | Me | Me | 4-Ph-Py-2-yl | - | S | C | C |
| 1-14 | Et | H | Me | Me | Py-2-yl | 4-Br | S | C | C |
| 1-15 | Et | H | Me | Me | Pyrimidin-2-yl | 4-Br | S | C | C |
| 1-16 | Et | H | Me | Me | Py-2-yl | - | O | C | C |
| 1-17 | Et | H | Me | Me | 4-SMe-Py-2-yl | - | S | C | C |
| 1-18 | Et | H | Me | Me | 4-SO2Me-Py-2-yl | - | S | C | C |

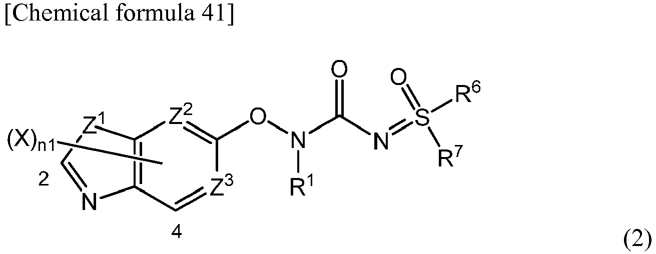

**[Table 2]**

| Compound No. | R¹ | R⁶ | R⁷ | (X)ₙ₁ | Z¹ | Z² | Z³ |
|---|---|---|---|---|---|---|---|
| 2-1 | Et | Me | Ph | - | S | C | C |

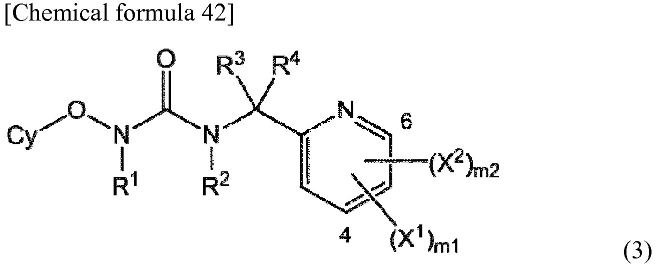

**[Table 3-1]**

| Compound No. | R¹ | R² | R³ | R⁴ | (X¹)ₘ₁ | (X²)ₘ₂ | Cy |
|---|---|---|---|---|---|---|---|
| 3-1 | Et | H | Me | Me | 4-SMe | - | 3-Br-5-Cl-Ph |
| 3-2 | Et | H | Me | Me | 4-SO₂Me | - | 3-Br-5-Cl-Ph |
| 3-3 | Et | H | Me | Me | 4-SMe | - | 4-Br-3,5-Cl₂-Ph |
| 3-4 | Et | H | Me | Me | 4-SO₂Me | 1-oxo | 4-Br-3,5-Cl₂-Ph |
| 3-5 | Et | H | Me | Me | 4-SO₂Me | - | 4-Br-3,5-Cl₂-Ph |
| 3-6 | Et | H | Me | Me | 4-SMe | - | 2-Cl-6-CF₃-Py-4-yl |
| 3-7 | Et | H | Me | Me | 4-SO₂Me | - | 2-Cl-6-CF₃-Py-4-yl |
| 3-8 | Et | H | Me | Me | 4-SO₂Me | 1-oxo | 2-Cl-6-CF₃-Py-4-yl |
| 3-9 | Et | H | Me | Me | 4-SMe | - | 4-CF₃-6-Cl-Py-2-yl |
| 3-10 | Et | H | Me | Me | 4-SO₂Me | 1-oxo | 4-CF₃-6-Cl-Py-2-yl |
| 3-11 | Et | H | Me | Me | 4-SO₂Me | - | 4-CF₃-6-Cl-Py-2-yl |
| 3-12 | Et | H | Me | Me | 4-SMe | - | 2,6-Br₂-Py-4-yl |
| 3-13 | Et | H | Me | Me | 4-SOMe | - | 2,6-Br₂-Py-4-yl |
| 3-14 | Et | H | Me | Me | 4-SO₂Me | - | 2,6-Br₂-Py-4-yl |
| 3-15 | Et | H | Me | Me | 4-SMe | - | 3-Cl-4,5-Br₂-Ph |
| 3-16 | Et | H | Me | Me | 4-SO₂Me | 1-oxo | 4-CF₃-6-Cl-Py-2-yl |
| 3-17 | Et | H | Me | Me | 4-SO₂Me | - | 3-Cl-4,5-Br₂-Ph |
| 3-18 | Et | H | Me | Me | 4-SEt | - | 4-CF₃-6-Cl-Py-2-yl |
| 3-19 | Et | H | Me | Me | 4-SEt | - | 3-Br-5-Cl-Ph |
| 3-20 | Et | H | Me | Me | 4-SEt | - | 2,6-Br₂-Py-4-yl |
| 3-21 | Et | H | Me | Me | 4-SEt | - | 3-Cl-4,5-Br₂-Ph |
| 3-22 | Et | H | Me | Me | 4-SⁱPr | - | 4-CF₃-6-Cl-Py-2-yl |
| 3-23 | Et | H | Me | Me | 4-SⁱPr | - | 3-Br-5-Cl-Ph |
| 3-24 | Et | H | Me | Me | 4-SⁱPr | - | 2,6-Br₂-Py-4-yl |
| 3-25 | Et | H | Me | Me | 4-SⁱPr | - | 3-Cl-4,5-Br₂-Ph |

**[Table 3-2]**

| Compound No. | R¹ | R² | R³ | R⁴ | (X¹)ₘ₁ | (X²)ₘ₂ | Cy |
|---|---|---|---|---|---|---|---|
| 3-26 | Et | H | Me | Me | 4-SO₂ⁱPr | - | 3-Br-5-Cl-Ph |
| 3-27 | Et | H | Me | Me | 4-SO₂ⁱPr | - | 3-Cl-4,5-Br₂-Ph |
| 3-28 | Et | H | Me | Me | 4-SO₂ⁱPr | - | 4-CF₃-6-Cl-Py-2-yl |
| 3-29 | Et | H | Me | Me | 4-SO₂ⁱPr | - | 2,6-Br₂-Py-4-yl |
| 3-30 | Et | H | Me | Me | 4-SOMe | - | 3,5-Cl₂-4-Br-Ph |
| 3-31 | Et | H | Me | Me | 4-SCH₂CF₃ | - | 3-Br-5-Cl-Ph |
| 3-32 | Et | H | Me | Me | 4-(Pyrrolidin-1-yl) | - | 3-Br-5-Cl-Ph |
| 3-33 | Et | H | Me | Me | 4-SO₂Et | - | 4-CF₃-6-Cl-Py-2-yl |
| 3-34 | Et | H | Me | Me | 4-SO₂Et | - | 2,6-Br₂-Py-4-yl |
| 3-35 | Et | H | Me | Me | 4-NMe₂ | - | 3-Br-5-Cl-Ph |
| 3-36 | Et | H | Me | Me | 5-SMe | - | 3-Br-5-Cl-Ph |
| 3-37 | Et | H | Me | Me | 5-SO₂Me | - | 3-Br-5-Cl-Ph |
| 3-38 | Et | H | Me | Me | 4-SO₂Et | - | 3-Br-5-Cl-Ph |
| 3-39 | Et | H | Me | Me | 4-SOEt | - | 3-Br-5-Cl-Ph |
| 3-40 | Et | H | Me | Me | 4-SO₂Et | - | 3-Cl-4,5-Br₂-Ph |
| 3-41 | Et | H | Me | Me | 4-SOEt | - | 3-Cl-4,5-Br₂-Ph |
| 3-42 | Et | H | Me | Me | 4-SMe | - | 2-NMe₂-6-CF₃-Pyrimidin-4-yl |
| 3-43 | Et | H | Me | Me | 4-SMe | - | 2,6-Cl₂-Py-4-yl |
| 3-44 | Et | H | Me | Me | 4-SO₂Me | - | 2,6-Cl₂-Py-4-yl |
| 3-45 | Et | H | Me | Me | 4-SMe | - | 2-Cl-6-NMe₂-Py-4-yl |
| 3-46 | Et | H | Me | Me | 4-SO₂Me | - | 2-Cl-6-NMe₂-Py-4-yl |
| 3-47 | Et | H | Me | Me | 4-SOMe | - | 2-Cl-6-NMe₂-Py-4-yl |
| 3-48 | Et | H | Me | Me | 4-SMe | - | 4-SMe-6-Cl-Py-2-yl |
| 3-49 | Et | H | Me | Me | 4-SO₂Me | - | 4-SO₂Me-6-Cl-Py-2-yl |
| 3-50 | Et | H | Me | Me | 4-SMe | - | 4-CF₃-6-SMe-Py-2-yl |
| 3-51 | Et | H | Me | Me | 4-SMe | - | 4-CF₃-6-NMe₂-Py-2-yl |
| 3-52 | Et | H | Me | Me | 4-SMe | - | 2-Cl-6-SMe-Py-4-yl |
| 3-53 | Et | H | Me | Me | 4-SO₂Me | - | 2-Cl-6-SMe-Py-4-yl |
| 3-54 | Et | H | Me | Me | 4-SO₂Me | - | 2-Cl-6-SO₂Me-Py-4-yl |
| 3-55 | Et | H | Me | Me | 4-SO₂Me | - | 4-CF₃-6-NMe₂-Py-2-yl |
| 3-56 | Et | H | Me | Me | 4-SMe | - | 4,6-Cl₂-Py-2-yl |
| 3-57 | Et | H | Me | Me | 4-SO₂Me | - | 4,6-Cl₂-Py-2-yl |
| 3-58 | Et | H | Me | Me | 4-SO₂Me | - | 4-CF₃-6-Me-Py-2-yl |
| 3-59 | Et | H | Me | Me | 4-SMe | - | 4-Br-6-Cl-Py-2-yl |
| 3-60 | Et | H | Me | Me | 4-SO₂Me | - | 4-Br-6-Cl-Py-2-yl |

**[Table 3-3]**

| Compound No. | R¹ | R² | R³ | R⁴ | (X¹)ₘ₁ | (X²) ₘ₂ | Cy |
|---|---|---|---|---|---|---|---|
| 3-61 | Et | H | Me | Me | 4-SO₂ⁿPr | - | 4-CF₃-6-Cl-Py-2-yl |
| 3-62 | Et | H | Me | Me | 4-SMe | - | 4-CF₃-6-Me-Py-2-yl |
| 3-63 | Et | H | Me | Me | 4-SMe | - | 2-Br-6-Cl-Py-4-yl |
| 3-64 | Et | H | Me | Me | 4-SO₂Me | - | 2-Br-6-Cl-Py-4-yl |
| 3-65 | Et | H | Me | Me | 4-SEt | - | 2-Br-6-Cl-Py-4-yl |
| 3-66 | Et | H | Me | Me | 4-SO₂Et | - | 2-Br-6-Cl-Py-4-yl |
| 3-67 | Et | H | Me | Me | 4-SO₂Et | 1-oxo | 2-Br-6-Cl-Py-4-yl |
| 3-68 | Et | H | Me | Me | 4-SEt | - | 2,6-Cl₂-Py-4-yl |
| 3-69 | Et | H | Me | Me | 4-SⁿPr | - | 2,6-Cl₂-Py-4-yl |
| 3-70 | Et | H | Me | Me | 4-SⁿPr | - | 2-Br-6-Cl-Py-4-yl |
| 3-71 | Et | H | Me | Me | 4-SⁿPr | - | 4-CF₃-6-Cl-Py-2-yl |
| 3-72 | Et | H | Me | Me | 4-SO₂Et | - | 2,6-Cl₂-Py-4-yl |
| 3-73 | Et | H | Me | Me | 4-SO₂ⁿPr | - | 2,6-Cl₂-Py-4-yl |
| 3-74 | Et | H | Me | Me | 4-SO₂ⁿPr | - | 2-Br-6-Cl-Py-4-yl |
| 3-75 | Et | H | Me | Me | 4-SⁿBu | - | 4-CF₃-6-Cl-Py-2-yl |
| 3-76 | Et | H | Me | Me | 4-SO₂ⁿBu | - | 4-CF₃-6-Cl-Py-2-yl |
| 3-77 | Et | H | Me | Me | 4-SⁿBu | - | 2-Br-6-Cl-Py-4-yl |
| 3-78 | Et | H | Me | Me | 4-SO₂ⁿBu | - | 2-Br-6-Cl-Py-4-yl |
| 3-79 | CH₂CHF₂ | H | Me | Me | 4-SMe | - | 4-CF₃-6-Cl-Py-2-yl |
| 3-80 | CH₂C≡CH | H | Me | Me | 4-SMe | - | 4-CF₃-6-Cl-Py-2-yl |
| 3-81 | Me | H | Me | Me | 4-SMe | - | 4-CF₃-6-Cl-Py-2-yl |
| 3-82 | Et | H | Me | Me | 4-SOMe | - | 4-CF₃-6-Cl-Py-2-yl |
| 3-83 | Et | H | Me | Me | 4-SMe | - | 4-CF₃-6-CN-Py-2-yl |
| 3-84 | Et | H | Me | Me | 4-SMe | - | 4-CHF₂-6-Cl-Py-2-yl |
| 3-85 | Et | H | Me | Me | 4-SO₂Me | - | 4-CHF₂-6-Cl-Py-2-yl |
| 3-86 | Et | H | Me | Me | 4-SOMe | - | 4-CHF₂-6-Cl-Py-2-yl |
| 3-87 | Et | H | Me | Me | 4-SMe | - | 2-Cl-6-CN-Py-4-yl |
| 3-88 | Et | H | Me | Me | 4-SMe | - | 4-CF₂CH₃-6-Cl-Py-2-yl |
| 3-89 | Et | H | Me | Me | 4-SO₂Me | - | 4-CF₂CH₃-6-Cl-Py-2-yl |
| 3-90 | Et | H | Me | Me | 4-SOEt | - | 4-CF₃-6-Cl-Py-2-yl |
| 3-91 | Et | H | Me | Me | 4-SEt | - | 4-CF₃-6-Cl-Py-2-yl |
| 3-92 | Et | H | Me | Me | 4-SMe | - | 4-CH₂F-6-Cl-Py-2-yl |

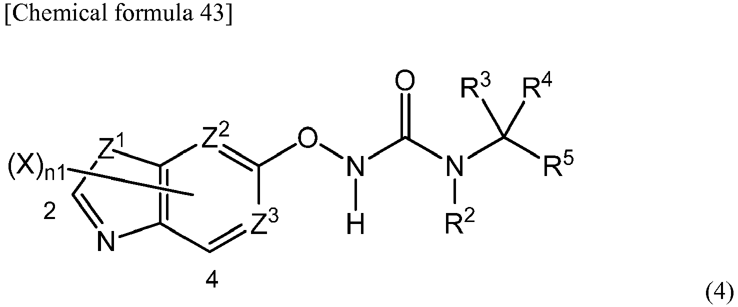

**[Table 4]**

| Compound No. | R² | R³ | R⁴ | R⁵ | (X)ₙ₁ | Z¹ | Z² | Z³ |
|---|---|---|---|---|---|---|---|---|
| a-1 | H | Me | Me | Py-2-yl | - | S | C | C |
| a-2 | H | Me | Me | Py-2-yl | - | S | N | C |
| a-3 | H | Me | Me | Pyrimidin-2-yl | - | S | C | C |
| a-4 | H | Me | Me | Py-2-yl | 2-Me | S | C | C |
| a-5 | H | Me | Me | CONHCH₂CF₃ | - | S | C | C |
| a-6 | H | Me | Me | Py-2-yl | 2-(2,5-Me₂-pyrrol-1-yl) | S | C | C |
| a-7 | H | Me | Me | Py-2-yl | 2-CN | S | C | C |
| a-8 | H | Me | Me | Py-2-yl | 2-CF₃ | S | C | C |
| a-9 | H | Me | Me | Py-2-yl | 2-Me | S | C | C |
| a-10 | H | Me | Me | Py-2-yl | 2-Me | S | N | C |
| a-11 | H | Me | Me | Py-2-yl | 2-CF₃ | S | C | N |
| a-12 | H | Me | Me | Pyrimidin-2-yl | 2-CF₃ | S | C | N |
| a-13 | H | Me | Me | 4-Ph-Py-2-yl | - | S | C | C |
| a-14 | H | Me | Me | Py-2-yl | 4-Br | S | C | C |
| a-15 | H | Me | Me | Pyrimidin-2-yl | 4-Br | S | C | C |
| a-16 | H | Me | Me | Py-2-yl | - | O | C | C |

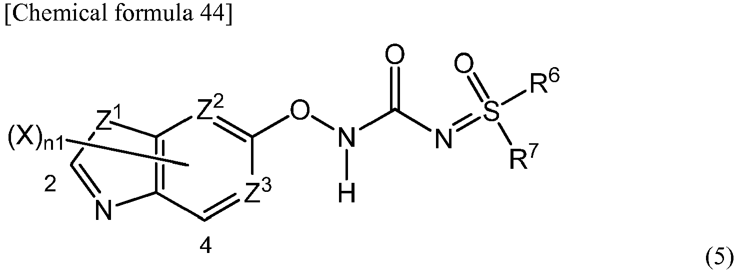

**[Table 5]**

| Compound No. | R⁶ | R⁷ | (X)ₙ₁ | Z¹ | Z² | Z³ |
|---|---|---|---|---|---|---|
| b-1 | Me | Ph | - | S | C | C |

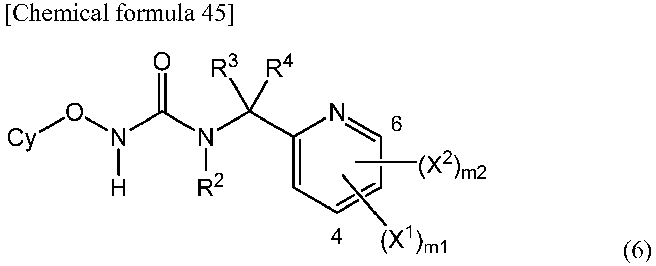

**[Table 6]**

| Compound No. | R² | R³ | R⁴ | (X¹)ₘ₁ | (X²)ₘ₂ | Cy |
|---|---|---|---|---|---|---|
| c-1 | H | Me | Me | 4-SMe | - | 3-Br-5-Cl-Ph |
| c-2 | H | Me | Me | 4-SO₂Me | - | 3-Br-5-Cl-Ph |
| c-3 | H | Me | Me | 4-SMe | - | 4-Br-3,5-Cl₂-Ph |
| c-4 | H | Me | Me | 4-SOMe | - | 4-Br-3,5-Cl₂-Ph |
| c-5 | H | Me | Me | 4-SO₂Me | - | 4-Br-3,5-Cl₂-Ph |
| c-6 | H | Me | Me | 4-SMe | - | 2-Cl-6-CF₃-Py-4-yl |
| c-7 | H | Me | Me | 4-SOMe | - | 2-Cl-6-CF₃-Py-4-yl |
| c-8 | H | Me | Me | 4-SO₂Me | - | 2-Cl-6-CF₃-Py-4-yl |
| c-9 | H | Me | Me | 4-SMe | - | 4-CF₃-6-Cl-Py-2-yl |
| c-10 | H | Me | Me | 4-SOMe | - | 4-CF₃-6-Cl-Py-2-yl |
| c-11 | H | Me | Me | 4-SO₂Me | - | 4-CF₃-6-Cl-Py-2-yl |
| c-12 | H | Me | Me | 4-SMe | - | 2,6-Br₂-Py-4-yl |
| c-13 | H | Me | Me | 4-SOMe | - | 2,6-Br₂-Py-4-yl |
| c-14 | H | Me | Me | 4-SO₂Me | - | 2,6-Br₂-Py-4-yl |
| c-15 | H | Me | Me | 4-SMe | - | 3-Cl-4,5-Br₂-Ph |
| c-16 | H | Me | Me | 4-SOMe | - | 3-Cl-4,5-Br₂-Ph |
| c-17 | H | Me | Me | 4-SO₂Me | - | 3-Cl-4,5-Br₂-Ph |
| c-18 | H | Me | Me | 4-NMe₂ | - | 3-Br-5-Cl-Ph |
| c-19 | H | Me | Me | 5-SMe | - | 3-Br-5-Cl-Ph |
| c-20 | H | Me | Me | 4-SMe | - | 4-CHF₂-6-Cl-Py-2-yl |
| c-21 | H | Me | Me | 4-SMe | - | 4-CF₂CH₃-6-Cl-Py-2-yl |

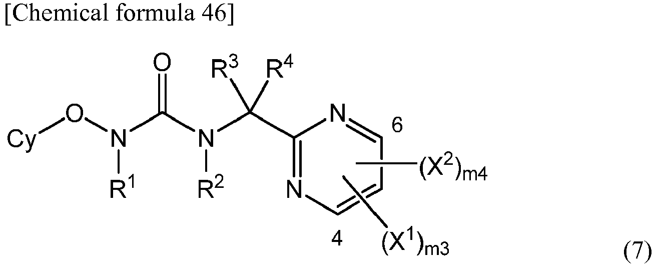

**[Table 7]**

| Compound No. | R¹ | R² | R³ | R⁴ | (X¹)m3 | (X²)m4 | Cy |
|---|---|---|---|---|---|---|---|
| 4-1 | Et | H | Me | Me | 4-SMe | - | 3-Br-5-Cl-Ph |
| 4-2 | Et | H | Me | Me | 4-SO₂Me | - | 3-Br-5-Cl-Ph |
| 4-3 | Et | H | Me | Me | 4-SMe | - | 4-CF₃-6-Cl-Py-2-yl |
| 4-4 | Et | H | Me | Me | 4-NMe₂ | - | 3-Br-5-Cl-Ph |
| 4-5 | Et | H | Me | Me | 4-NMe₂ | - | 3-Cl-4,5-Br₂-Ph |
| 4-6 | Et | H | Me | Me | 4-NMe₂ | - | 4-CF₃-6-Cl-Py-2-yl |
| 4-7 | Et | H | Me | Me | 4-N=S(=O)Me₂ | - | 3-Br-5-Cl-Ph |
| 4-8 | Et | H | Me | Me | 4-NMe-NHMe | - | 4-CF₃-6-Cl-Py-2-yl |

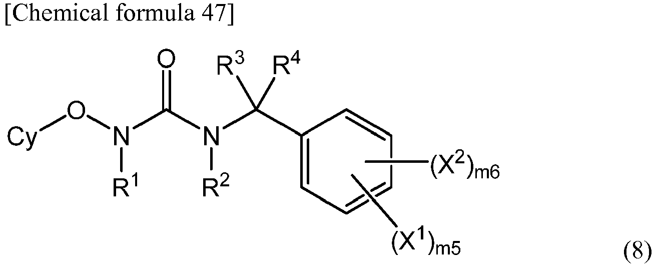

**[Table 8]**

| Compound No. | R¹ | R² | R³ | R⁴ | (X¹)ₘ₅ | (X²)ₘ₆ | Cy |
|---|---|---|---|---|---|---|---|
| 5-1 | Et | H | Me | Me | 3-SMe | - | 3-Br-5-Cl-Ph |
| 5-2 | Et | H | Me | Me | 3-SO₂Me | - | 3-Br-5-Cl-Ph |
| 5-3 | Et | H | Me | Me | 3-SOMe | - | 3-Br-5-Cl-Ph |

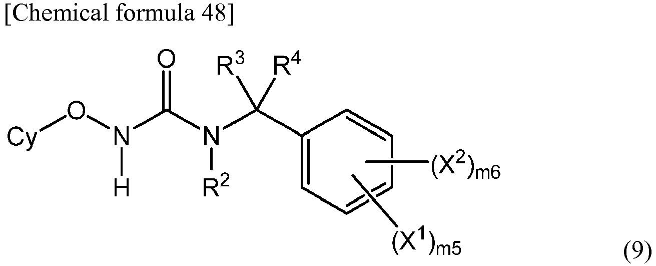

**[Table 9]**

| Compound No. | R² | R³ | R⁴ | (X¹)ₘ₅ | (X²)ₘ₆ | Cy |
|---|---|---|---|---|---|---|
| d-1 | H | Me | Me | 3-SMe | - | 3-Br-5-Cl-Ph |

Physical properties of some compounds shown in Tables 1-9 were measured and the results are shown below. In addition, ¹H-NMR (CDCl₃) or infraction index was recorded for the oil-form compounds and amorphous compounds. The records are shown below. The numbers described on the left side correspond to the Compound No. in the tables.

1-1: melting point 76-77 °C
1-2: melting point 116-118 °C
1-3: viscosity oil
1-4: melting point 106-108 °C
1-5: viscosity oil; ¹H-NMR (CDCl₃, δ(ppm)) 8.93(s, 1H), 8.09(d, 1H), 7.73(d, 1H), 7.35(br, 1H), 7.29(dd, 1H), 6.01(s, 1H), 3.98-3.89(m, 2H), 3.71(q, 2H), 1.55(s, 6H), 1.20(t, 3H)
1-6: viscosity oil; ¹H-NMR (CDCl₃, δ(ppm)) 8.42(d, 1H), 8.27(d, 1H), 8.00(s, 1H), 7.67(ddd, 1H), 7.42(d, 1H), 7.39(d, 1H), 7.13(ddd, 2H), 5.94(s, 2H), 3.81(q, 2H), 2.32(s, 6H), 1.75(s, 6H), 1.26(t, 3H)
1-7: amorphous; ¹H-NMR (CDCl₃, δ(ppm)) 8.34-8.32(m, 1H), 8.21(br, 1H), 8.15(d, 1H), 7.85(d, 1H), 7.66(dt, 1H), 7.50(dd, 1H), 7.35(d, 1H), 7.13-7.10(m, 1H), 3.74(t, 2H), 1.73(s, 6H), 1.22(t, 3H)
1-8: infraction index (23.8 °C) 1.5341
1-9: melting point 140-142 °C
1-10: melting point 146-147 °C
1-11: melting point 120-122 °C
1-12: melting point 127-128 °C
1-13: amorphous; ¹H-NMR (CDCl₃, δ(ppm)) 8.89(s, 1H), 8.44(d, 1H), 8.09(d, 1H), 8.03(s, 1H), 7.86(d, 1H), 7.60-7.55(m, 3H), 7.50-7.40(m, 4H), 7.34(dd, 1H), 3.75(q, 2H), 1.81(s, 6H), 1.24(t, 3H)
1-14: melting point 133-135 °C
1-15: melting point 139-141 °C
1-17: infraction index (20.3 °C) 1.5967
1-18: infraction index (20.4 °C) 1.5621

2-1: amorphous; ¹H-NMR (CDCl₃, δ(ppm)) 8.85(s, 1H), 8.03(d, 1H), 7.85-7.83(m, 2H), 7.66(d, 1H), 7.64-7.60(m, 1H), 7.53-7.49(m, 2H), 7.28(dd, 1H), 3.82(t, 2H), 3.33(s, 3H), 1.24(t, 3H)
3-1: amorphous; ¹H-NMR (CDCl₃, δ(ppm)) 8.23(d, 1H), 7.92(br, 1H), 7.31(d, 1H), 7.20-7.18(m, 2H), 7.13(d, 1H), 6.95(dd, 1H), 3.65(t, 2H), 2.47(s, 3H), 1.71(s, 6H), 1.17(t, 3H)
3-2: melting point 93-95 °C
3-3: melting point 120-121 °C
3-4: melting point 161-162 °C
3-5: melting point 135-136 °C
3-6: infraction index (22.1 °C) 1.5338
3-7: melting point 149-150 °C
3-8: melting point 148-149 °C
3-9: melting point 64-67 °C
3-10: infraction index (22.5 °C) 1.5350
3-11: melting point 110-112 °C
3-12: melting point 133-134 °C
3-13: infraction index (24.8 °C) 1.5779
3-14: melting point 160-161 °C
3-15: melting point 107-108 °C
3-16: melting point 156-157 °C
3-17: melting point 129-130 °C
3-18: melting point 59-61 °C
3-19: infraction index (20.4 °C) 1.5806
3-20: infraction index (20.7 °C) 1.5858
3-21: infraction index (20.8 °C) 1.5932
3-22: infraction index (20.9 °C) 1.5247
3-23: infraction index (21.5 °C) 1.5698
3-29: infraction index (20.4 °C) 1.5526

3-33: melting point 98-100 °C; ¹H-NMR (CDCl₃, δ(ppm)) 8.76(d, 1H), 7.84(d, 1H), 7.64(dd, 1H), 7.41(s, 1H), 7.36(s, 1H), 7.22(s, 1H), 3.71(br, 2H), 3.15(q, 2H), 1.76(s, 6H), 1.32(t, 3H), 1.22(t, 3H)
3-34: ¹H-NMR (CDCl₃, δ(ppm)) 8.75(d, 1H), 7.84(d, 1H), 7.66(dd, 1H), 7.36(s, 2H), 7.33(s, 1H), 3.67(br, 2H), 3.15(q, 2H), 1.77(s, 6H), 1.32(t, 3H), 1.18(t, 3H)
3-39: infraction index (20.6 °C) 1.5642
3-42: ¹H-NMR (CDCl₃, δ(ppm)) 8.21(d, 1H), 7.79(s, 1H), 7.14(d, 1H), 6.95(dd, 1H), 6.69(s, 1H), 3.74(br, 2H), 3.21(s, 6H), 2.48(s, 3H), 1.71(s, 6H), 1.21(t, 3H) 3-43: ¹H-NMR (CDCl₃, δ(ppm)) 8.20(d, 1H), 8.14(s, 1H), 7.17(s, 2H), 7.15(d, 1H), 6.99(dd, 1H), 3.70(br, 2H), 2.50(s, 3H), 1.72(s, 6H), 1.19(t, 3H)
3-44: ¹H-NMR (CDCl₃, δ(ppm)) 8.76(d, 1H), 7.89(s, 1H), 7.70(dd, 1H), 7.30(s, 1H), 7.17(s, 2H), 3.66(br, 2H), 3.11(s, 3H), 1.77(s, 6H), 1.18(t, 3H)
3-45: ¹H-NMR (CDCl₃, δ(ppm)) 8.23(d, 1H), 7.81(s, 1H), 7.14(d, 1H), 6.96(dd, 1H), 6.46(d, 1H), 6.21(d, 1H), 3.67(br, 2H), 3.06(s, 6H), 2.48(s, 3H), 1.71(s, 6H), 1.18(t, 3H) 3-46: ¹H-NMR (CDCl₃, δ(ppm)) 8.76(d, 1H), 7.87(s, 1H), 7.65(dd, 1H), 6.94(s, 1H), 6.44(d, 1H), 6.19(d, 1H), 3.63(br, 2H), 3.08(s, 9H), 1.75(s, 6H), 1.17(t, 3H) 3-48: infraction index (21.4 °C) 1.5888
3-49: amorphous
3-50: ¹H-NMR (CDCl₃, δ(ppm)) 8.20(d, 1H), 7.87(s, 1H), 7.14-7.13(m, 2H), 7.08(s, 1H), 6.95(dd, 1H), 3.76(br, 2H), 2.62(s, 3H), 2.47(s, 3H), 1.72(s, 6H), 1.23(t, 3H) 3-51: ¹H-NMR (CDCl₃, δ(ppm)) 8.23(d, 1H), 7.55(s, 1H), 7.14(d, 1H), 6.93(dd, 1H), 6.61(s, 1H), 6.39(s, 1H), 3.73(br, 2H), 3.14(s, 6H), 2.46(s, 3H), 1.70(s, 6H), 1.21(t, 3H) 3-52: ¹H-NMR (CDCl₃, δ(ppm)) 8.26(d, 1H), 7.69(s, 1H), 7.15(d, 1H), 6.97-6.95(m, 2H), 6.87(d, 1H), 3.71(br, 2H), 2.49(s, 3H), 2.47(s, 3H), 1.71(s, 6H), 1.21(t, 3H) 3-53: melting point 145-146 °C
3-54: ¹H-NMR (CDCl₃, δ(ppm)) 8.79(d, 1H), 7.84(d, 1H), 7.68(d, 1H), 7.66(dd, 1H), 7.62(d, 1H), 7.15(s, 1H), 3.73(br, 2H), 3.16(s, 3H), 3.10(s, 3H), 1.76(s, 6H), 1.22(t, 3H) 3-55: ¹H-NMR (CDCl₃, δ(ppm)) 8.75(d, 1H), 7.87(s, 1H), 7.64-7.62(m, 1H), 6.85(s, 1H), 6.58(s, 1H), 6.43(s, 1H), 3.69(q, 2H), 3.15(s, 6H), 3.07(s, 3H), 1.73(s, 6H), 1.21(t, 3H)
3-56: ¹H-NMR (CDCl₃, δ(ppm)) 8.26(d, 1H), 7.86(s, 1H), 7.23(d, 1H), 7.14(s, 1H), 7.11(d, 1H), 6.96(dd, 1H), 3.71(br, 2H), 2.48(s, 3H), 1.72(s, 6H), 1.21(t, 3H) 3-57: melting point 140-141 °C
3-58: melting point 132-133 °C
3-59: ¹H-NMR (CDCl₃, δ(ppm)) 8.26(d, 1H), 7.87(s, 1H), 7.40(s, 1H), 7.27(s, 1H), 7.14(d, 1H), 6.96(dd, 1H), 3.72(br, 2H), 2.48(s, 3H), 1.72(s, 6H), 1.21(t, 3H) 3-60: melting point 151-152 °C
3-61: ¹H-NMR (CDCl₃, δ(ppm)) 8.74(d, 1H), 7.82(s, 1H), 7.62(dd, 1H), 7.41(s, 1H), 7.35(s, 1H), 7.23(s, 1H), 3.71(br, 2H), 3.10-3.06(m, 2H), 1.75(s, 6H), 1.83-1.73(m, 2H), 1.22(t, 3H), 1.03(t, 3H)
3-62: ¹H-NMR (CDCl₃, δ(ppm)) 8.21(d, 1H), 7.73(s, 1H), 7.30(s, 1H), 7.14(s, 1H), 7.13(s, 1H), 6.94(dd, 1H), 3.74(br, 2H), 2.61(s, 3H), 2.46(s, 3H), 1.71(s, 6H), 1.22(t, 3H) 3-63: melting point 122-123 °C
3-64: melting point 179-180 °C
3-65: ¹H-NMR (CDCl₃, δ(ppm)) 8.21(s, 1H), 8.19(d, 1H), 7.34(d, 1H), 7.20(d, 1H), 7.14(d, 1H), 6.99(dd, 1H), 3.70(br, 2H), 3.01(q, 2H), 1.72(s, 6H), 1.39(t, 3H), 1.19(t, 3H) 3-66: melting point 121-123 °C
3-67: melting point 158-159 °C
3-68: ¹H-NMR (CDCl₃, δ(ppm)) 8.22(s, 1H), 8.18(d, 1H), 7.18(s, 2H), 7.14(d, 1H), 6.99(dd, 1H), 3.72(br, 2H), 3.01(q, 2H), 1.72(s, 6H), 1.39(t, 3H), 1.20(t, 3H) 3-69: melting point 102-103 °C
3-70: melting point 98-99 °C
3-71: ¹H-NMR (CDCl₃, δ(ppm)) 8.20(d, 1H), 8.05(s, 1H), 7.42(s, 1H), 7.31(s, 1H), 7.14(d, 1H), 6.96(dd, 1H), 3.75(br, 2H), 2.94(t, 2H), 1.71(s, 6H), 1.78-1.69(m, 2H), 1.23(t, 3H), 1.06(t, 3H)
3-72: melting point 148-150 °C
3-73: melting point 115-117 °C
3-74: melting point 125-126 °C
3-75: ¹H-NMR (CDCl₃, δ(ppm)) 8.21(d, 1H), 8.06(s, 1H), 7.42(s, 1H), 7.31(s, 1H), 7.14(d, 1H), 6.96(dd, 1H), 3.75(br, 2H), 2.96(t, 2H), 1.71(s, 6H), 1.73-1.65(m, 2H), 1.55-1.42(m, 2H), 1.23(t, 3H), 0.96(t, 3H)
3-76: melting point 88-90 °C
3-77: melting point 74-76 °C
3-78: melting point 134-136 °C
3-79: infraction index (22.2 °C) 1.5263
3-80: ¹H-NMR (CDCl₃, δ(ppm)) 8.27(s, 1H), 8.24(d, 1H), 7.46(s, 1H), 7.33(s, 1H), 7.17(d, 1H), 6.98(dd, 1H), 4.48(d, 2H), 2.49(s, 3H), 2.24(t, 1H), 1.74(s, 6H) 3-81: melting point 70-72 °C
3-82: ¹H-NMR (CDCl₃, δ(ppm)) 8.58(d, 1H), 7.66(s, 1H), 7.54(d, 1H), 7.41(s, 1H), 7.33-7.31(m, 2H), 3.72(br, 2H), 2.76(s, 3H), 1.75(s, 3H), 1.73(s, 3H), 1.21(t, 3H) 3-83: ¹H-NMR (CDCl₃, δ(ppm)) 8.23(s, 1H), 8.21(d, 1H), 7.75(s, 1H), 7.66(s, 1H), 7.13(d, 1H), 6.97(dd, 1H), 3.78(br, 2H), 2.49(s, 3H), 1.72(s, 6H), 1.24(t, 3H) 3-84: ¹H-NMR (CDCl₃, δ(ppm)) 8.23(d, 1H), 7.91(s, 1H), 7.30(s, 1H), 7.20(s, 1H), 7.11(d, 1H), 6.93(dd, 1H), 6.57(t, 1H), 3.72(br, 2H), 2.46(s, 3H), 1.69(s, 6H), 1.20(t, 3H) 3-85: melting point 128-130 °C
3-86: ¹H-NMR (CDCl₃, δ(ppm)) 8.59(d, 1H), 7.65(s, 1H), 7.36(s, 1H), 7.32-7.24(m, 3H), 6.65(t, 1H), 3.70(br, 2H), 2.75(s, 3H), 1.74(s, 3H), 1.72(s, 3H), 1.20(t, 3H) 3-87: 8.31(s, 1H), 8.17(d, 1H), 7.52(d, 1H), 7.46(d, 1H), 7.14(d, 1H), 6.99(dd, 1H), 3.79(br, 2H), 2.50(s, 3H), 1.72(s, 6H), 1.21(t, 3H)
3-88: melting point 127-129 °C
3-89: melting point 139-141 °C
3-90: ¹H-NMR (CDCl₃, δ(ppm)) 8.56(d, 1H), 7.62(s, 1H), 7.59(s, 1H), 7.40(s, 1H), 7.32-7.28(m, 2H), 3.71(br, 2H), 2.99-2.92(m, 1H), 2.77-2.70(m, 1H), 1.74(s, 3H), 1.72(s, 3H), 1.24(t, 3H), 1.20(t, 3H)
3-91: melting point 70-72 °C
3-92: melting point 88-90 °C

4-1: ¹H-NMR (CDCl₃, δ(ppm)) 8.28(d, 1H), 7.80(s, 1H), 7.32-7.31(m, 1H), 7.21-7.19(m, 2H), 7.01(d, 1H), 3.67(q, 2H), 2.42(s, 3H), 1.80(s, 6H), 1.18(t, 3H)
4-3: ¹H-NMR (CDCl₃, δ(ppm)) 8.27(d, 1H), 7.96(s, 1H), 7.45(s, 1H), 7.31(s, 1H), 7.02(d, 1H), 3.75(br, 2H), 2.53(s, 3H), 1.79(s, 6H), 1.23(t, 3H)
4-8: ¹H-NMR(CDCl₃, δ(ppm)) 8.34(s, 1H), 8.14(d, 1H), 7.46(s, 1H), 7.29(s, 1H), 6.75(br, 1H), 3.77(br, 2H), 3.23(s, 3H), 2.64(s, 3H), 1.76(s, 6H), 1.23(t, 3H) 5-1: melting point 88-90 °C
5-2: melting point 135-137 °C
5-3: melting point 116-118 °C

a-1: melting point 164-166 °C
a-4: melting point 152-154 °C
a-7: melting point 100-102 °C
a-8: melting point 149-151 °C
b-1: melting point 165-168 °C
c-1: melting point 139-141 °C
c-20: melting point 95-97 °C
c-21: melting point 116-118 °C
d-1: melting point 125-127 °C

Some preparation examples of the pest control agent according to the present invention are shown below. However, additives and addition ratios are not limited to the preparation examples, and can be modified over a wide range. Moreover, the term "parts" used in the preparation examples indicates "weight parts." The following are the preparation examples for agricultural and horticultural use.

| Preparation example 1 | (wettable powder) |
|---|---|
| Compound of the present invention | 40 parts |
| Diatom earth | 53 parts |
| Fatty alcohol sulfate | 4 parts |
| Alkylnaphthalene sulfonate | 3 parts |

The foregoing was uniformly mixed and finely pulverized to obtain a wettable powder including 40% of active ingredient.

| Preparation example 2 | (emulsion) |
|---|---|
| Compound of the present invention | 30 parts |
| Xylene | 33 parts |
| Dimethylformamide | 30 parts |
| Polyoxyethylene alkylaryl ether | 7 parts |

The foregoing was mixed and dissolved to obtain an emulsion including 30% of active ingredient.

The following are the preparation examples for epidemic-prevention and animals.

| Preparation example 3 (granulated powder) | |
|---|---|
| Compound of the present invention | 5 parts |
| Kaolin | 94 parts |
| White carbon | 1 part |

The compound of the present invention was dissolved in an organic solvent, and sprayed on a carrier, followed by evaporating the solvent under reduced pressure. This kind of granulated powder may be mixed with animal food.

| Preparation example 4 (impregnating agent) | |
|---|---|
| Compound of the present invention | 0.1-1 parts |
| Peanut oil | balance |

The impregnating agent was filter-sterilized by a sterilizing filter after adjustment.

| Preparation example 5 (pour-on agent) | |
|---|---|
| Compound of the present invention | 5 parts |
| Myristic acid ester | 10 parts |
| Isopropanol | balance |

| Preparation example 6 (spot-on agent) | |
|---|---|
| Compound of the present invention | 10-15 parts |
| Palmitic acid ester | 10 parts |
| Isopropanol | balance |

| Preparation example 7 (spray-on agent) | |
|---|---|
| Compound of the present invention | 1 part |
| Propylene glycol | 10 parts |
| Isopropanol | balance |

### [Biological examination]

The following test examples demonstrate that the compound of the present invention is useful as an active ingredient of acaricide.

### Test Example 1 Efficacy Test Against Tetranychus urticae

Ten organic phosphorous-resistant adult female Tetranychus urticae acarus were inoculated onto the first leaves of a kidney bean plant planted in a No.3 pot 7 to 10 days after germination. Next, an emulsion was prepared having the formula indicated in the aforementioned Preparation example 2. This emulsion was diluted with water to a compound concentration of 125 ppm after which the diluted liquids were sprayed onto the kidney bean plant. The kidney bean plant was then placed in a temperature-controlled room at a temperature of 25°C and humidity of 65%. The life and death of the adult insects were investigated 3 days after the spraying. In addition, the development from eggs laid to adult was investigated 14 days after the spraying.

The aforementioned test was carried out on the emulsions containing the Compound Nos. 1-1, 1-2, 1-3, 1-4, 1-5, 1-7, 1-8, 1-11, 1-12, 1-13, 2-1, 3-1, 3-2, 3-3, 3-5, 3-6, 3-7, 3-8, 3-9, 3-10, 3-11, 3-12, 3-13, 3-14, 3-15, 3-17, 3-18, 3-19, 3-20, 3-21, 3-22, 3-23, 3-24, 3-25, 3-26, 3-27, 3-28, 3-29, 3-30, 3-31, 3-32, 3-33, 3-34, 3-35, 3-36, 3-37, 3-38, 3-39, 3-40, 3-41, 3-42, 3-43, 3-44, 3-45, 3-46, 3-47, 3-48, 3-49, 3-50, 3-51, 3-52, 3-54, 3-55, 3-56, 3-57, 3-58, 3-59, 3-60, 3-61, 3-62, 3-63, 3-64, 3-65, 3-66, 3-67, 3-68, 3-69, 3-70, 3-71, 3-72, 3-73, 3-74, 3-75, 3-76, 3-77, 3-78, 3-79, 3-80, 3-81, 3-82, 3-83, 4-1, 4-2, 4-3, 4-4, 4-5, 4-6, 4-7, 5-1, and 5-3. As a result, the insect mortality rates after 3 days and 10 days for all of the compounds were 90% or higher.

### Test Example 2 Efficacy Test Against Panonychus citri

Eight adult female Panonychus citri acarus from Kanagawa Prefecture were inoculated onto a mandarin orange leaf placed in a Petri dish. Next, an emulsion was prepared having the formula indicated in the aforementioned Preparation example 2. This emulsion was diluted with water to a compound concentration of 125 ppm after which the diluted liquids were sprayed onto the mandarin orange leaf with a rotary spraying tower. The mandarin orange leaf was then placed in a temperature-controlled room at a temperature of 25°C and a humidity of 65%. The life and death of the adult insects were investigated 3 days after the spraying. In addition, the development from eggs laid to adult was investigated 10 days after the spraying.

The aforementioned test was carried out on the emulsions containing the Compound Nos. 1-1, 1-2, 1-3, 1-4, 1-5, 1-8, 1-13, 2-1, 3-1, 3-2, 3-3, 3-5, 3-6, 3-7, 3-9, 3-11, 3-12, 3-14, 3-18, 3-19, 3-20, 3-21, 3-22, 3-23, 3-24, 3-26, 3-27, 3-28, 3-33, 3-34, 3-35, 3-36, 3-37, 3-38, 3-39, 3-40, 3-41, 3-43, 3-44, 3-45, 3-46, 3-47, 3-48, 3-52, 3-53, 3-55, 3-56, 3-57, 3-58, 3-59, 3-60, 3-61, 3-62, 3-63, 3-64, 3-65, 3-66, 3-68, 3-69, 3-70, 3-71, 3-72, 3-73, 3-74, 3-75, 3-79, 3-80, 3-81, 3-82, 3-83, 4-4, 4-6, 4-7, and 5-1. As a result, the insect mortality rates after 3 days and 10 days for all of the compounds were 90% or higher.

### Test Example 3 Efficacy Test Against Panonychus citri

Eight acaricide-resistant adult female Panonychus citri acarus from Wakayama Prefecture were inoculated onto a mandarin orange leaf placed in a Petri dish. Next, an emulsion was prepared having the formula indicated in the aforementioned Preparation example 2. This emulsion was diluted with water to a compound concentration of 125 ppm after which the diluted liquids were sprayed onto the mandarin orange leaf with a rotary spraying tower. The mandarin orange leaf was then placed in a temperature-controlled room at a temperature of 25°C and a humidity of 65%. The life and death of the adult insects were investigated 3 days after the spraying. In addition, the development from eggs laid to adult was investigated 10 days after the spraying.

The aforementioned test was carried out on the emulsions containing the Compound Nos. 1-1, 1-3, 1-8, 1-13, 3-1, 3-2, 3-3, 3-9, 3-11, 3-18, 3-22, 3-23, 3-28, 3-33, 3-46, 3-47, 3-56, 3-58, 3-82, and 4-7. As a result, the insect mortality rates after 3 days and 10 days for all of the compounds were 90% or higher.

### Test Example 4 Efficacy Test Against Tetranychus kanzawai

Ten adult female Tetranychus kanzawai acarus from Okayama Prefecture were inoculated onto the first leaves of a kidney bean plant planted in a No.3 pot 7 to 10 days after germination. Next, an emulsion was prepared having the formula indicated in the aforementioned Preparation example 2. This emulsion was diluted with water to a compound concentration of 500 ppm or 125 ppm after which the diluted liquids were sprayed onto the kidney bean plant. The kidney bean plant was then placed in a temperature-controlled room at a temperature of 25°C and humidity of 65%. The life and death of the adult insects were investigated 3 days after the spraying. In addition, the development from eggs laid to adult was investigated 14 days after the spraying.

The aforementioned test was carried out on the emulsion containing the Compound No. 2-1 at 500 ppm. As a result, the insect mortality rates after 3 days and 14 days for the compound were 90% or higher.

In addition, the aforementioned test was carried out on the emulsions containing the Compound Nos. 1-1, 1-3, 1-4, 1-7, 1-8, 1-11, 1-13, 1-14,3-1,3-2,3-3,3-4,3-5,3-6, 3-7, 3-8, 3-9, 3-10, 3-11, 3-12, 3-13, 3-14, 3-15, 3-16, 3-17, 3-18, 3-19, 3-20, 3-21, 3-22, 3-23, 3-24, 3-25, 3-26, 3-27, 3-28, 3-29, 3-30, 3-31, 3-32, 3-33, 3-34, 3-35, 3-36, 3-37, 3-38, 3-39, 3-40, 3-41, 3-42, 3-43, 3-44, 3-45, 3-46, 3-47, 3-48, 3-50, 3-51, 3-52, 3-53, 3-55, 3-56, 3-57, 3-58, 3-59, 3-60, 3-61, 3-62, 3-63, 3-64, 3-65, 3-66, 3-67, 3-68, 3-69, 3-70, 3-71, 3-72, 3-73, 3-74, 3-75, 3-76, 3-77, 3-79, 3-80, 3-81, 3-82, 3-83, 3-84, 3-85, 3-86, 4-1, 4-2, 4-3, 4-4, 4-5, 4-6, 4-7, 4-8, 5-1, 5-3, and Compound 20 in Example 4 at 125 ppm. As a result, the insect mortality rates after 3 days and 14 days for all of the compounds were 90% or higher.

### Test Example 5 Efficacy Test Against Aculops pelekassi

Twenty acaricide-resistant adult female Aculops pelekassi acarus were inoculated onto a mandarin orange leaf placed in a Petri dish. Next, an emulsion was prepared having the formula indicated in the aforementioned Preparation example 2. This emulsion was diluted with water to a compound concentration of 125 ppm after which the diluted liquids were sprayed onto the mandarin orange leaf with a rotary spraying tower. The mandarin orange leaf was then placed in a temperature-controlled room at a temperature of 25°C and a humidity of 65%. The life and death of the adult insects were investigated 3 days after the spraying. The development from eggs laid to adult was investigated 10 days after the spraying.

The aforementioned test was carried out on the emulsion containing the Compound No. 1-1, 3-18, and 4-7. As a result, the insect mortality rates after 3 days and 10 days for the compound were 90% or higher.

### INDUSTRIAL APPLICABILITY

The aryloxyurea compound or salt thereof according to the present invention can prevent pests which are harmful for agricultural crops and cause problem of hygiene. Particularly, the compound can effectively prevent acarus and insecticides. Therefore, the present invention can be used for preventing pests, and extremely useful for industry.

## Claims

1. An aryloxyurea compound represented by formula (I) or salt thereof: 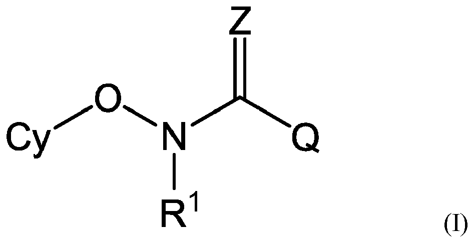 in formula (I),
Cy represents an unsubstituted or X-substituted C6-10 aryl group, or unsubstituted or X-substituted heteroaryl group;
X represents an unsubstituted or substituted C1-6 alkyl group, unsubstituted or substituted C3-8 cycloalkyl group, unsubstituted or substituted C2-6 alkenyl group, unsubstituted or substituted C2-6 alkynyl group, hydroxy group, unsubstituted or substituted C1-6 alkoxy group, unsubstituted or substituted amino group, unsubstituted or substituted C1-7 acyl group, unsubstituted or substituted C1-6 alkoxycarbonyl group, unsubstituted or substituted C1-6 alkylthio group, unsubstituted or substituted C1-6 alkyl sulfonyl group, unsubstituted or substituted C1-6 alkoxysulfonyl group, unsubstituted or substituted C6-10 aryl group, unsubstituted or substituted heteroaryl group, unsubstituted or substituted hydroxyimino C1-6 alkyl group, nitro group, cyano group, or halogen atom; X may be 2 or more, when X is 2 or more, Xs may be the same as or different from each other;
R¹ represents an unsubstituted or substituted C1-6 alkyl group, unsubstituted or substituted C2-6 alkenyl group, unsubstituted or substituted C2-6 alkynyl group, unsubstituted or substituted C1-7 acyl group, or unsubstituted or substituted C1-6 alkoxycarbonyl group;
Z represents an oxygen atom or sulfur atom;
Q represents a group represented by formula (II) or (III): 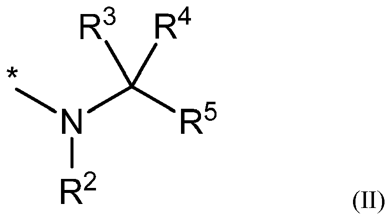 in formula (II),
* represents bonding position;
R² represents a hydrogen atom, unsubstituted or substituted C1-6 alkyl group, unsubstituted or substituted C2-6 alkenyl group, unsubstituted or substituted C2-6 alkynyl group, unsubstituted or substituted C1-7 acyl group, or unsubstituted or substituted C1-6 alkoxycarbonyl group;
R³ and R⁴ each independently represents a hydrogen atom, unsubstituted or substituted C1-6 alkyl group, unsubstituted or substituted C2-6 alkenyl group, unsubstituted or substituted C2-6 alkynyl group, unsubstituted or substituted C6-10 aryl group, unsubstituted or substituted heteroaryl group, or cyano group; R³ and R⁴ may bond to form a ring together with the carbon atom which bonds with R³ and R⁴;
R⁵ represents an unsubstituted or substituted C1-6 alkyl group, unsubstituted or substituted C3-8 cycloalkyl group, unsubstituted or substituted C2-6 alkenyl group, unsubstituted or substituted C2-6 alkynyl group, unsubstituted or substituted C1-7 acyl group, carboxyl group, unsubstituted or substituted C1-6 alkoxycarbonyl group, unsubstituted or substituted C2-6 alkenyloxycarbonyl group, unsubstituted or substituted C2-6 alkynyloxycarbonyl group, unsubstituted or substituted aminocarbonyl group, unsubstituted or substituted C6-10 aryl group, unsubstituted or substituted heteroaryl group, unsubstituted or substituted hydroxyimino C1-6 alkyl group, or cyano group; 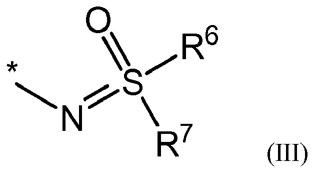 in formula (III),
* represents bonding position;
R⁶ and R⁷ each independently represents an unsubstituted or substituted C1-6 alkyl group, unsubstituted or substituted C3-8 cycloalkyl group, unsubstituted or substituted C2-6 alkenyl group, unsubstituted or substituted C2-6 alkynyl group, unsubstituted or substituted C6-10 aryl group, or unsubstituted or substituted heteroaryl group; R⁶ and R¹ may bond to form a ring together with the sulfur atom which bonds with R⁶ and R⁷.

2. The aryloxyurea compound or salt thereof according to Claim 1, wherein
Cy of formula (I) is a group represented by formula (IV): 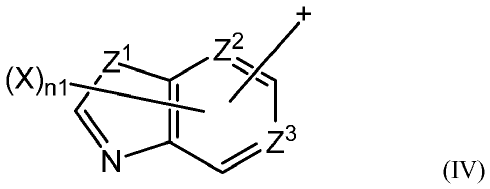 in formula (IV),
+ represents bonding position;
X is as defiend above;
n1 represents the number of X and represents an integer of 0 to 4;
Z¹ represents an oxygen atom or sulfur atom;
Z² and Z³ each independently represents a carbon atom or nitrogen atom.

3. The aryloxyurea compound or salt thereof according to Claim 1 or 2, wherein
Q of formula (I) is a group represented by formula (V): 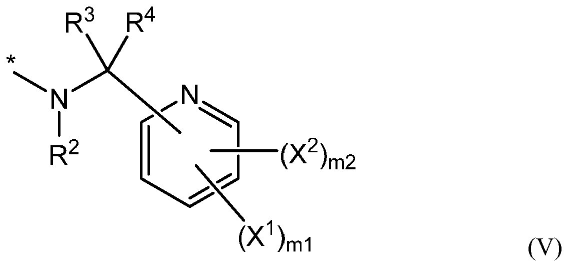 in formula (V),
* represents bonding position;
R², R³ and R⁴ are as defiend above;
X¹ represents an unsubstituted or substituted amino group, unsubstituted or substituted C1-6 alkylthio group, unsubstituted or substituted C1-6 alkyl sulfinyl group, or unsubstituted or substituted C1-6 alkyl sulfonyl group;
m1 represents the number of X¹ and represents an integer of 1 to 4, when m1 is 2 or more, X¹s may be the same as or different from each other;
X² represents an unsubstituted or substituted C1-6 alkyl group, unsubstituted or substituted C3-8 cycloalkyl group, unsubstituted or substituted C2-6 alkenyl group, unsubstituted or substituted C2-6 alkynyl group, hydroxy group, unsubstituted or substituted C1-6 alkoxy group, unsubstituted or substituted C1-7 acyl group, unsubstituted or substituted C1-6 alkoxycarbonyl group, unsubstituted or substituted C6-10 aryl group, unsubstituted or substituted heteroaryl group, oxo group, nitro group, cyano group, or halogen atom;
m2 represents the number of X² and represents an integer of 0 to 3, when m2 is 2 or more, X²s may be the same as or different from each other, the sum of m1 and m2 is 4 or less.

4. The aryloxyurea compound or salt thereof according to Claim 1 or 2, wherein
Q of formula (I) is a group represented by formula (VI): 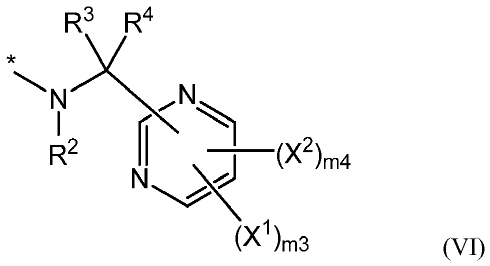 in formula (VI),
* represents bonding position;
R², R³, and R⁴ are as difined above;
X¹ represents an unsubstituted or substituted amino group, unsubstituted or substituted C1-6 alkylthio group, unsubstituted or substituted C1-6 alkyl sulfinyl group, or unsubstituted or substituted C1-6 alkyl sulfonyl group;
m3 represents the number of X¹ and represents an integer of 1 to 3, when m3 is 2 or more, X¹s may be the same as or different from each other;
X² represents an unsubstituted or substituted C1-6 alkyl group, unsubstituted or substituted C3-8 cycloalkyl group, unsubstituted or substituted C2-6 alkenyl group, unsubstituted or substituted C2-6 alkynyl group, hydroxy group, unsubstituted or substituted C1-6 alkoxy group, unsubstituted or substituted C1-7 acyl group, unsubstituted or substituted C1-6 alkoxycarbonyl group, unsubstituted or substituted C6-10 aryl group, unsubstituted or substituted heteroaryl group, oxo group, nitro group, cyano group, or halogen atom;
m4 represents the number of X² and represents an integer of 0 to 2, when m4 is 2 or more, X²s may be the same as or different from each other; the sum of m3 and m4 is 3 or less.

5. The aryloxyurea compound or salt thereof according to Claim 1 or 2, wherein
Q of formula (I) is a group represented by formula (VII): 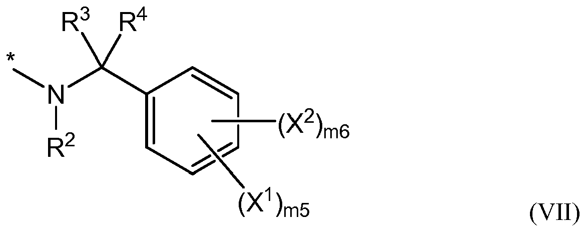 in formula (VII),
* represent bonding position;
R², R³, and R⁴ are as difined above;
X¹ represents an unsubstituted or substituted amino group, unsubstituted or substituted C1-6 alkylthio group, unsubstituted or substituted C1-6 alkyl sulfinyl group, or unsubstituted or substituted C1-6 alkyl sulfonyl group;
m5 represents the number of X¹ and represents an integer of 1 to 5, when m5 is 2 or more, X¹s may be the same as or different from each other;
X² represents an unsubstituted or substituted C1-6 alkyl group, unsubstituted or substituted C3-8 cycloalkyl group, unsubstituted or substituted C2-6 alkenyl group, unsubstituted or substituted C2-6 alkynyl group, hydroxy group, unsubstituted or substituted C1-6 alkoxy group, unsubstituted or substituted C1-7 acyl group, unsubstituted or substituted C1-6 alkoxycarbonyl group, unsubstituted or substituted C6-10 aryl group, unsubstituted or substituted heteroaryl group, oxo group, nitro group, cyano group, or halogen atom;
m6 represents the number X² and represents an integer of 0 to 4, when m6 is 2 or more, X²s may be the same as or different from each other, the sum of m5 and m6 is 5 or less.

6. A pest control agent comprising as an active ingredient at least one selected from the aryloxyurea compounds and salts thereof defined in any one of Claims 1 to 5.

7. An acaricide or insenticide comprising as an active ingredient at least one selected from the aryloxyurea compounds and salts thereof defined in any one of Claims 1 to 5.

8. An ectoparasite control agent comprising as an active ingredient at least one selected from the aryloxyurea compounds and salts thereof defined in any one of Claims 1 to 5.

9. A benzyloxyurea compound represented by formula (VIII): 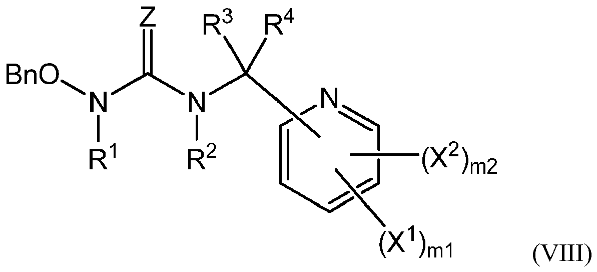 in formula (VIII),
R¹ represents an unsubstituted or substituted C1-6 alkyl group, unsubstituted or substituted C2-6 alkenyl group, unsubstituted or substituted C2-6 alkynyl group, unsubstituted or substituted C1-7 acyl group, or unsubstituted or substituted C1-6 alkoxycarbonyl group;
R² represents a hydrogen atom, unsubstituted or substituted C1-6 alkyl group, unsubstituted or substituted C2-6 alkenyl group, unsubstituted or substituted C2-6 alkynyl group, unsubstituted or substituted C1-7 acyl group, or unsubstituted or substituted C1-6 alkoxycarbonyl group;
R³ and R⁴ each independently represents a hydrogen atom, unsubstituted or substituted C1-6 alkyl group, unsubstituted or substituted C2-6 alkenyl group, unsubstituted or substituted C2-6 alkynyl group, unsubstituted or substituted C6-10 aryl group, unsubstituted or substituted heteroaryl group, or cyano group; R³ and R⁴ may bond to form a ring together with the carbon atom which bonds with R³ and R⁴.
Z represents an oxygen atom or sulfur atom;
X¹ represents an unsubstituted or substituted amino group, unsubstituted or substituted C1-6 alkylthio group, unsubstituted or substituted C1-6 alkyl sulfinyl group, or unsubstituted or substituted C1-6 alkyl sulfonyl group;
m1 represents the number of X¹ and represents an integer of 1 to 4, when m1 is 2 or more, X¹s may be the same as or different from each other;
X² represents an unsubstituted or substituted C1-6 alkyl group, unsubstituted or substituted C3-8 cycloalkyl group, unsubstituted or substituted C2-6 alkenyl group, unsubstituted or substituted C2-6 alkynyl group, hydroxy group, unsubstituted or substituted C1-6 alkoxy group, unsubstituted or substituted C1-7 acyl group, unsubstituted or substituted C1-6 alkoxycarbonyl group, unsubstituted or substituted C6-10 aryl group, unsubstituted or substituted heteroaryl group, oxo group, nitro group, cyano group, or halogen atom;
m2 represents the number of X² and represents an integer of 0 to 3, when m2 is 2 or more, X²s may be the same as or different from each other, the sum of m1 and m2 is 4 or less.
